# EUROPEAN PATENT SPECIFICATION

(11) **EP 2 236 503 B1**
(45) Date of publication and mention of the grant of the patent: **26.02.2014**
(21) Application number: 09004981.8
(22) Date of filing: 03.04.2009
(51) Int. Cl.: C07D 303/40, C07D 307/86, C07C 59/64, C07C 69/708, C07C 235/38, C07C 259/06, A61K 31/165, A61K 31/216, A61K 31/336, A61K 31/343, A61P 3/10, A61P 25/28, A61P 35/00

(54) **Cinamic compounds and derivatives therefrom for the inhibition of histone deacetylase**
Zimtzusammensetzungen und Derivate davon zur Hemmung von Histon-Deacetylase
Composants cinnamiques et dérivés de celui-ci pour l'inhibition de l'histone désacétylase

(43) Date of publication of application: 06.10.2010
(73) Proprietor: NatureWise Biotech & Medicals Corporation, Taipei City (TW)
(72) Inventor: Huang, Chung-Yang, Peitou Taipei 112 (TW); Chen, Chia-Nan, Peitou Taipei 112 (TW); Huang, Wei-Jan, Peitou Taipei 112 (TW); Lin, Chia-Wei, Peitou Taipei 112 (TW); Huang, Jing-Shi, Peitou Taipei 112 (TW); Chi, Li-Ling, Peitou Taipei 112 (TW); Chen Al-Ling, Peitou Taipei 112 (TW); Lee, Chi-Yun, Peitou Taipei 112 (TW); Huang, Yu-Chen, Peitou Taipei 112 (TW)
(74) Representative: Blasberg, Tilo

(56) References cited:
- WO-A-03/087066
- WO-A-2007/054776
- WO-A-2008/087514

## Description

### Field of the Invention

The present invention relates to novel cinamic compounds which are useful as agents for the prevention or treatment of diseases associated with histone deacetylase (HDAC). They also can be used as agents for enhancing the neurite outgrowth. In particular, they can be used as agents for anti-cancer, anti-diabetic, or anti-neurodegenerative diseases such as Alzheimer's disease, Huntington's disease, Spinocerebellar Ataxias (SCA) and human spinal muscular atrophy (SMA).

### Background of the Invention

Eukaryotic DNA is highly organized and packaged in the nucleus. The organization and packaging are achieved through the addition of proteins, including core histones H2A, H2B, H3 and H4, which form a complex structure, the chromatin, together with DNA. The modification of core histones is of fundamental importance to conformational changes of the chromatin. The level of acetylation is related to transcription activity, and then the acetylation induces an open chromatin conformation that allows the transcription machinery access to promoters. Histone deacetylase (HDAC) and histone acetyltransferase (HAT) are enzymes that influence transcription by selectively deacetylating or acetylating the ε-amino groups of lysine located near the amino termini of core histone proteins. HDACs are a family of 18 enzymes (isoforms) that may act as master regulators of many diseases, including cancer, because they are involved in the control of gene expressions. Disruption of HDACs has been linked to a wide variety of human cancers. HDAC enzymes or isoforms appear to be involved in many different types of cancer.

Histone deacetylase (HDAC) inhibitors are emerging as an exciting new class of potential anticancer agents for the treatment of solid and hematological malignancies. In recent years, an increasing number of structurally diverse HDAC inhibitors have been identified; they inhibit proliferation and induce differentiation and/or apoptosis of tumor cells in cultures and in animal models. HDAC inhibition causes acetylated nuclear histones to accumulate in both tumoral and normal tissues, providing a surrogate marker for the biological activity of HDAC inhibitors *in vivo.* The effects of HDAC inhibitors on gene expression are highly selective, leading to transcriptional activation of certain genes such as the cyclin-dependent kinase inhibitor p21^{WAF1/CIP1} but repression of others. HDAC inhibition results in acetylation of not only histones but also transcription factors such as p53, GATA-1 and estrogen receptor-alpha. The functional significance of acetylation of non-histone proteins and the precise mechanisms whereby HDAC inhibitors induce tumor cell growth arrest, differentiation and/or apoptosis are currently the focus of intensive research. HDAC inhibitors currently in clinical trials have shown activity and represent a class of molecularly targeted anti-tumor agents with potential for efficacy based on a novel mechanism of action.

A review article published in Medicinal Research Reviews, Vol. 26, No. 4, pp. 397-413, 2006 states that four classes of HDAC inhibitors, short-chain fatty acids, hydroxamic acids, benzamides and cyclic peptides, had been reported. Hydroxamic acid-based hybrid polar compounds (HPCs) are HDAC inhibitors, which induce differentiation at micromolar or lower concentrations (Journal of the National Cancer Institute, Vol. 92, No. 15, August 2, 2000, pp. 1210-1216). U.S. Pat. No. 6,174,905, EP 0847992, JP 258863/96, and Japanese Application No. 10138957 disclose that benzamide derivatives induce cell differentiation and inhibit HDAC. SNDX-275 (Entinostat) especially disclosed in Example 48 of U.S. Pat. No. 6,174,905 has become a candidate for cancer treatment drug. WO 01/38322 discloses additional compounds that serve as HDAC inhibitors. It is reported in Hum Genet, 2006, 120, pp. 101-110 that the benzamide M344 up-regulates SMN2 protein expression in fibroblast cells derived from SMA patients up to 7-fold after 64 hours of treatment. It is reported that sodium butyrate ameliorates phenotypic expression in a transgenic mouse model of spinal and bulbar muscular atrophy (Human Molecular Genetics, 2004, Vol. 13, No. 11, pp. 1183-1192). Trichostatin A, a histone deacetylase inhibitor, was found to induce ubiquitin-dependent cyclin D1 degradation in MCF-7 breast cancer cells (Molecular Cancer 2006, 5:8). U.S. Pat. No. 7,169,801 discloses that compounds having the formula of Z-Q-L-M or Z-L-M may be used to inhibit histone deacetylase. US Patent Number 6888027 covers a family of Sulphonamide HDAC inhibitors including PXD101. European Patent Number EP 1 301 184 covers the use of valproic acid and derivatives as HDAC inhibitors in the treatment of solid tumors. WO0222577 indicates that hydroxamate compounds are inhibitors of histone deacetylase and are useful aspharmaceuticals for the treatment of proliferative diseases. Moreover, the anti-diabetic activity of HDAC inhibitors is reported in the FASEB Journal, 2008, Vol. 22, pp. 944-945 and Diabetes, 2008, Vol. 57, pp. 860-867.

N,N'-hexamethylene bisacetamide (HMBA) is an effective inducer of differentiation in a number of transformed cell lines. U.S. Pat. Nos. 6,087,367 and RE38506 reports that a number of compounds related to HMBA with polar groups separated by apolar linkages on a molar basis, are as active or 100 times more active than HMBA. Furthermore, U.S. Pat. No. 7,399,787 reports that histone deacetylase inhibitors related to HMBA such as suberoylanilide hydroxamide acid (SAHA) have the ability to induce growth arrest, differentiation and/or apoptosis of tumor cells. In addition, Laurence Catley et al. reports that NVP-LAQ824 (a hydroxamic acid derivative) and NVP-LAQ824 (a derivative of 4-aminomethylcinnamic hydroxamic acid) are potent histone deacetylase inhibitors (Blood, 1 October 2003, Vol. 102, No. 7, pp. 2615-2622). George P et al. reports that LBH589 induces growth inhibition and regression in tumor cell lines by triggering apoptosis and LBH589 is now being tested in phase I clinical trials as an anticancer agent (Blood 105(4): 1768-76 February 15, 2005). Other histone deacetylase inhibitors known in the art include pyroxamide, M-carboxycinnamic acid bishydroxamide (CBHA), trichostatin A (TSA), trichostatin C, salicylihydroxamic acid (SBHA), azelaic bishydroxamic acid (ABHA), azelaic-1-hydroxamate-9-anilide (AAHA), 6-(3-chlorophenylureido) carpoic hydroxamic acid (3C1-UCHA), oxamflatin, A-161906, scriptaid, PXD-101, cyclic hydroxamic acid-containing peptide (CHAP), ITF-2357, MW2796, MW2996, trapoxin A, FR901228 (FK 228 or Depsipeptide), FR225497, apicidin, CHAP, HC-toxin, WF27082, chlamydocin, sodium butyrate, isovalerate, valerate, 4-phenylbutyrate (4-PBA), 4-phenylbutyrate sodium (PBS), arginine butyrate, propionate, butyramide, isobutyramide, phenylacetate, 3-bromopropionate, tributyrin, valproic acid, valproate, CI-994, 3'-amino derivative of MS-27-275, MGCD0103 and Depudecin (U. S. Publication No. 20080242648). From WO 2007/054776 stilbene-like compounds as HDAC inhibitors are known. From WO 2008/087514 3-phenylacryl amide derivatives are known as HDAC inhibitors, wherein the phenyl group may be m-substituted by a heterocyclic group, a carboxylic group or an alkyl group. WO 03/087066 discloses alpha, Beta-unsaturated hydroxamic acid derivatives and their use as histone deacetylase inhibitors.

However, there is still a need to develop a new class of HDAC inhibitors to prevent or treat cancers and other diseases involving HDAC.

### Summary of the Invention

The object of the invention is to provide a group of compounds represented by the following formula (I): 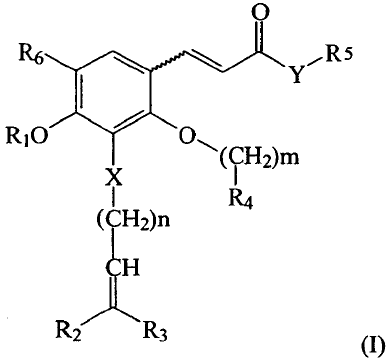 and pharmaceutically acceptable salts, stereoisomers, enantiomers and solvates thereof. The compounds are useful as an agent for enhancing the neurite outgrowth and preventing or treating diseases associated with HDAC

### Brief Description of the Drawings

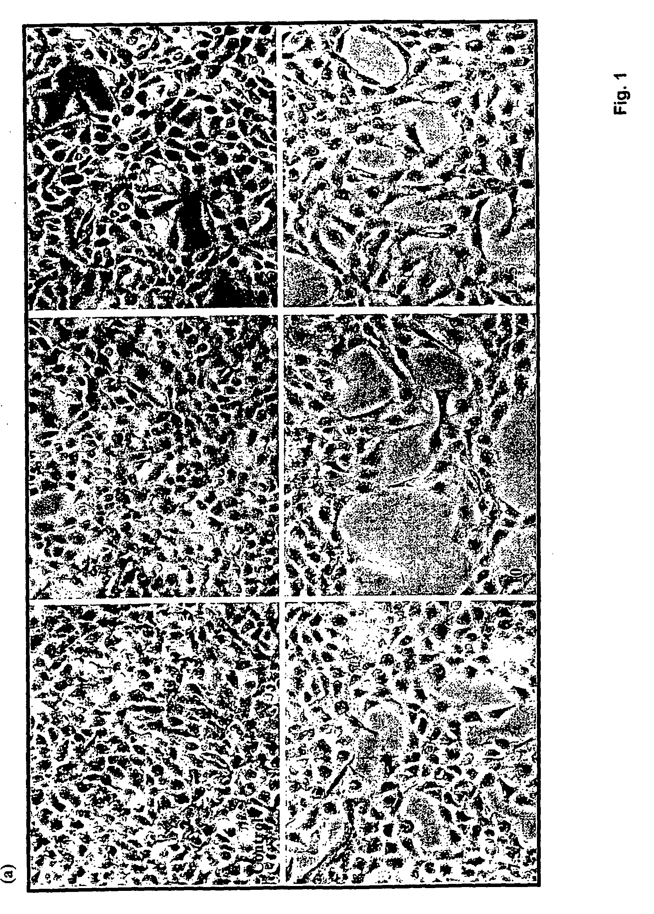

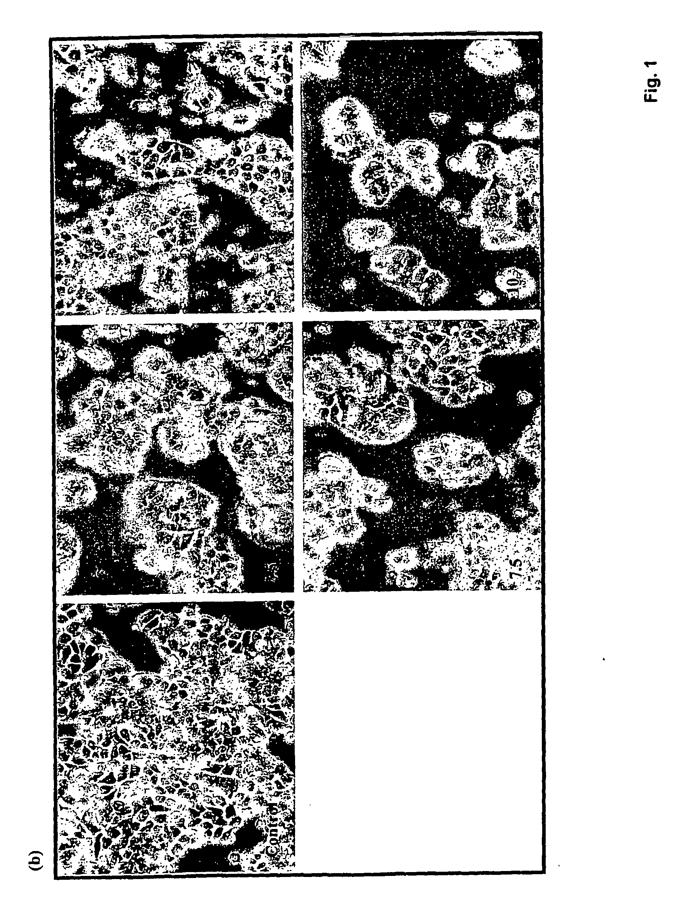

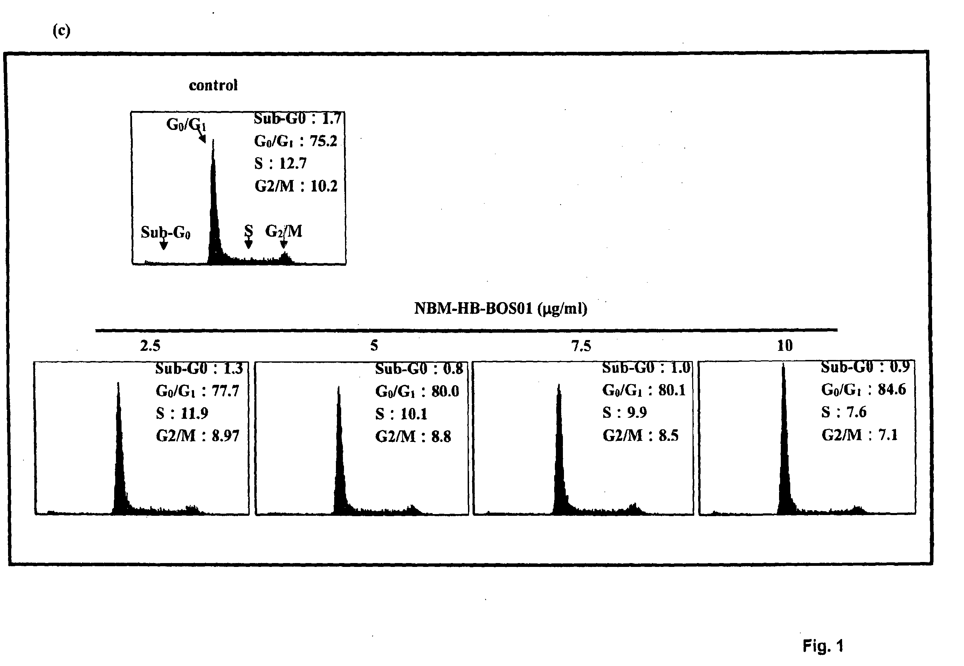

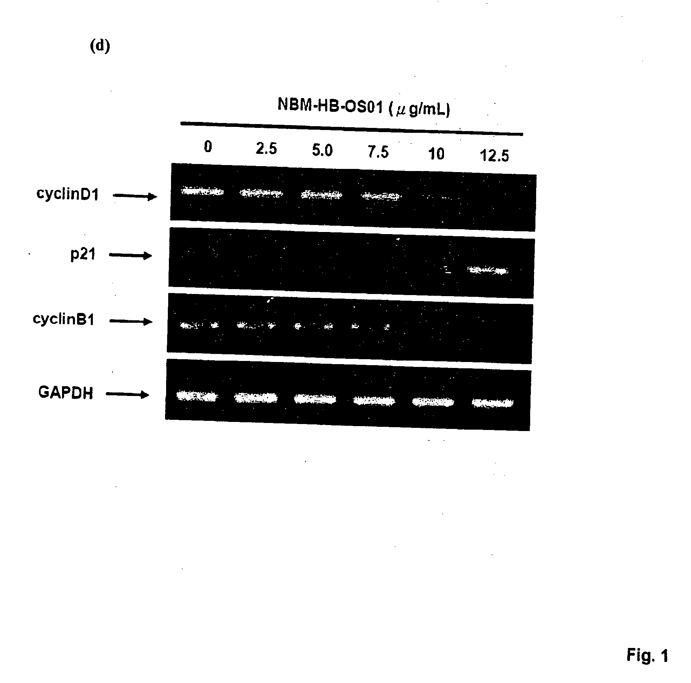

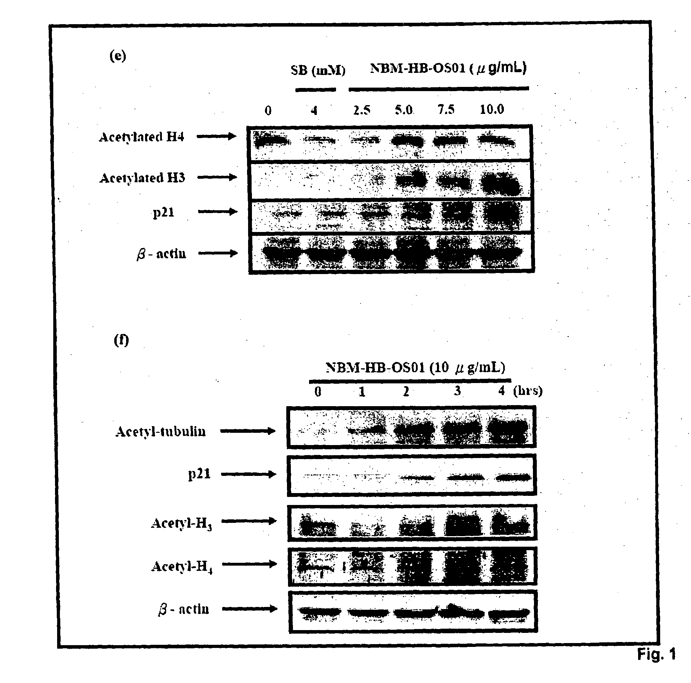

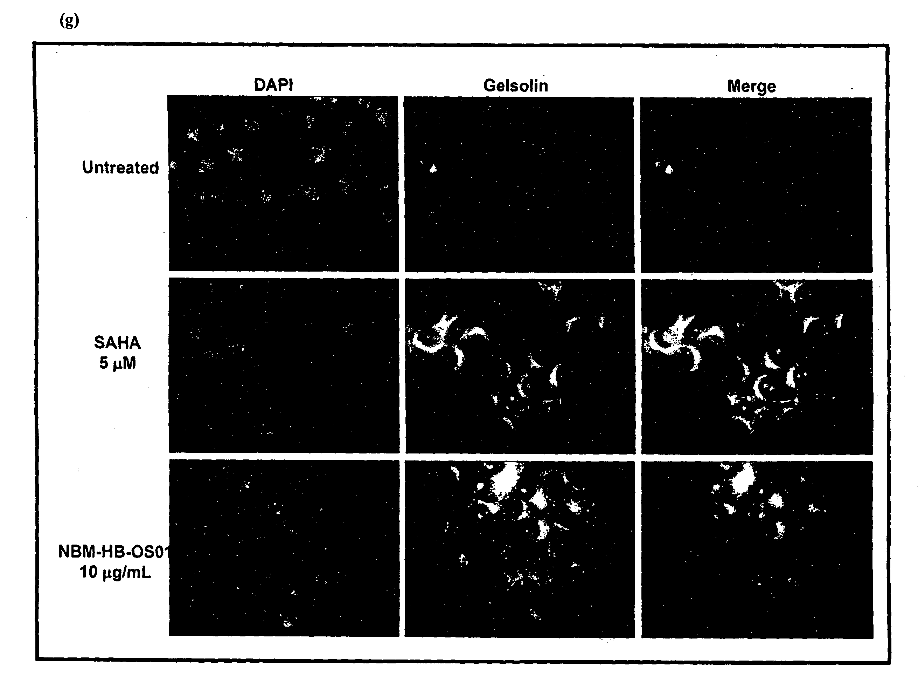

Figure 1 shows the effects of NBM-HB-OS01(**2h**) on the inhibition of cells growth of various cancer cell lines. Inhibition of cancer cells was shown in Rat C6 glioma cells and Human colon cancer HT-29 cells after they were treated with various concentrations of NBM-HB-OS01 for 48 hrs and the results are shown in Figs. 1(a) and (b), respectively. Fig. 1(c) shows the results of a flow analysis of A549 treated with various concentrations of NBM-HB-OS01 for 24 hrs. Fig. 1(d) shows that NBM-HB-OS01 increased p21 gene expression. Rat C6 glioma cells were treated with NBM-HB-OS01 for 48 hrs and GAPDH served as an internal control. Fig. 1(e) shows the results of treatment of Rat C6 glioma cells with various concentrations of NBM-HB-OS01 for 72 hrs. Accumulation of hyperacetylated histone H3, hyperacetylated histone H4 and p21 was detected in a dose-dependent manner by western blotting. Fig. 1(f) shows the results of treatment of Rat C6 glioma cells with 10 µg/mL NBM-HB-OS01 for 1, 2, 3 and 4 hrs. The accumulation of acetylated H3, acetylated H4, acetylated-tubulin and p21 was seen in a dose-dependent manner. β-actin was the internal control. Fig. 1(g) shows the results of ,treatment of Human breast cancer MCF-7 (Estrogen Receptor positive) cells with 10 µg/mL NBM-HB-OS01 for 24 hrs. Overexpression of Gelsolin protein was observed.

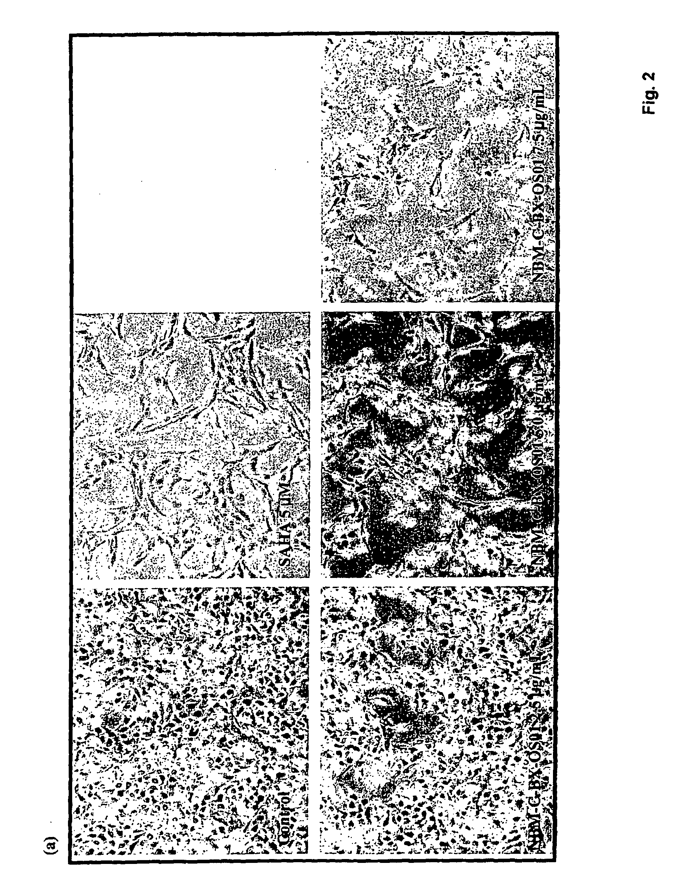

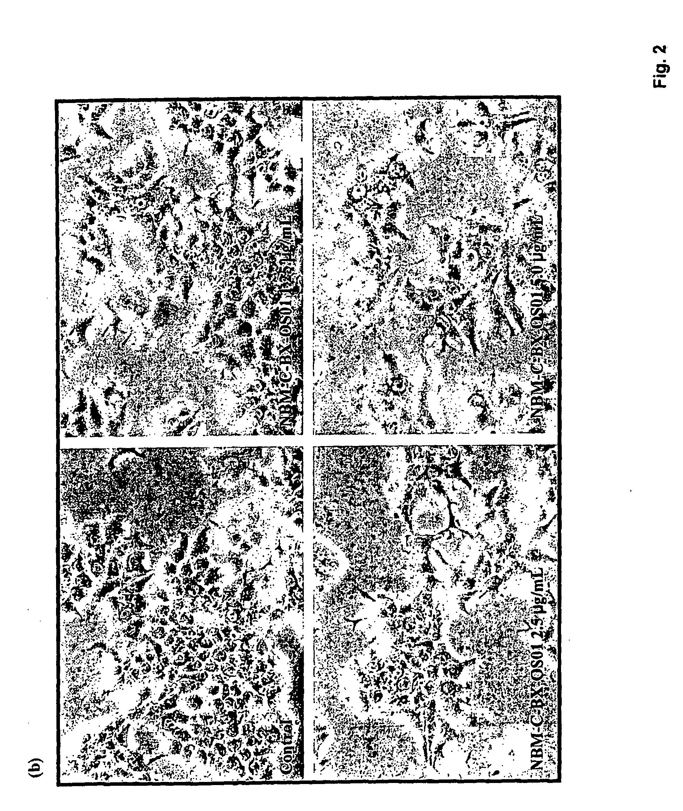

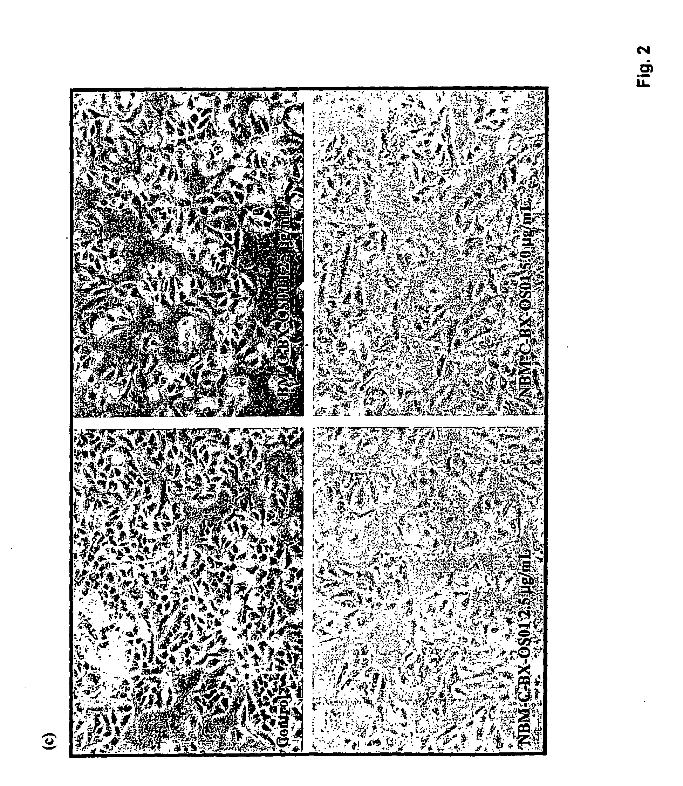

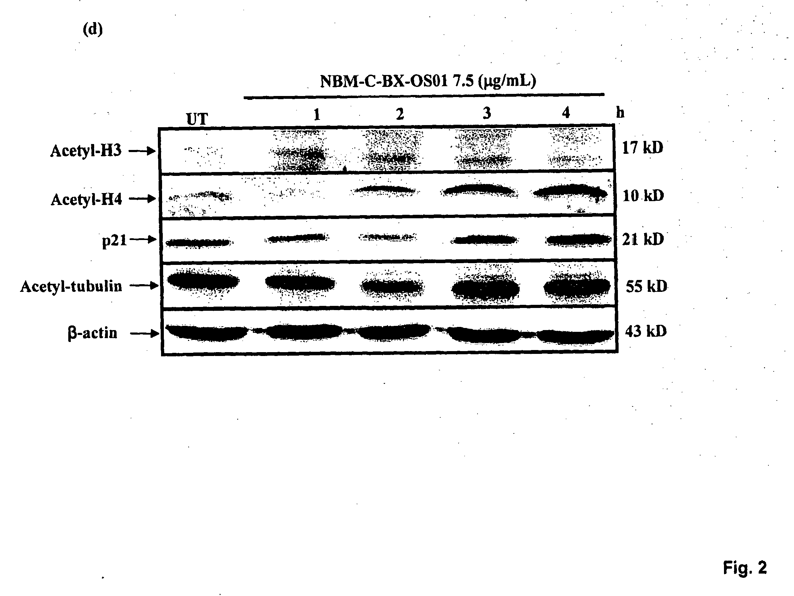

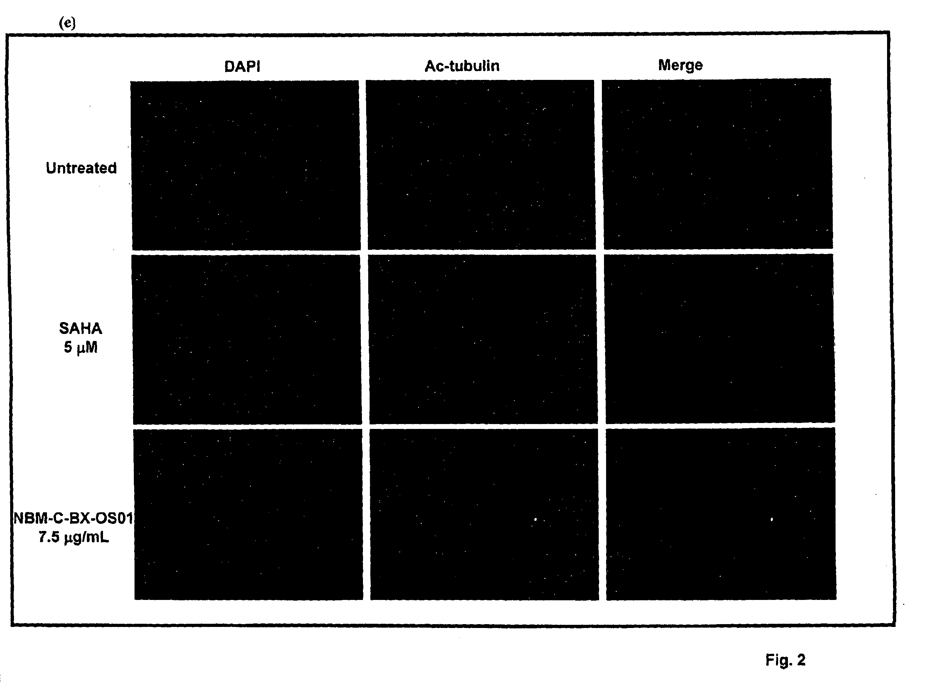

Figure 2 shows the effects of NBM-C-BX-OS01 (**4a**) on the inhibition of cells growth of various cancer cell lines. Inhibition of cancer cells growth was shown after treating cell lines with various concentrations of NBM-C-BX-OS01 in Rat C6 glioma cells, Human breast cancer MCF-7 cells for 24 hrs, and Human lung cancer A549 cells for 48 hrs. The results are shown in Figs. 2(a), (b) and (c), respectively. Fig. 2(d) shows the results of treatment of MCF-7 cells with 10 µg/mL NBM-C-BX-OS01 for 1, 2, 3 and 4 hrs. The expression of acetylated H3, acetylated H4, acetylated-tubulin, and p21 increased in a dose-dependent manner. β-actin was the internal control. Fig. 2(e) shows the results of treatment of Rat C6 glioma cells with NBM-C-BX-OS01 of 7.5 µg/mL for 6 hrs. Overexpression of acetylated tubulin protein was observed.

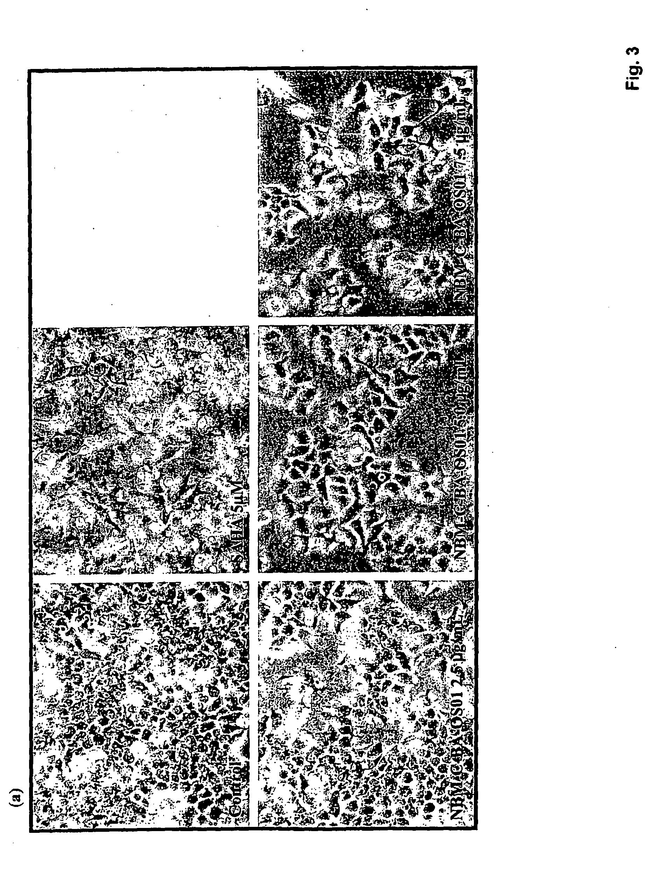

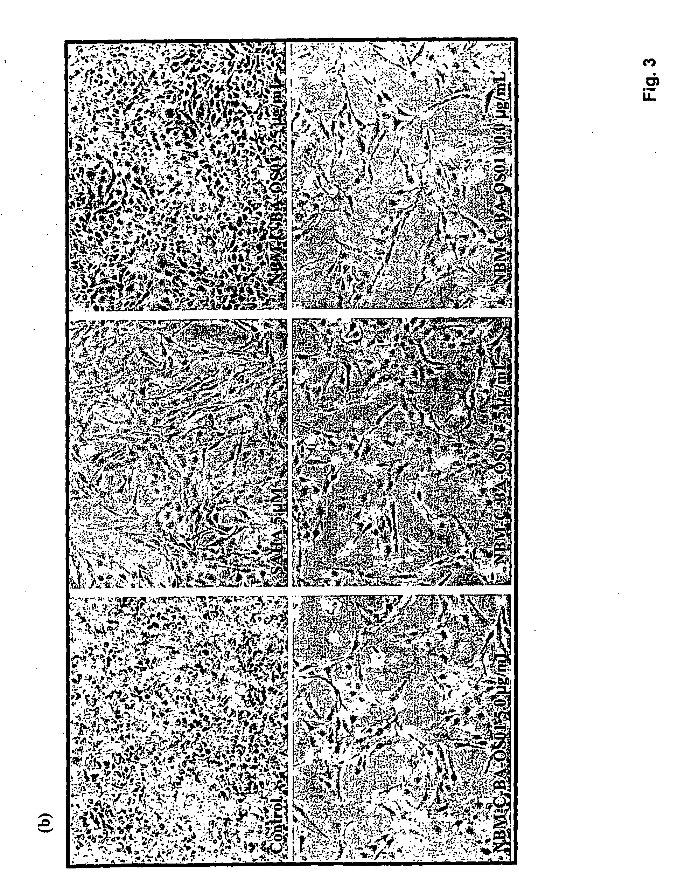

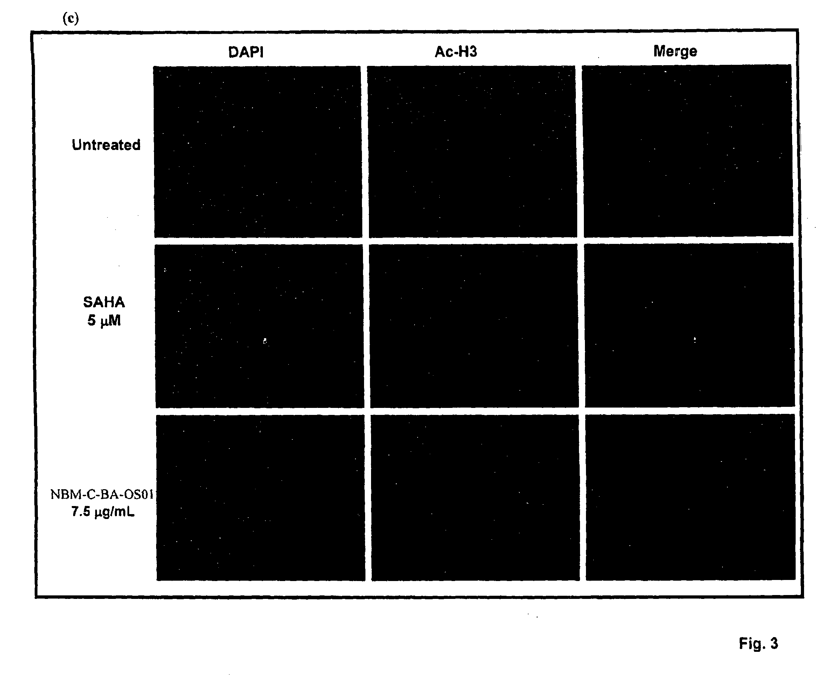

Figure 3 shows the effect of NBM-C-BA-OS01 (**17a**) on the inhibition of cells growth of three cancer cell lines. Morphology changes of Human breast cancer MCF-7 cells and Rat C6 glioma cells in response to NBM-C-BA-OS01 (2.5, 5.0, 7.5 µg/mL) for 72 hrs are shown in Figs 3(a) and (b). Hyperacetylation of Histone H3 protein was detected by treating Human lung cancer A549 cells for 6 hrs with NBM-C-BA-OS01 of 7.5µg/mL. The results are shown in Fig 3(c).

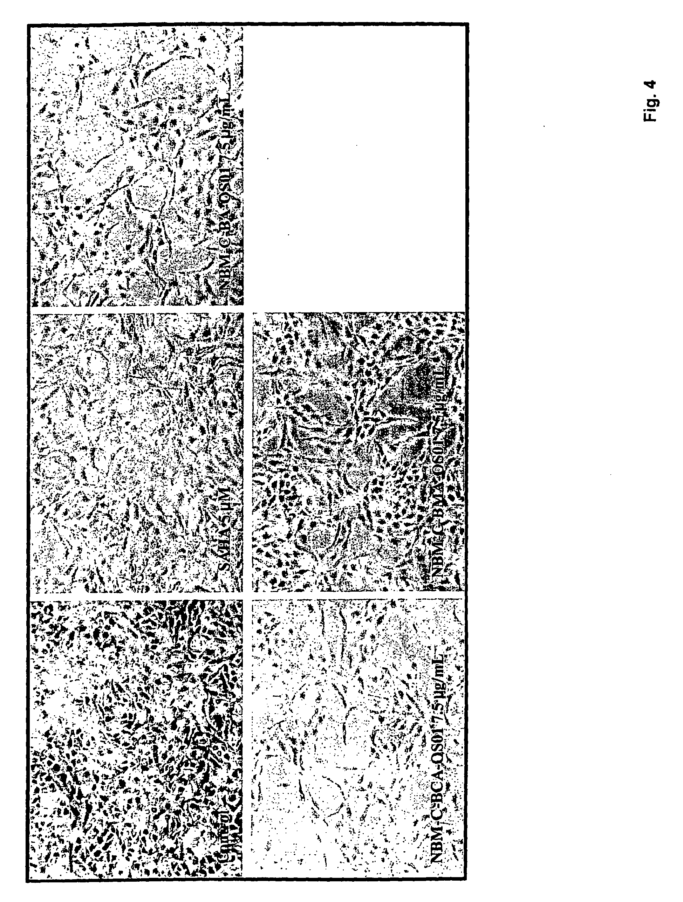

Figure 4 shows the cell growth inhibition by the treatment of various NBM-C-BA-OS01 derivatives. Treatment of NBM-C-BA-OS01, NBM-C-BCA-OS01 (**17d**), and NBM-C-BMA-OS01 (**17b**) of 7.5 µg/mL inhibited the Rat C6 glioma cells growth in 48 hrs.

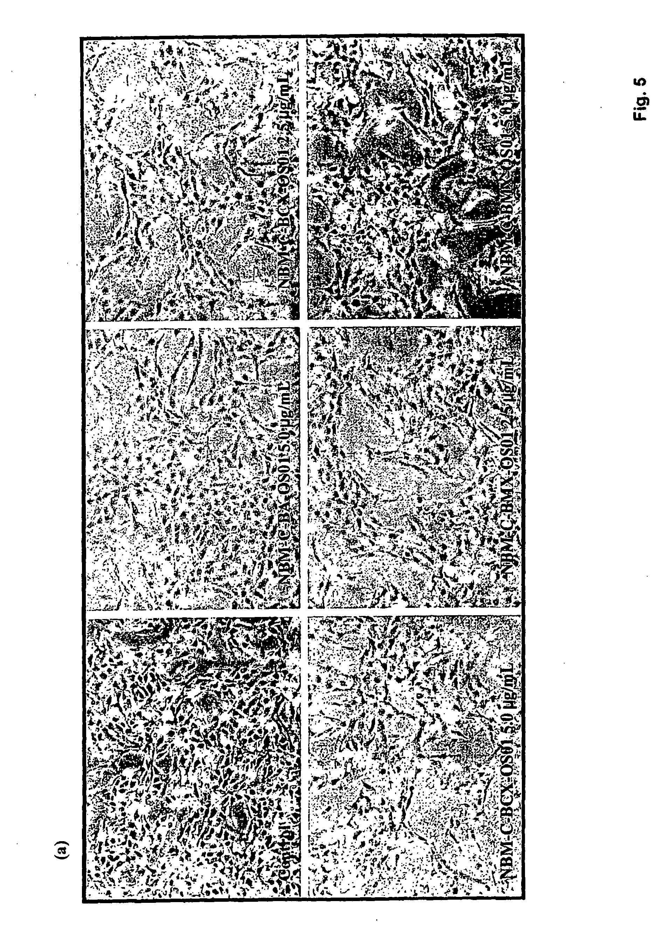

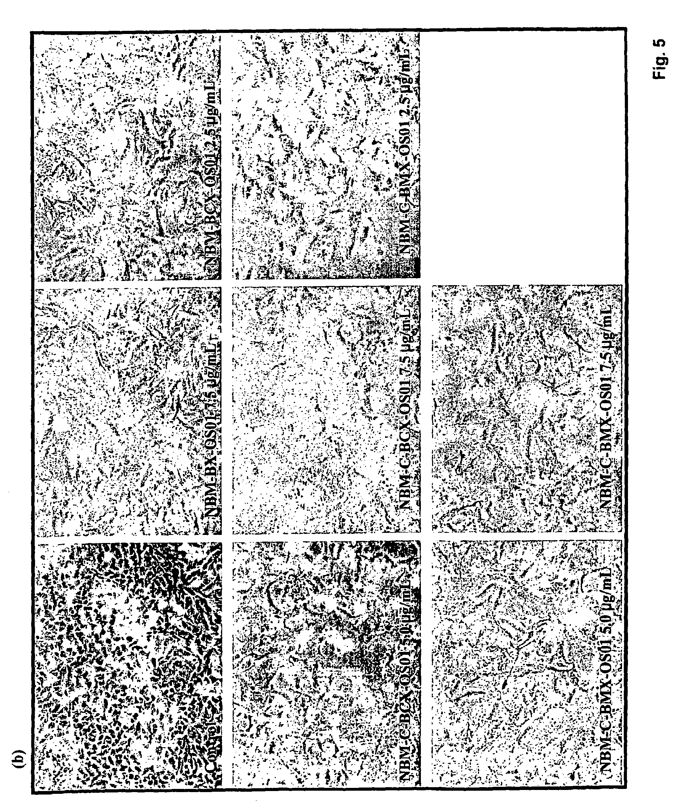

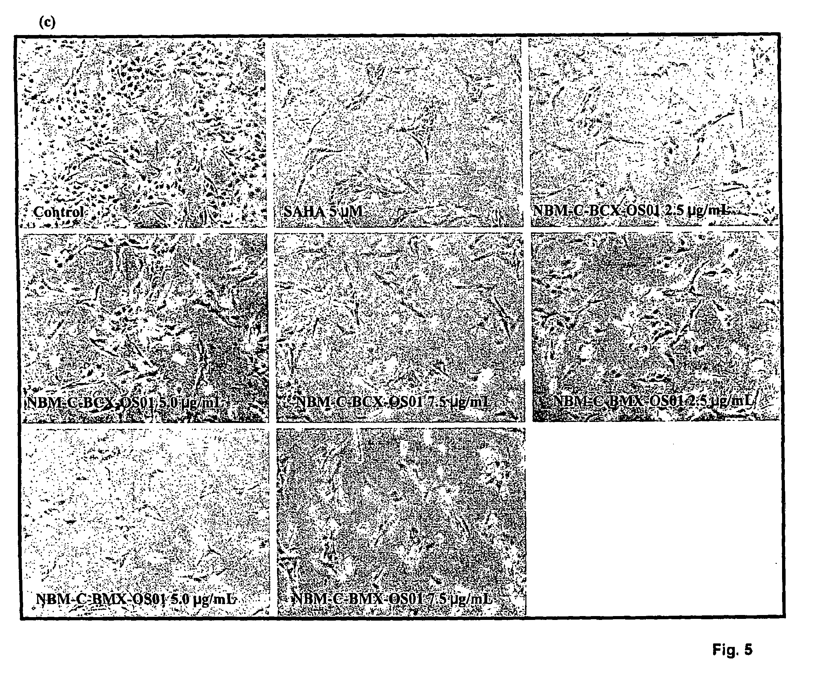

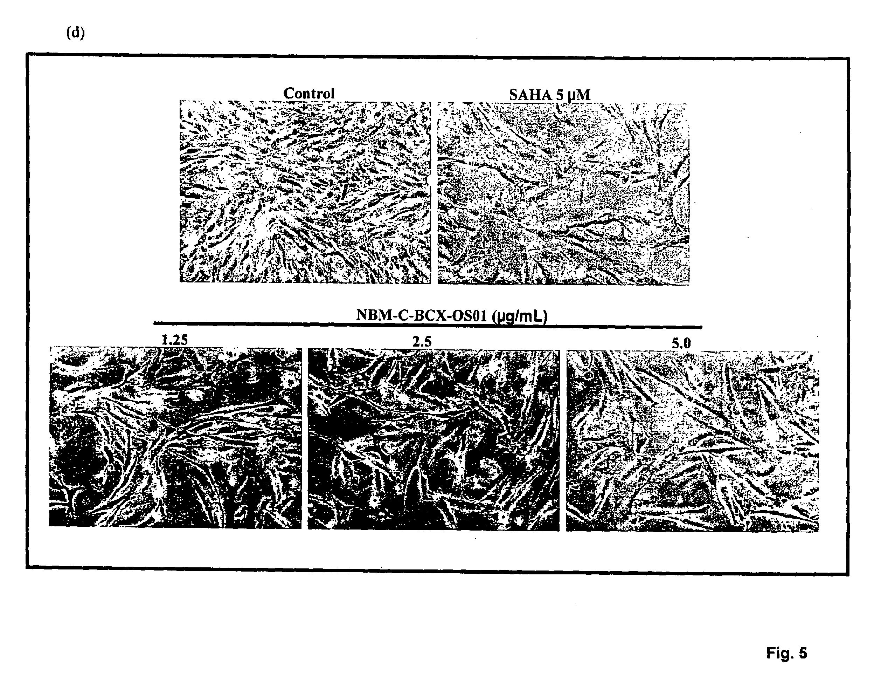

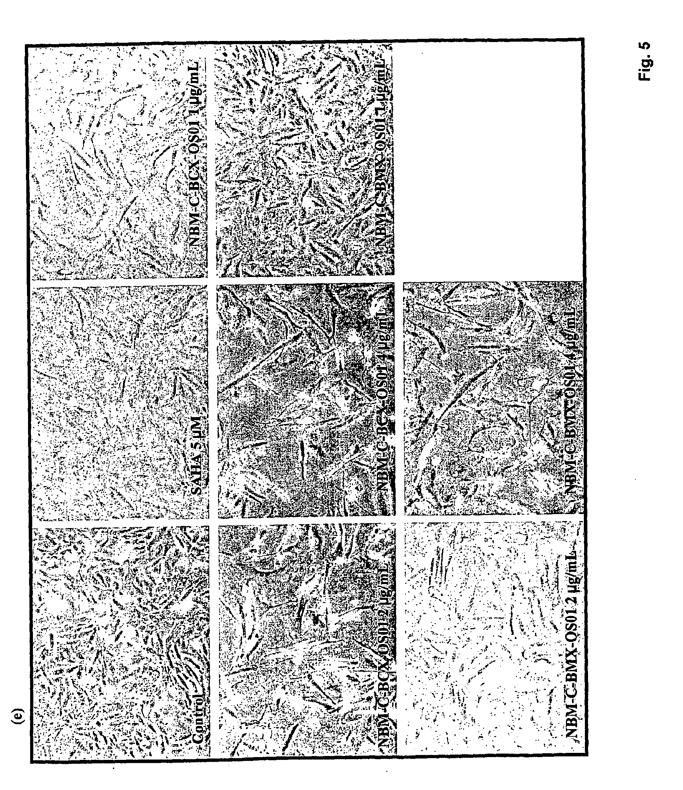

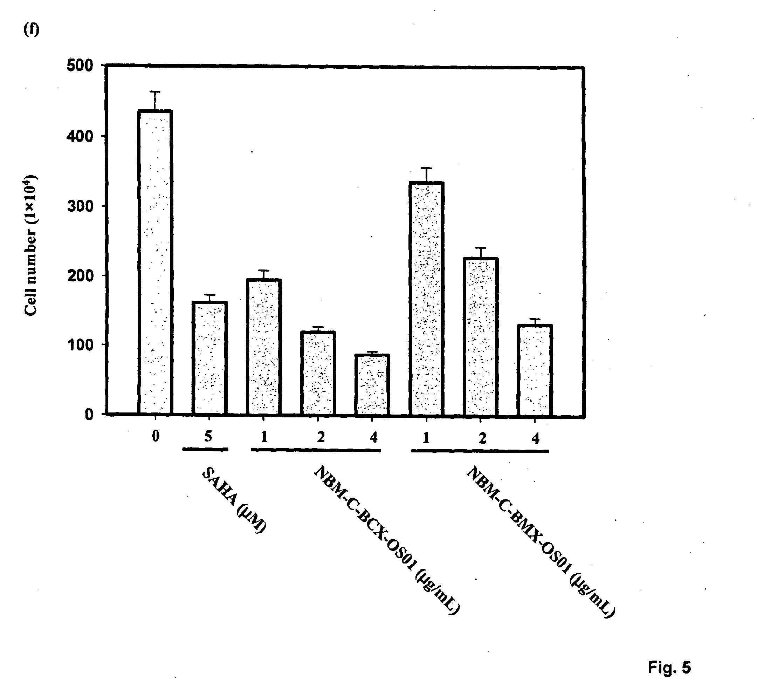

Figure 5 shows the effect of NBM-HB-OS01 derivatives on the cell growth inhibition. Fig. 5(a) shows that NBM-C-BA-OS01 (5µg/mL), NBM-C-BCX-OS01 (**4d**) (2.5, 5.0 µg/mL), and NBM-C-BMX-OS01 (**4b**) (2.5, 5.0µg/mL) inhibited the cell growth of Rat C6 glioma cells in 24 hrs. Fig. 5(b) shows that treatment of NBM-C-BX-OS01 (7.5µg/mL), NBM-C-BCX-OS01 (2.5, 5.0, 7.5 µg/mL) and NBM-C-B11X-OS01 (2.5, 5.0, 7.5 µg/mL) for 72 hrs induced A549 cells growth inhibition in a dose-dependent manner. Similar results can be seen in treatment of Rat C6 glioma cells with NBM-C-BCX-OS01 and NBM-C-BMX-OS01 of various concentrations for 24 hrs, as shown in Fig. 5(c). Fig. 5(d) shows the results of treatment of Human glioma Hs683 cells with NBM-C-BX-OS01 (1.25, 2.5, 5.0 µg/mL) for 72 hrs and in Fig. 5(e) shows the results of treatment of Human glioblastoma 05-MG cells with NBM-C-BCX-OS01 (1.0, 2.0, 4.0 µg/mL), and NBM-C-BMX-OS01 (1.0, 2.0, 4.0 µg/mL) for 72 hrs. Cell growth inhibition was observed. The treated cells counted by a trypan blue exclusion assay are plotted in Fig. 5(f).

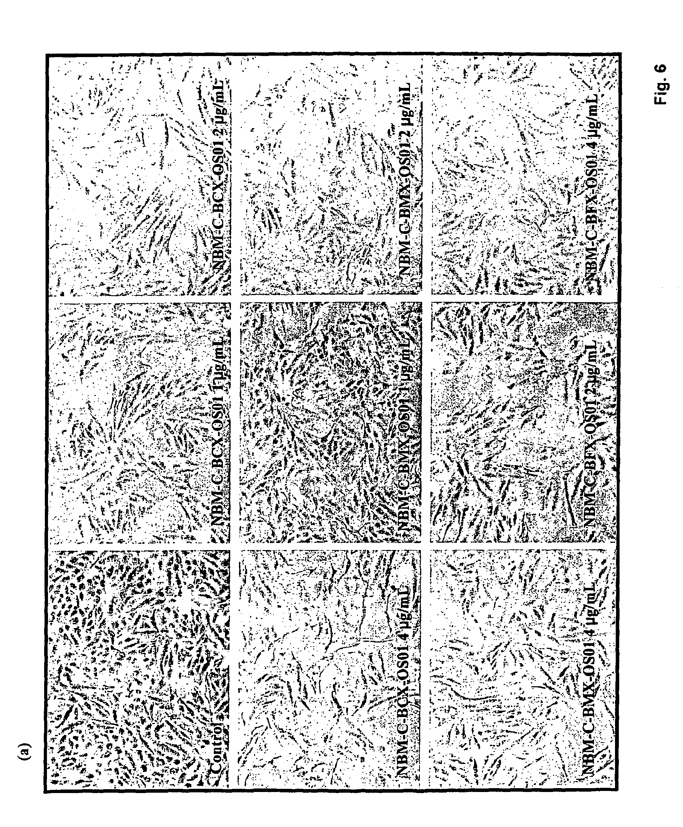

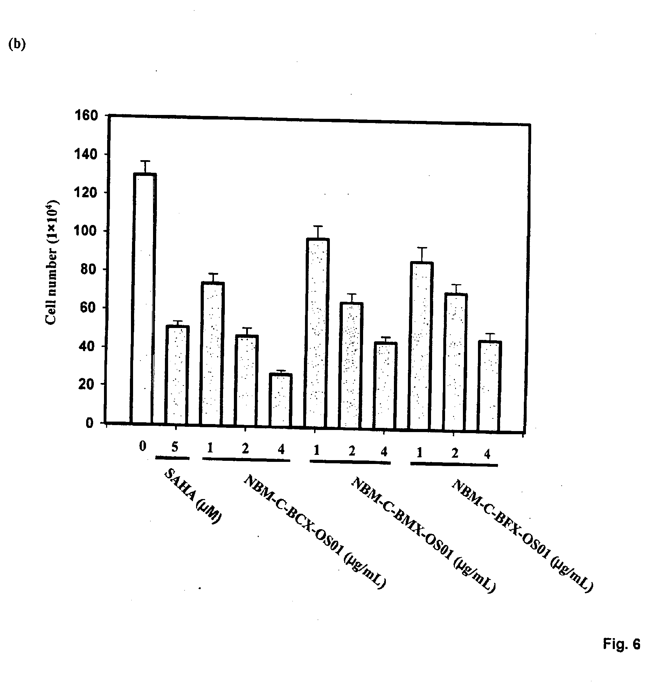

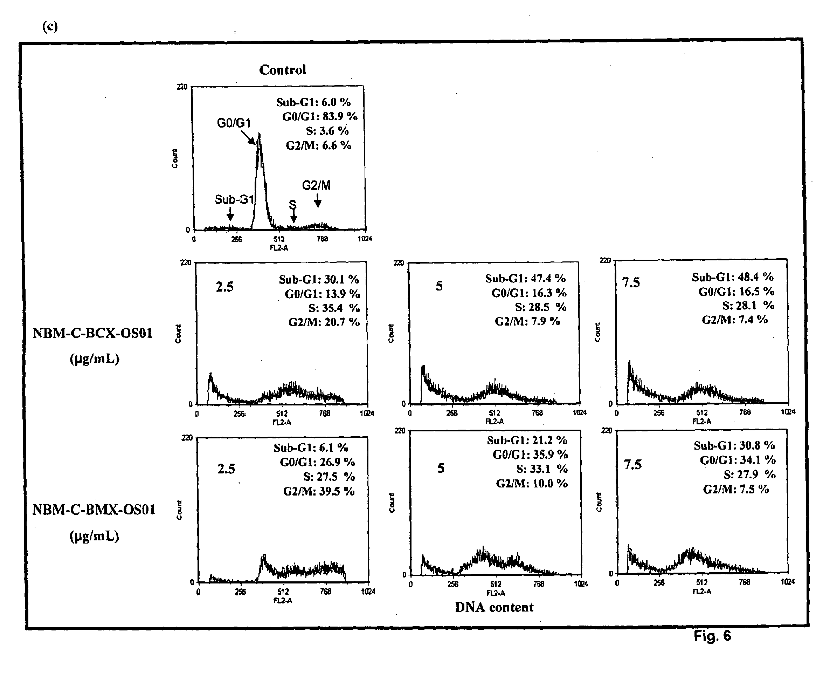

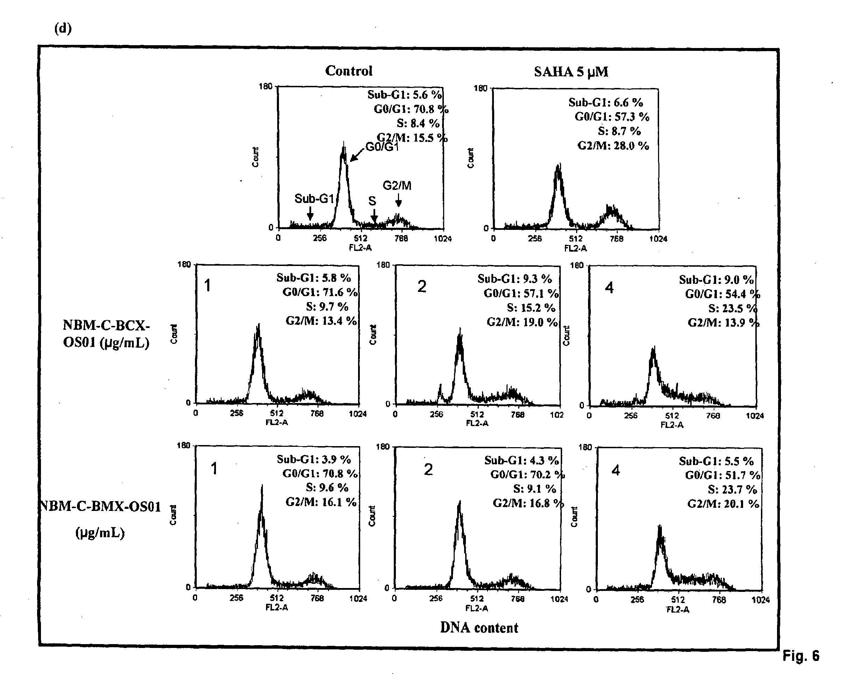

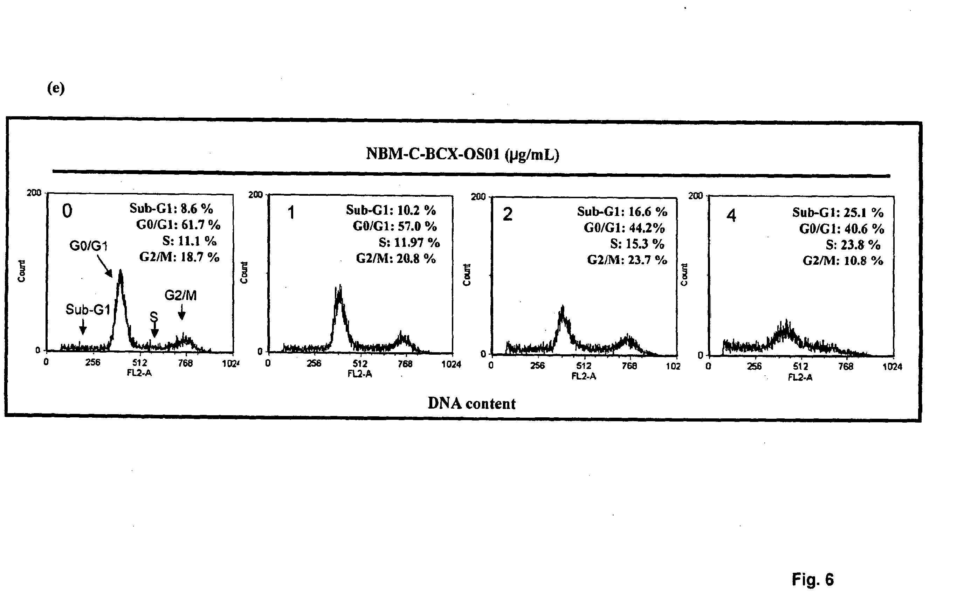

Figure 6 shows the effects of NBM-HB-OS01 derivatives on the biological activity in various human cancer cell lines. As shown in Fig. 6(a), inhibition of the cell growth of human breast cancer MDA-MB-231 cells was observed after treating the cells with various concentrations of NBM-C-BCX-OS01, NBM-C-BMX-OS01, and NBM-C-BFX-OS01 (4c) for 72 hrs. The NBM-HB-OS01 derivatives significantly inhibited the growth of human breast cancer MDA-MB-231 cells. The treated cells counted by a trypan blue exclusion assay are shown in Fig. 6(b). Figs. 6(c) and (d) shows the results of treatment of Human lung cancer A549 cells and Human glioblastoma 05-MG cells with NBM-C-BCX-OS01 and NBM-C-BMX-OS01 for 72 hrs, respectively. Fig. 6(e) shows that NBM-C-BCX-OS01 arrested the growth of Human glioma Hs683 cells. Human glioma Hs683 cells were treated with NBM-C-BCX-OS01 (1.0, 2.0, 4.0 µg/mL) for 72 hrs.

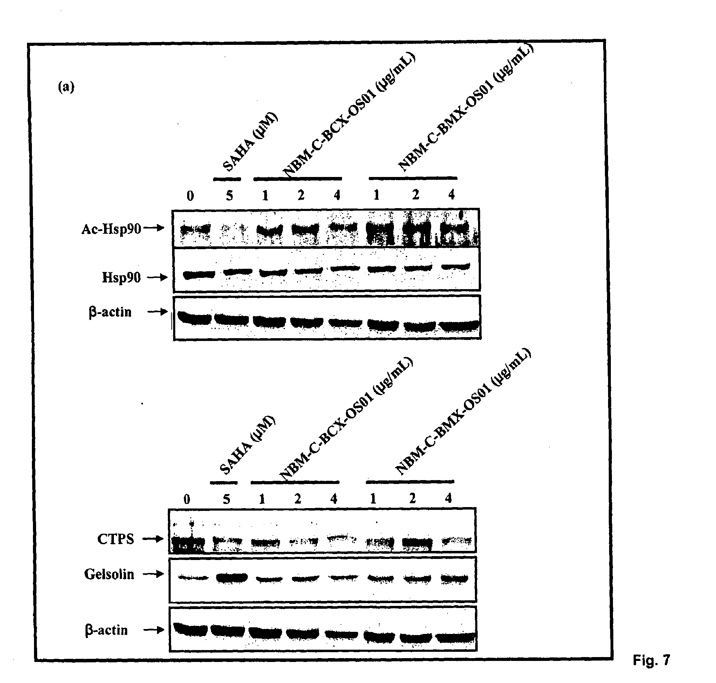

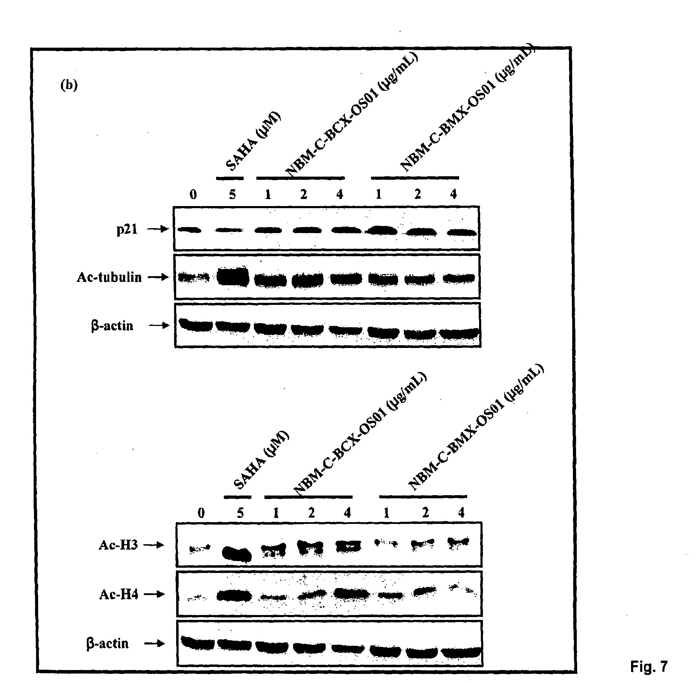

Figure 7 shows the effects of NBM-C-BCX-OS01 and NBM-C-BMX-OS01 on histones and associated proteins. Human glioma Hs683 cells were treated with various concentrations of NBM-C-BCX-OS01 and NBM-C-BMX-OS01 for 72 hrs. As shown in Fig. 7(a), the induction of acetylated Hsp90 and gelsolin proteins was detected in a dose-dependent manner, i.e., Hsp90 and CTPS proteins decreased in a dose-dependent manner. It can be observed in Fig. 7(b) that the expression of p21, acetylated tubulin, acetylated Histone H3, and acetylated Histone H4 was significantly increased in a dose-dependent manner. SAHA was used as a positive control and β-action as an internal control.

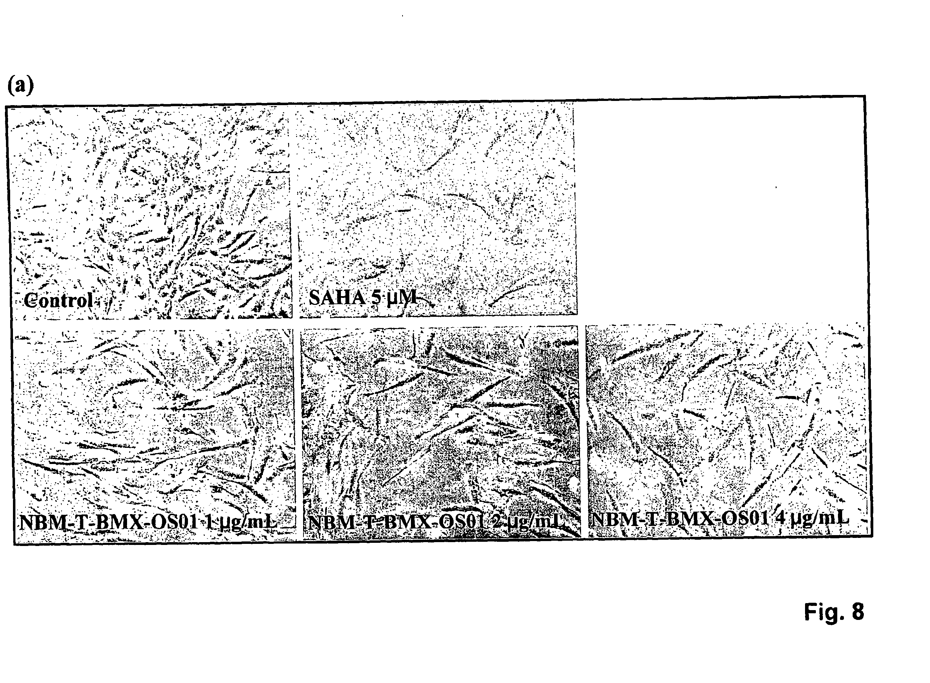

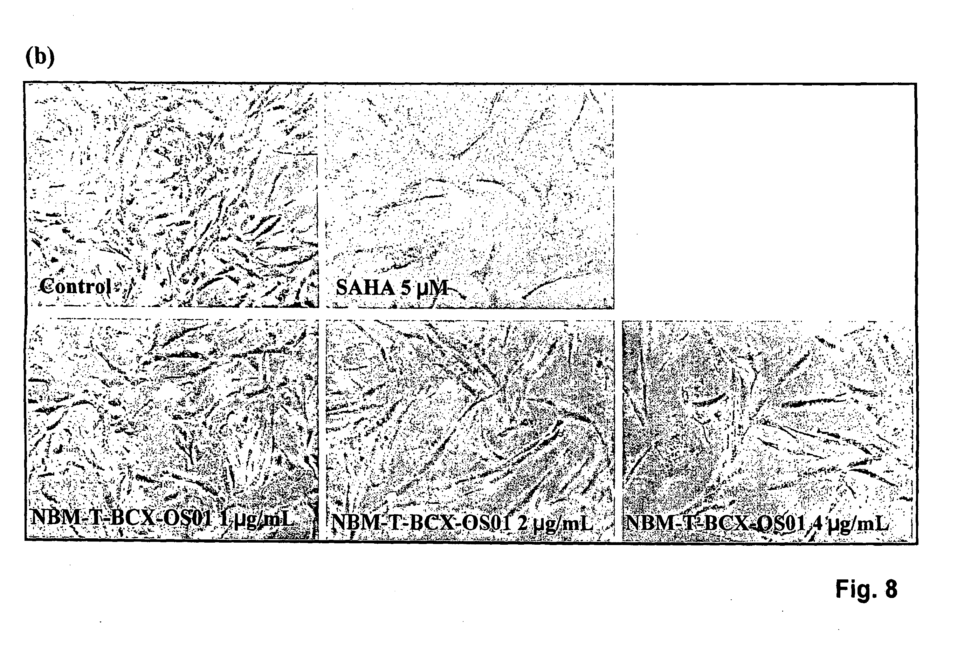

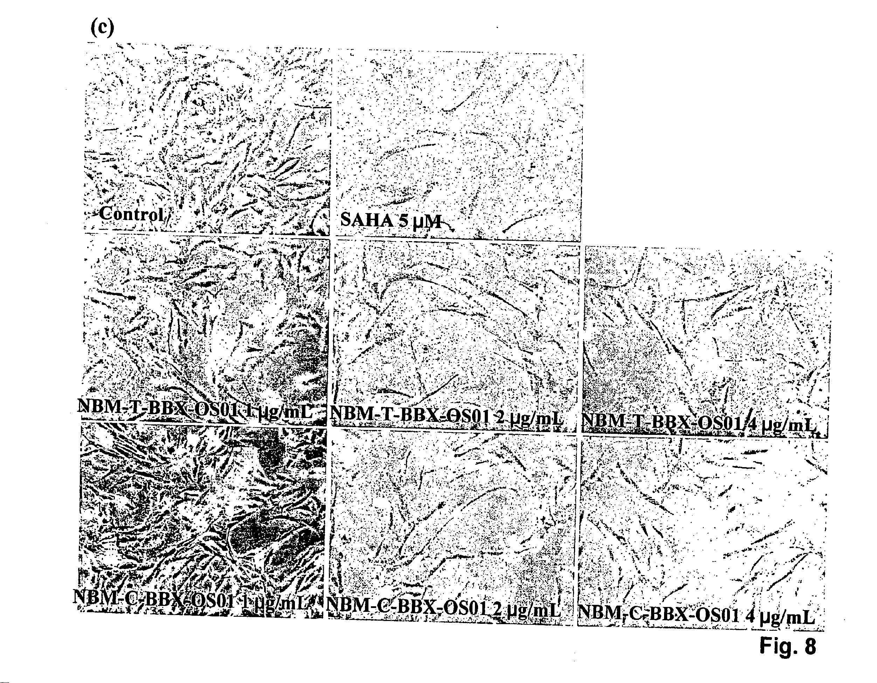

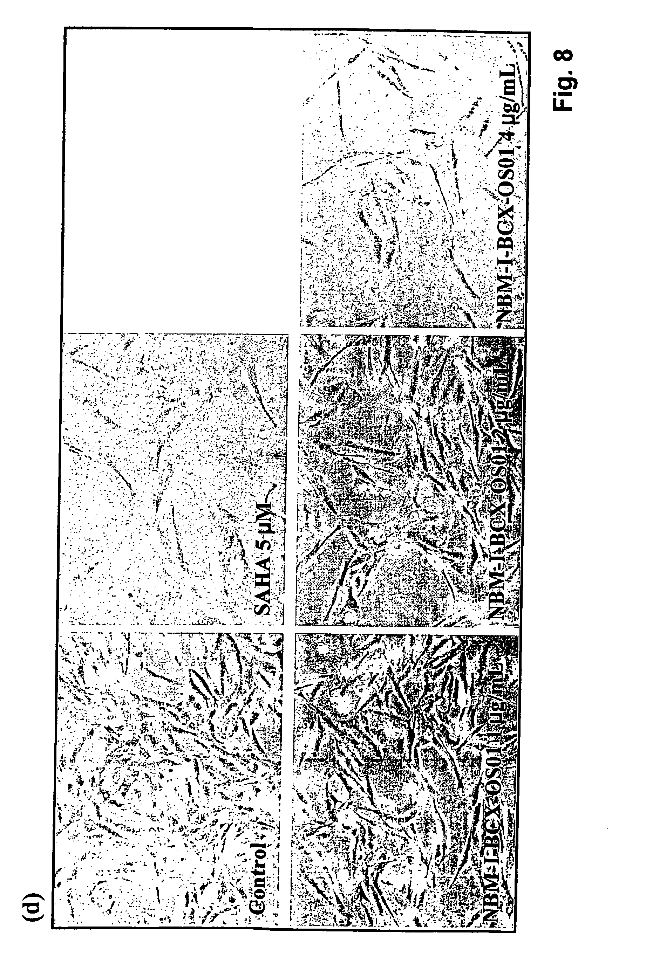

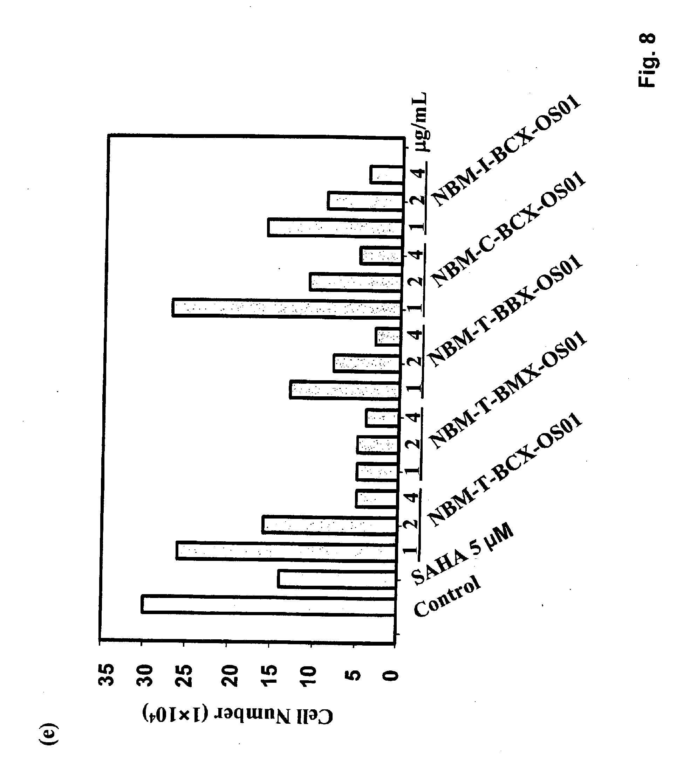

Figure 8 shows the effects of: (a) NBM-T-BMX-OS01 (**4b**), (b) NBM-T-BCX-OS01 (4d), (c) NBM-T-BBX-OS01 **(4e)** and NBM-C-BBX-OS01 **(4e),** and (d) NBM-I-BCX-OS01 on the inhibition of cell growth (see Figs. 8(a) to (d)). Human glioma Hs683 cells were grown in the presence of the above compounds of various concentrations (1.0, 2.0 and 4.0 µg/mL) for 72 hours. The treated cells counted by a trypan blue exclusion assay are plotted in Fig. 8(e).

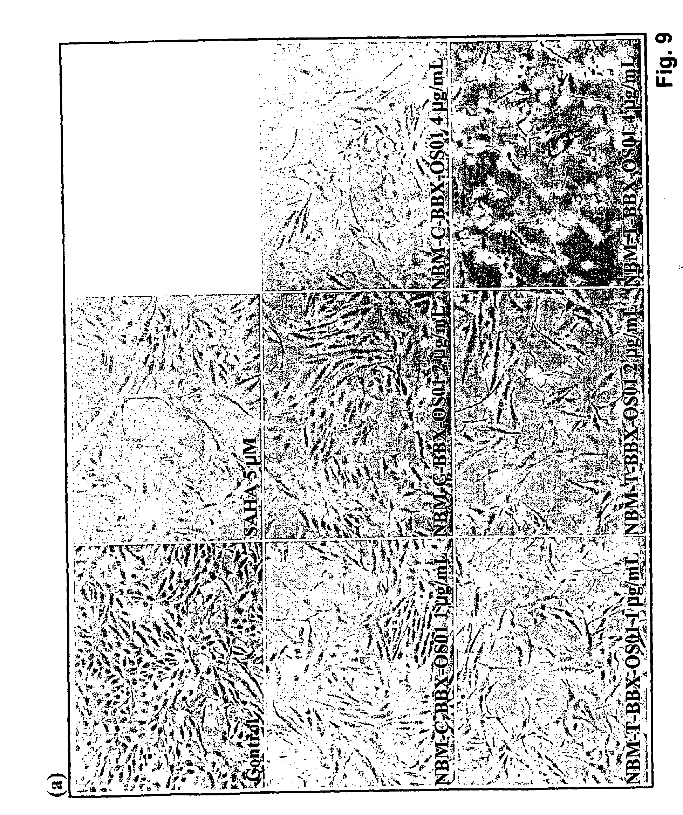

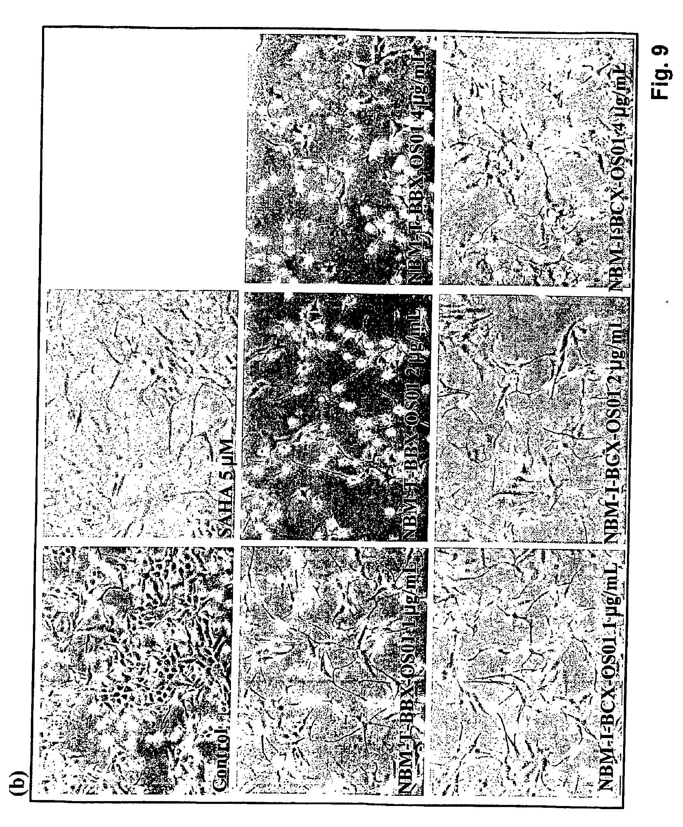

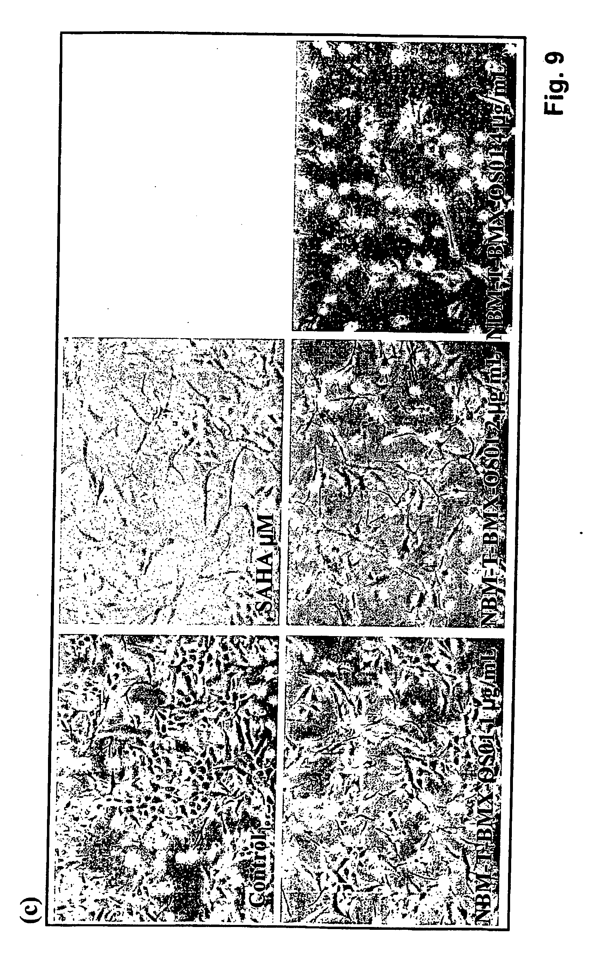

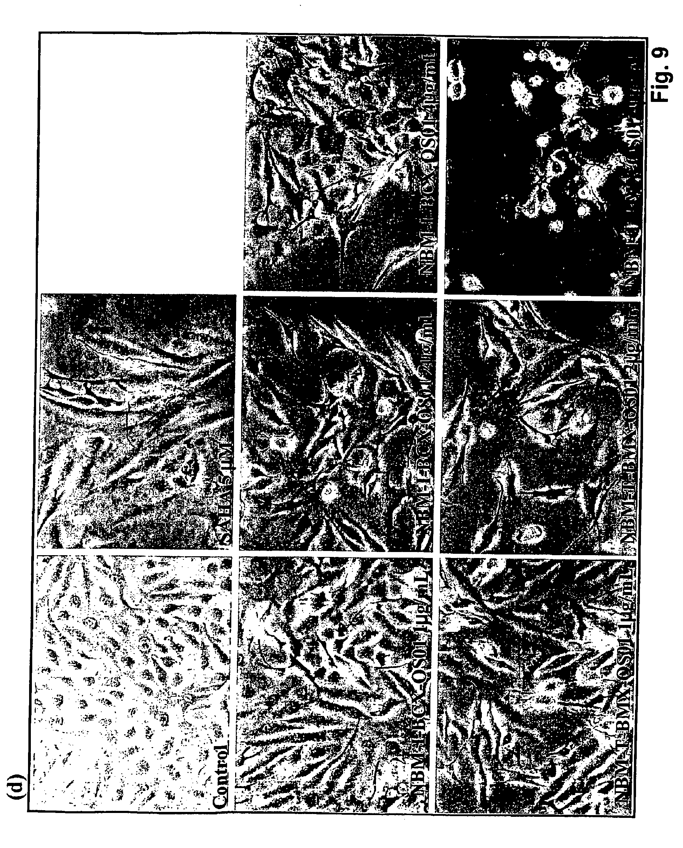

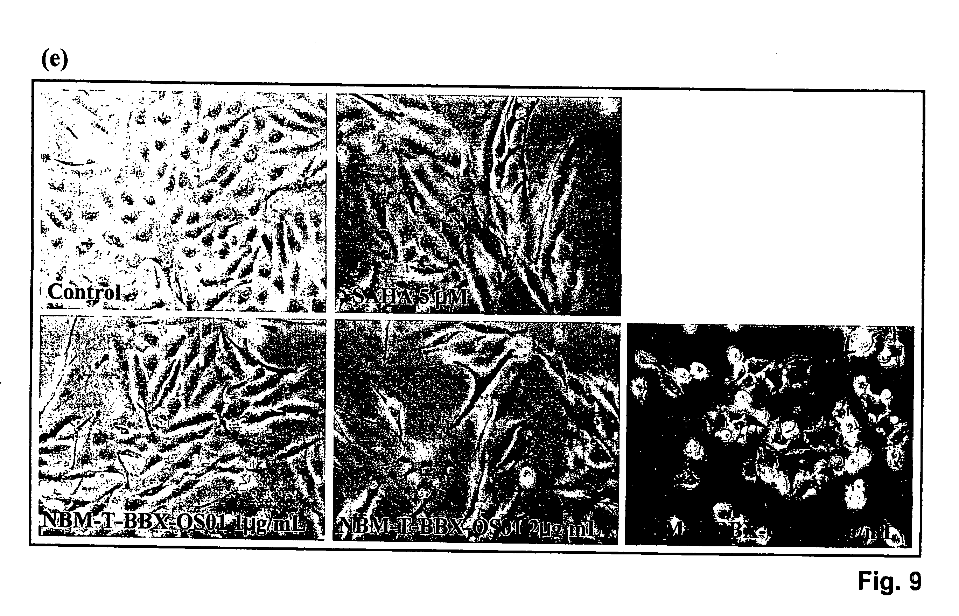

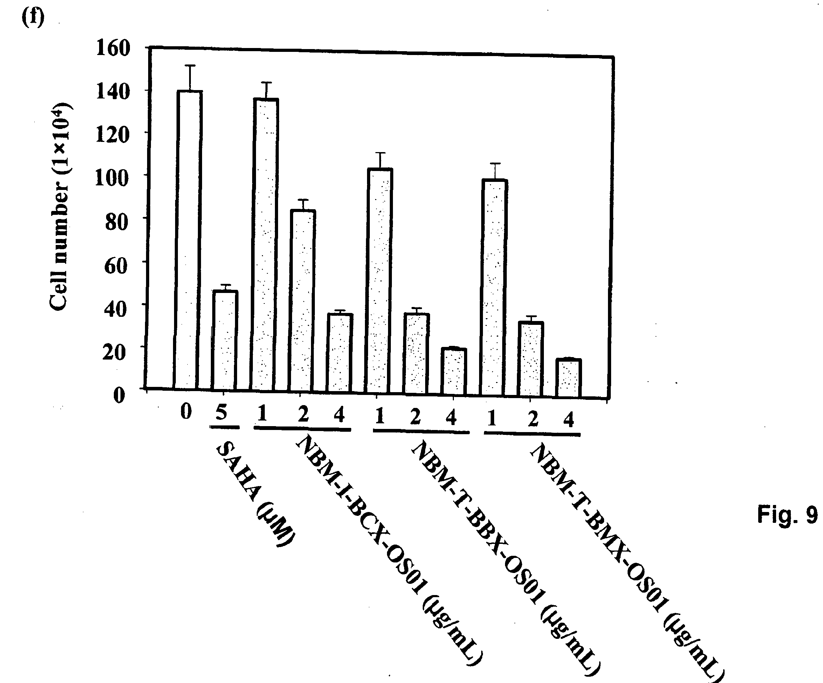

Figure 9 shows the effects of (a) NBM-C-BBX-OS01 and NBM-T-BCX-OS01, (b) NBM-T-BBX-OS01 and NBM-I-BCX-OS01, (c) NBM-T-BMX-OS01, (d) NBM-I-BCX-OS01 and NBM-T-BMX-OS01 and (e) NBM-T-BBX-OS01 on the inhibition of human breast cancer MDA-MB-231 cells (see Figs 9(a) to (e)). The results of trypan blue exclusion assay are shown in Fig. 9(f).

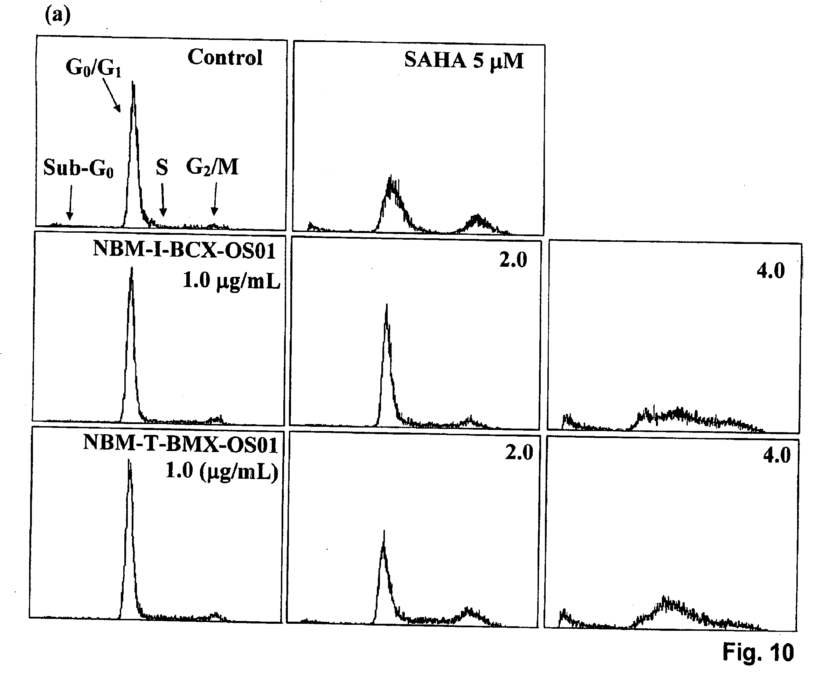

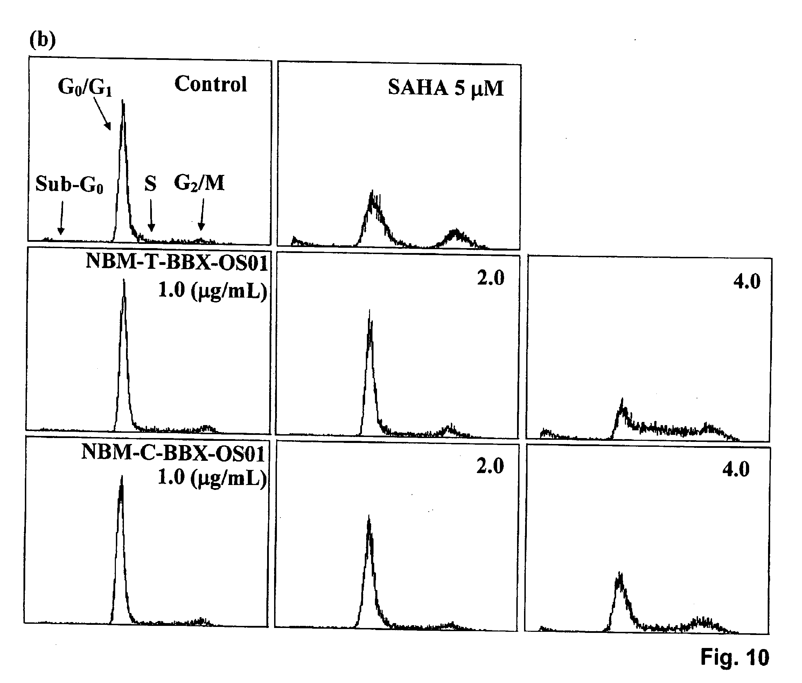

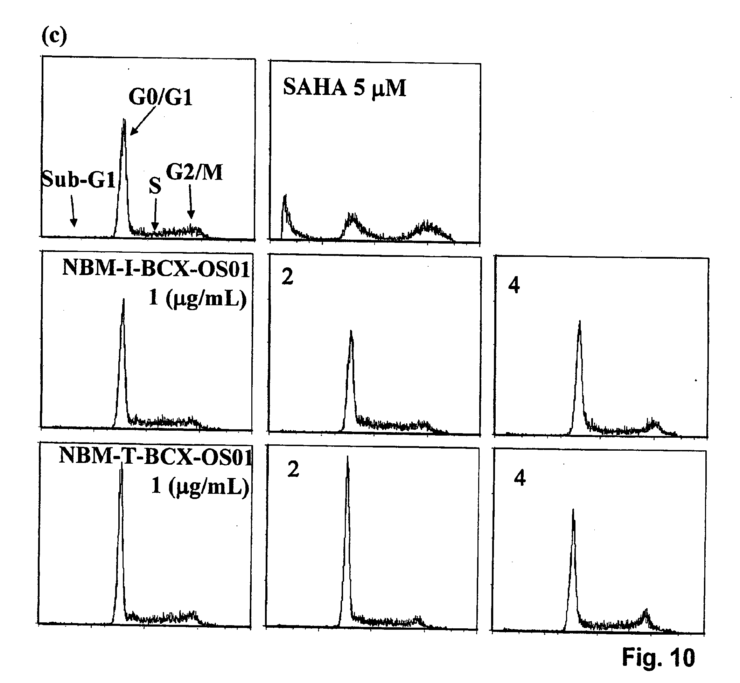

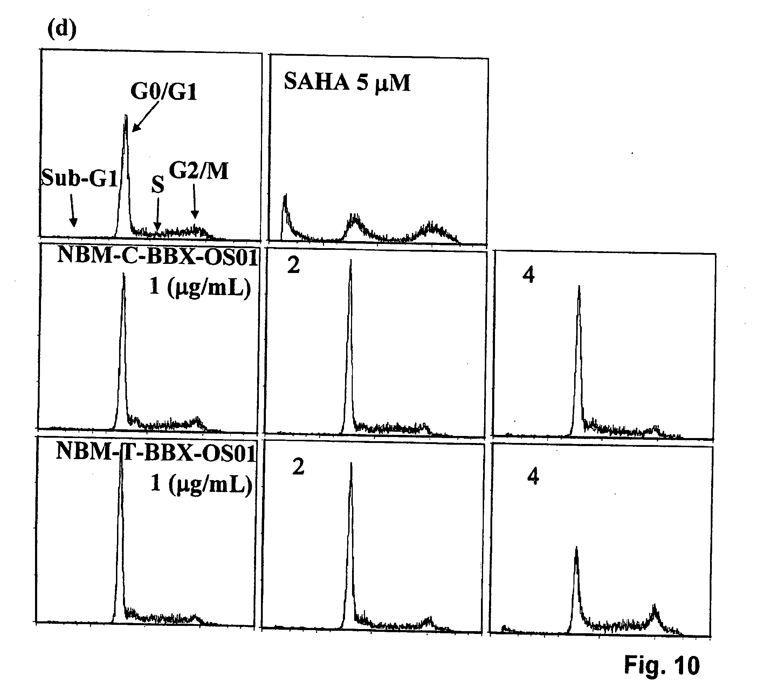

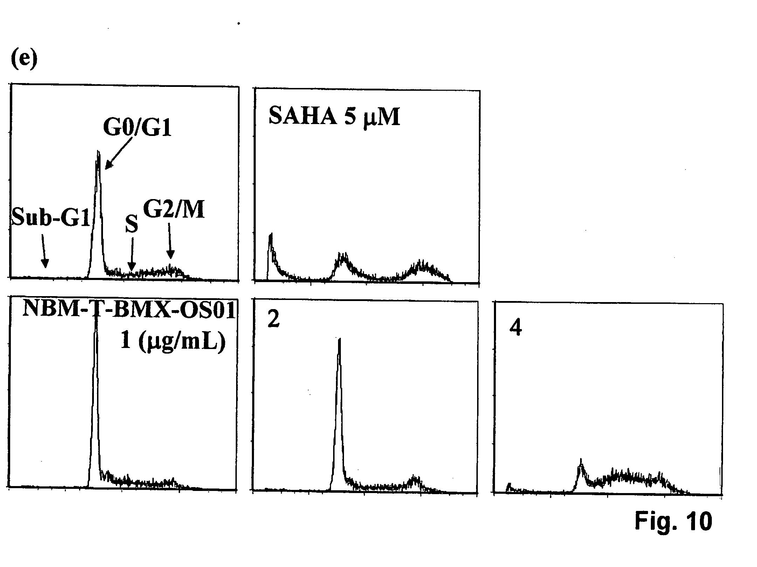

Figure 10 shows that (a) NBM-I-BCX-OS01 and NBM-T-BMX-OS01, and (b) NBM-T-BBX-OS01 and NBM-C-BBX-OS01 arrested the human lung cancer A549 cells on the S and G2/M phases (see Figs. (a) and (b)). The results were similar to those in Human breast cancer MCF-7 cells (see Figs. 10(c) and, (d)).

### Detailed Description of the Invention

The present invention relates to novel cinamic derivatives, which are useful as agents for enhancing the neurite outgrowth and preventing and treating of diseases associated with

HDAC, in particular, neurodegenerative diseases, stroke, diabetic, tumor or other cell proliferative diseases. The compounds of the invention are potent in inhibiting growth in cancer cells via differentiation pathway. In particular, they can be used as agents for anti-neurodegenerative diseases such as: Alzheimer's disease, Huntington's disease, Spinocerebellar Ataxias (SCA), and human spinal muscular atrophy (SMA).

### Compounds of the Invention

Accordingly, the present invention relates to compounds represented by the following formula (I): 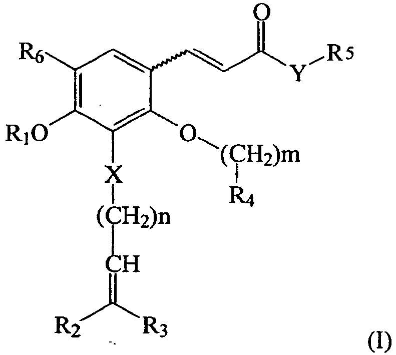 wherein
R₁ is hydrogen, alkyl, alkenyl, C₃₋₈-cycloalkyl, 5-membered or 6-membered unsaturated carbocycle or 5-membered or 6-membered heterocycle;
X is C, O, N or S;
Y is O, NH or O-C₁₋₄alkyl;
n is an integer of 0 to 10;
m is an integer of 0 to 5;
R₂ and R₃ is independently C₁₋₆ alkyl;
R₄ is C₅₋₆ cycloalkyl or 5-membered or 6-membered unsaturated carbocycle or heterocycle which may be substituted with halogen, CF₃, OR₇ or NR₇R₈, wherein R₇ and R₈ are independently hydrogen or C₁₋₆ alkyl;
R₅ is OH, NH₂ or C₅₋₆ cycloalkyl, 5-membered or 6-membered unsaturated carbocycle or heterocycle wherein the cycloalkyl, carbocycle and heterocycle may be optionally substituted with halogen, NH₂, NO₂, C₁₋₆alkoxy, C₁₋₆ alkylthio, OR₇, NR₇R₈ or CF₃,; and R₆ is H, C₁₋₁₀alkyl which may be substituted by hydroxy or C₂₁₀alkenyl, or together with R₁ being -C₂H₂-;
and pharmaceutically acceptable salts, stereoisomers, enantiomers and solvates thereof.

In the context of the present specification, the term "alkyl" means straight or branched hydrocarbon chains. The alkyl is preferably C₁₋₁₀ alkyl. Preferably, the carbon number of alkyl is selected from the group consisting of 1 to 8; more preferably, it is C₁₋₆ alkyl or C₁₋₄ alkyl. Examples of alkyl groups include methyl (-CH₃), ethyl (-CH₂CH₃), propyl (-CH₂CH₂CH₃), isopropyl (CH₃)₂CH and butyl (-C₄H₉).

In the context of the present specification, the term "alkenyl" means both straight and branched chain unsaturated hydrocarbon groups, wherein the unsaturation is present only as double bonds. According to the invention, the alkenyl includes one or more double bonds. The alkenyl is preferably C₂₋₁₆ alkenyl. More preferably, the carbon number of alkenyl is selected from the group consisting of 2 to 12. Examples of alkenyl groups include ethenyl (-CH=CH₂), propenyl (-CH=CHCH₃ or -CH₂CH=CH₂), butenyl (-CH₂CH=CHCH₃ or -CH=CHCH₂CH₃ or -CH₂CH₂CH=CH₂), -CH₂CH=C(CH₃)CH₃, -CH₂-CH=CH-CH₂-CH₂-CH₌CH-CH₃ and -CH₂-CH=C(CH₃)-CH₂-CH₂-CH=C(CH₃)-CH₃.

In the context of the present specification, the term "cycloalkyl" means an aliphatic ring (saturated carbocyclic ring). Preferably, the carbon number of cycloallcyl is selected from the group consisting of 3 to 8. More preferably, the carbon number of cycloalkyl is selected from the group consisting of 5 to 6. Examples of cycloalkyl groups include cyclopropyl, cyclobutyl, cyclopentyl and cyclohexyl.

In the context of the present specification, the term "unsaturated carbocycle" includes a cyclic substituent consisting of carbon atom and hydrogen atom, and the cyclic part is unsaturated cycle, for example, aryl or cycloalkenyl or the like. The term "cycloalkenyl" includes alkenyl which is the cycloalkyl having one or more double bond, for example, cyclopropenyl (e.g., 1-cyclopropenyl), cyclobutenyl (e.g., 1-cyclobutenyl), cyclopentenyl (e.g., 1-cyclopenten-1-yl, 2-cyclopenten-1-yl, and 3-cyclopenten-1-yl), cyclohexenyl (e.g., 1-cyclohexen-1-yl, 2-cyclohexen-1-yl, and 3-cyclohexen-1-yl), cycloheptenyl (e.g., 1-cycloheptenyl), cyclooctenyl (e.g., 1-cyclooctenyl) or the like. Especially, preferred is 1-cyclohexen-1-yl, 2-cyclohexen-1-yl, or 3-cyclohexen-1-yl. The term "aryl" includes single and fused rings wherein at least one ring is aromatic, for example, phenyl, naphthyl and tetrahydronaphthalenyl..

In the context of the present specification, the phrase "5-membered or 6-membered heterocycle" refers to a cyclic ring of five or six atoms, wherein at least one atom of the ring is a heteroatom. The 5-membered or 6-membered heterocycle can be aromatic or non-aromatic which is saturated or unsaturated. Preferably, the heteroatom is selected from N, O and S. Examples of heterocycle includes, but not limited to, furyl (e.g., 2-furyl, 3-furyl), thienyl (e.g., 2-thienyl, 3-thienyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), triazolyl (e.g., 1,2,4-triazol-1-yl, 1,2,4-triazol-3-yl, 1,2,4-triazol-4-yl) tetrazolyl (e.g., 1-tetrazolyl, 2-tetrazolyl, 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl) pyrimidinyl (e.g., 2-pyrimidinyl, 4-pyrimidinyl, 5-pyrimidinyl), furazanyl (e.g., 3-furazanyl), pyrazinyl (e.g., 2-pyrazinyl), oxadiazolyl (e.g., 1,3,4-oxadiazol-2-yl), 1-pyrrolinyl, 2-pyrrolinyl, 3-pyrrolinyl, pyrrolidino, 2-pyrrolidinyl, 3-pyrrolidinyl, 1-imidazolinyl, 2-imidazolinyl, 4-imidazolinyl, 1-imidazolidinyl, 2-in-lidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidyl, 3-piperidyl, 4-piperidyl, piperazino, 2-piperazinyl, 2-morpholinyl, 3-morpholinyl, morpholino, tetrahydropyranyl or the like.

In the context of the present specification, the term "halogen" means fluorine, chlorine, bromine and iodine.

In the context of the present specification, the term "pharmaceutically acceptable salt" includes those formed with both organic and inorganic acids and bases. Pharmaceutically acceptable acid addition salts include those formed from mineral acids such as: hydrochloric, hydrobromic, sulphuric, and phosphoric, acid; and organic acids such as: citric, tartaric, lactic, pyruvic, acetic, trifluoroacetic, succinic, oxalic, formic, fumaric, maleic, oxaloacetic, methanesulphonic, ethanesulphonic, p-toluenesulphonic, benzenesulphonic and isethionic acids. Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines.

In the context of the present specification, the term "solvate" means a complex comprising the compound of the invention and a solvent in which they are reacted or from which they are precipitated or crystallized.

In the context of the present specification, the term "stereoisomers" are isomeric molecules whose atomic connectivity is the same but whose atomic arrangement in space is different.

In the context of the present specification, the term "enantiomers" are stereoisomers that are nonsuperimposable complete mirror images of each other, much as one's left and right hands are "the same" but opposite.

According to the invention, the preferred compound of formula (I) of the invention is selected from the group consisting of the following:

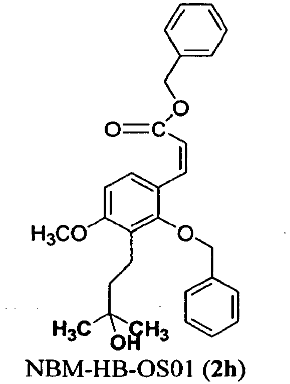

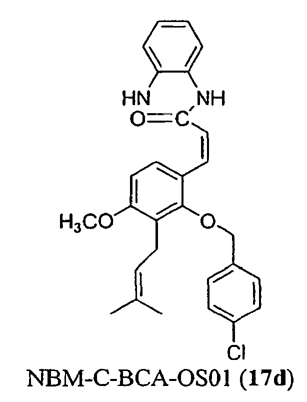

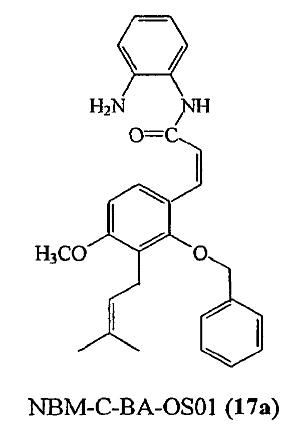

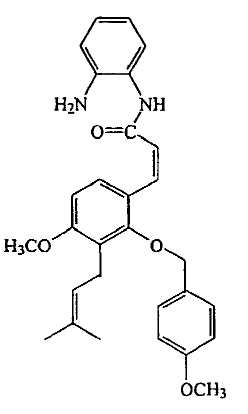

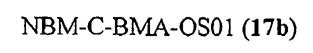

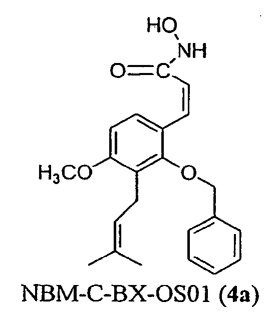

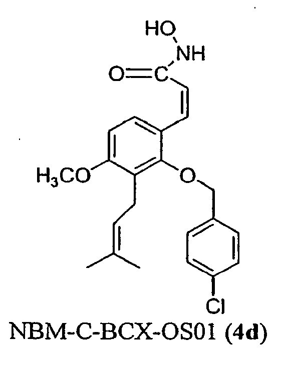

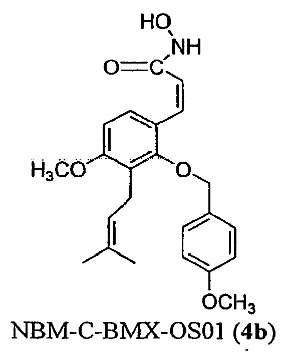

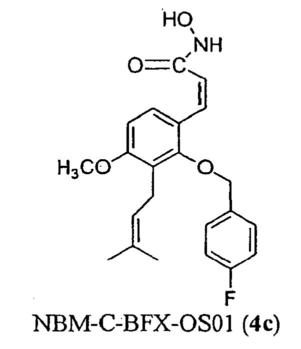

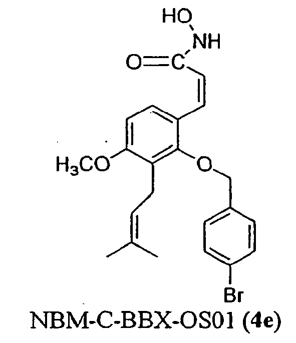

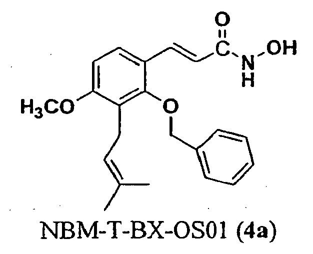

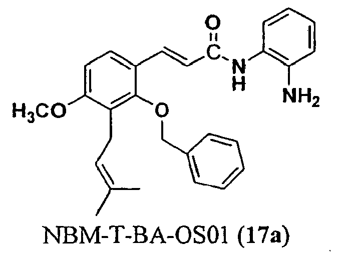

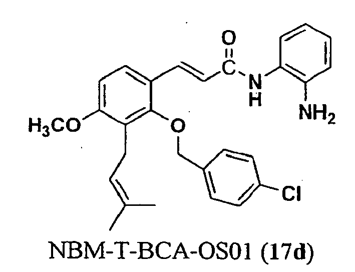

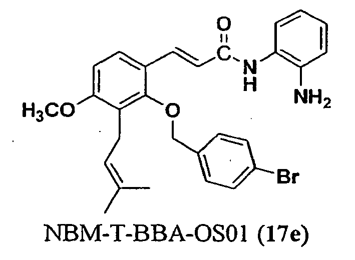

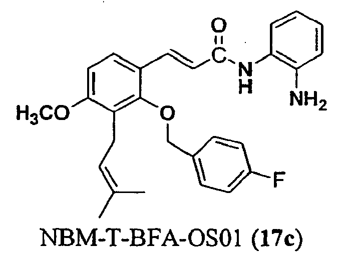

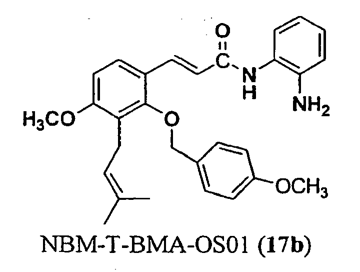

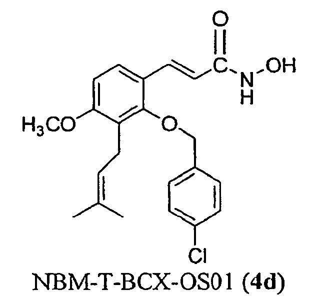

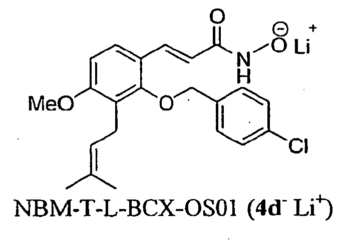

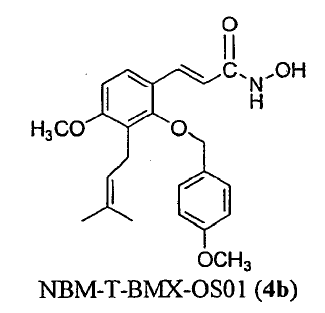

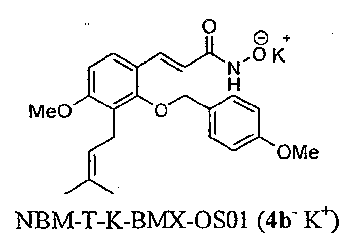

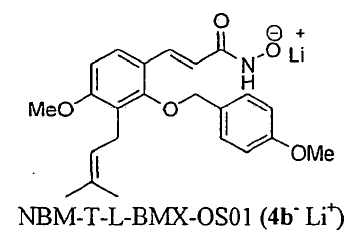

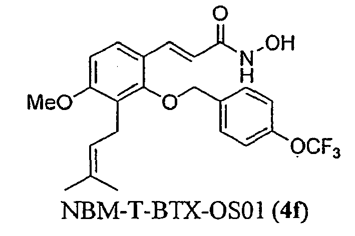

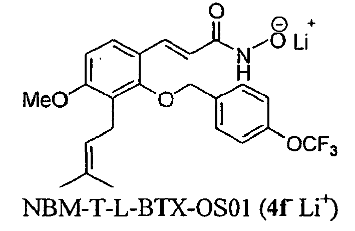

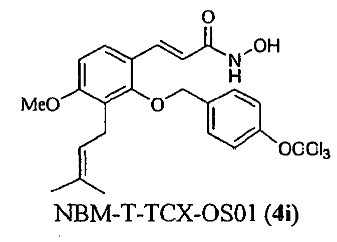

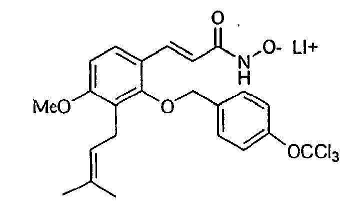

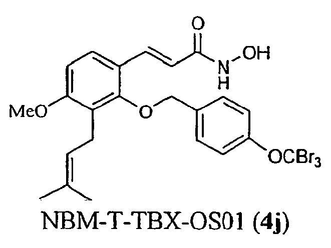

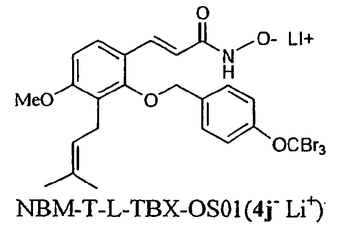

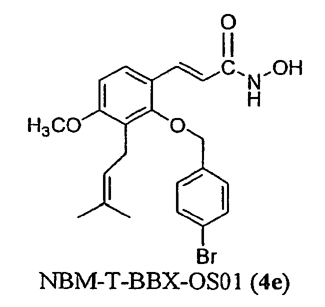

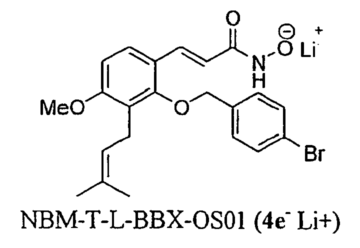

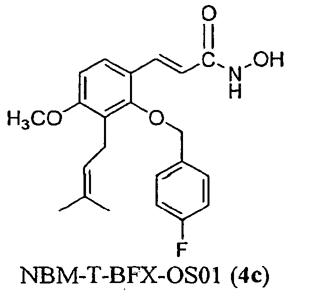

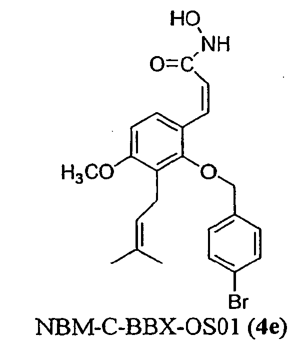

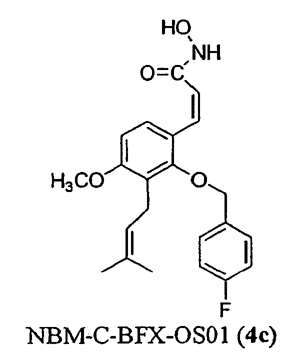

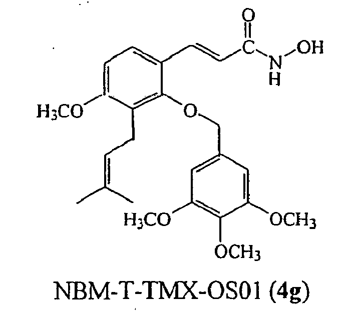

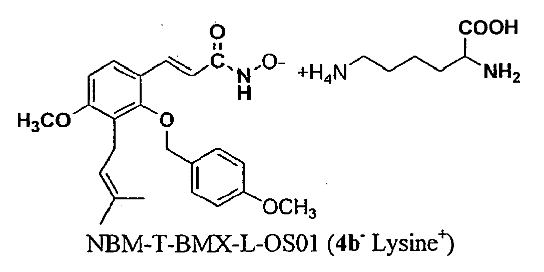

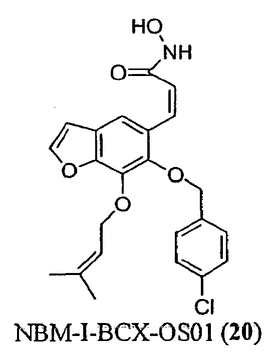

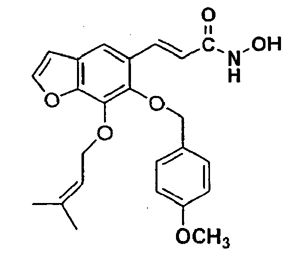

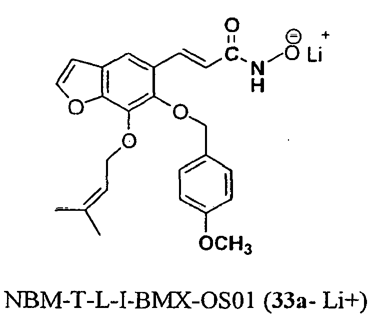

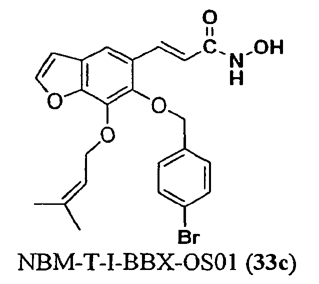

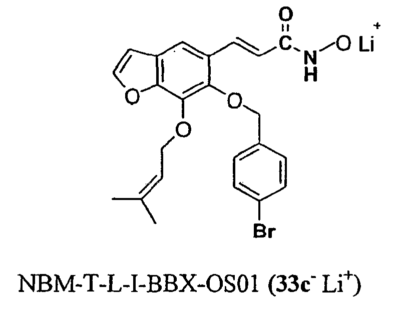

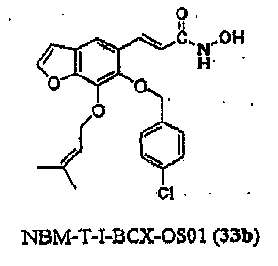

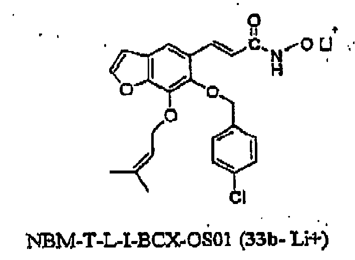

According to the invention, the compounds of formula (I) of the invention can inhibit HDAC and thus can be used as agents for the prevention or treatment of diseases associated with histone deacetylase (HDAC). In addition, the compounds of the invention significantly inhibit growth of multiple cancer cell lines, including those of rat C6 glioma, human glioblastoma, human breast cancer cells, human leukemia cells, and human melanoma cells. The mechanism for inhibiting the growth of cancer cells may be via differentiation pathway, in particular via induced differentiation and regulated cell cycle regulator gene expression, including those of p21 and cyclin B1. In addition, the compounds of formula (I) of the invention can mediate neuronal differentiation of neural stem cells and thus can be used as agents against stroke and anti-neurodegenerative diseases such as Huntington's disease and poly-glutamine disease. These compounds also can be used to enhance long-term memory. In addition, the compounds of formula (I) of the invention can control whole-body energy balance through the regulation of GLUT4 transcription and therefore provide new therapeutic targets for the treatment of Type 2 diabetes.

For the therapeutic uses of the compounds of the invention, the dosage administered will, of course, vary with the compound employed, the mode of administration, the treatment desired and the disorder indicated. The daily dosage of the compound of formula (I) may be in the range from 1 mg/kg to 20 mg/kg. The invention provides the methods of inhibiting HDAC, treating tumor or cell proliferative disease, stroke, diabetic, or neurodegenerative diseases such as Alzheimer's disease, Huntington's disease, Spinocerebellar Ataxias (SCA) and human spinal muscular atrophy (SMA) and enhancing the neurite outgrowth in a subject, comprising administrating to the subject a therapeutically effective amount of the compounds of the invention, respectively.

### General Synthesis of the Compounds of Formula I of the Invention

The compounds of the present invention can be prepared by any conventional means. Suitable processes for synthesizing these compounds are provided in the examples. Generally, compounds of formula (I) can be prepared according to one of the described synthetic schemes below:

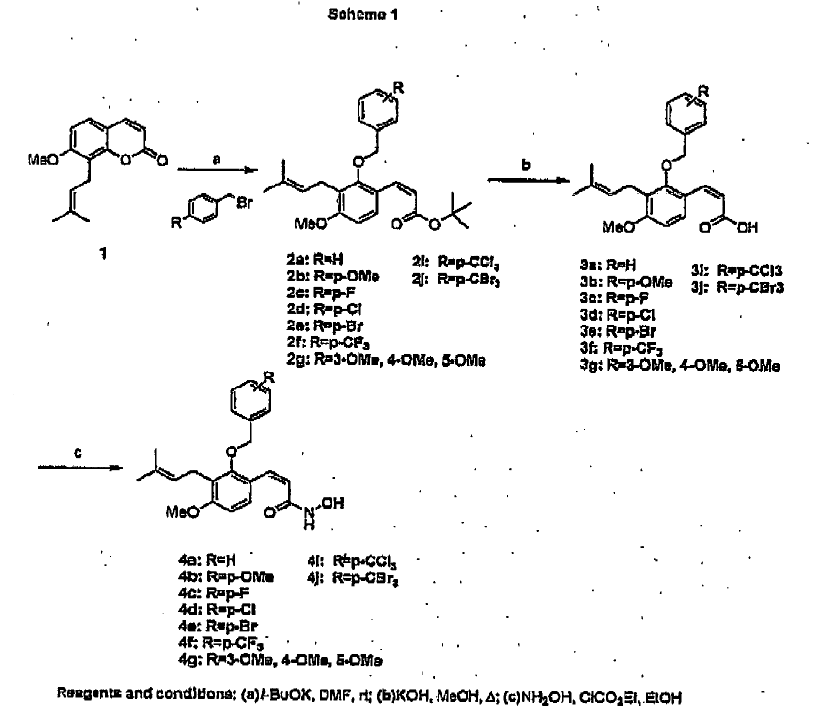

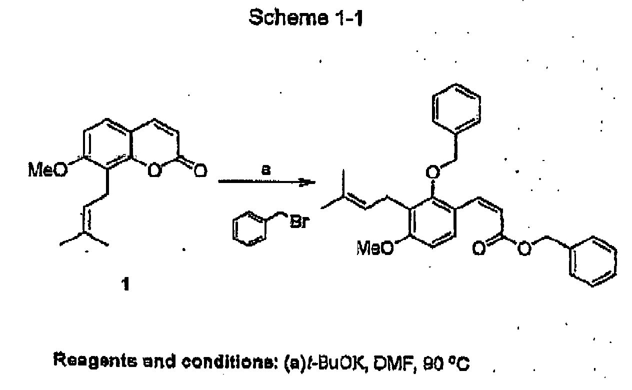

### General procedure for the preparation of 2

To the mixture of **1** (2 g, 8.20 mmol) and potassium *t*-butoxide (1.84 g, 16.4 mmol) in dry DMF (20 mL) was added various benzyl chlorides (16.4 mmol), the resulting solution was stirred at room temperature under nitrogen for 6h and then diluted with EtOAc (50 mL), washed with dis-H₂O (25 mL x3) and dried over Na₂SO₄. After removal of EtOAC under reduced pressure, the residue was purified by silica gel (EtOAc: *n*-Hexane=1:10~1:1) to give **2**.

**2h** can be prepared by a similar procedure with reaction temperature of 90°C.

### (Z)-2-Benzoxy-3-prenyl-4-methoxy- t-butylcinamate (2a)

¹H-NMR (500 MHz, CDCl₃): δ7.50 (1H, d, J=8.7 Hz), 7.43-7.32 (5H, m), 7.06 (1H, d, J=12.4 Hz), 6.67 (1H, d, J=8.7 Hz), 5.82 (1H, d, J=12.4 Hz), 5.15 (1H, t, J=6.5 5 Hz), 4.82 (2H, s), 3.84 (3H, s), 3.35 (2H, d, J=6.7 Hz), 1.70 (3H, s), 1.65 (3H, s), 1.44 (9H, s). (cis)

### (Z)-2-(4-Methoxybenzoxy)- 3-prenyl-4-methoxy-t-butyl cinamate (2b)

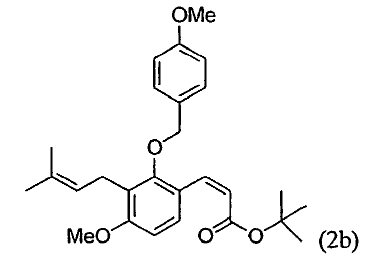

¹H-NMR (500 ^{MHz}, CDCl₃): δ7.50 (1H, d, J=8.6 Hz), 7.35-7.30 (2H, m), 7.06 (1H, d, J=12.3 Hz),

6.90 (2H, d, J=8.4 Hz), 6.67 (1H, d, J=8.6 Hz), 5.83 (1H, d, J=12.4 Hz), 5.15 (1H, t, J=6.5 Hz), 4.76 (2H, s), 3.86 (3H, s), 3.82 (3H, s), 3.34 (2H, d, J=6.6 Hz), 1.71 (3H, s), 1.65 (3H, s), 1.44 (9H, s). (cis)

### (Z)-2-Benzoxy-4-methoxy-3-(2-Hydroxy-2-methylbutyl)benzyl cinamate (2h)

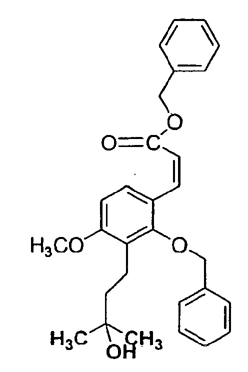

1H-NMR (500 MHz, CDCl3): δ 7.3 (1H, d, J=8.6 Hz), 7.25-7.12 (10H, m), 7.01 (1H, d, J=12.3 Hz), 6.45 (1H, d, J=8.6 Hz), 5.77 (1H, d, J=12.3Hz), 4.98 (2H, s), 4.66 (2H, s), 3.68 (3H, s), 1.47 (2H, m), 1.04 (6H, s)

### General procedure for the preparation of 3

The mixture of **2** (11.36 mmol) and 10% KOH/MeOH (40 mL) was refluxed overnight under N₂ and then diluted with dis-H₂O (100 mL), acidified with 2N HCl to pH 5~6 and extracted with EtOAC (50 mL x 3), respectively. The combined EtOAc layer was dried over Na₂SO₄ and concentrated under reduced pressure. The residual was purified by silica gel (EtOAc : *n*-Hexane= 1: 2) to give 3.

### (Z)-2-Benzoxy-3-prenyl-4-methoxy-cinamate (3a)

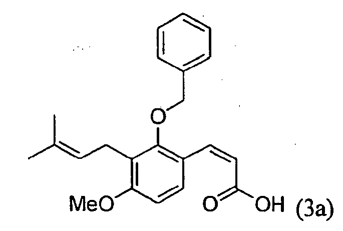

¹H-NMR (500 MHz, CDCl₃):δ7.63 (1H, d, J=8.6 Hz), 7.42-7.26 (5H, m), 7.25 (1H, d, J=12.5 Hz), 6.80 (1H, d, J=8.7 Hz), 5.88 (1H, d, J=12.5 Hz), 5.16 (1H, t, J=6.6 Hz), 4.82 (2H, s), 3.85 (3H, s), 3.36 (2H, d, J=6.7 Hz), 1.65 (3H, s), 1.62 (3H, s). (cis)

### General procedure for the preparation of 4

To a solution of potassium hydroxide (637 mg, 11.36 mmol) in MeOH (4mL) was added hydroxylamine hydrochloride (790 mg, 11.36 mmol) dropwise and then stirred at ice-bath for 1 h. Filtration to remove the white salt yielded free hydroxylame in MeOH solution. To the mixture of **3a** (1 g, 2.84 mmol) in dry THF (25 mL) was added ethyl chloroformate (0.6 mL, 5.68 mmol) and triethylamine (0.6 mL, 5.68 mmol) and the solution was stirred at for 0.5 h and then the prepared free hydroxylamine solution was added. After reacting for 3 h, the reaction mixture was concentrated under reduced pressure to give residue. The residue was purified by silica gel (EtOAc : *n*-Hexane= 1 : 2) to give **4a.**

### (Z)-2-Benzoxy-3-prenyl -4-methoxy- N-hydroxy-cinamide (4a)

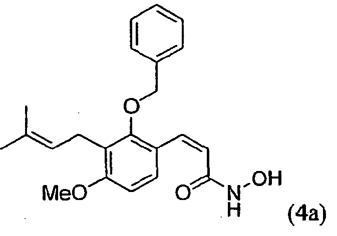

¹H-NMR (500 MHz, CDCl₃): δ7.40-7.33 (5H, m), 7.30 (1H, d, J=8.6 Hz), 7.01 (1H, d, J= 12.5 Hz), 6.66 (1H, d, J=8.7 Hz), 5.79 (1H, d, J=12.4 Hz), 5.17 (1H, t. J=6.6 Hz), 4.82 (2H, s), 3.83 (3H, s), 3.37 (2H, d, J=6.7 Hz), 1.69 (3H, s), 1.66 (3H, s); ¹³C-NMR (100 MHz, CDCl₃): δ159.7 (s), 159.2 (s), 155.9 (s), 136.9 (s), 135.9 (d), 131.8 (s), 128.6 (d), 128.5 (d), 128.2 (d), 128.1 (d), 128.0 (d), 123.9 (s), 122.7 (d), 121.1 (s), 118.2 (d), 106.8 (d), 76.6 (t), 55.7 (q), 25.7 (q), 23.2 (t), 17.9 (q), 14.4 (q). (cis)

### (Z)-2-(4-Methoxybenzoxy)-3-prenyl-4-methoxy-N-hydroxycinamide (4b)

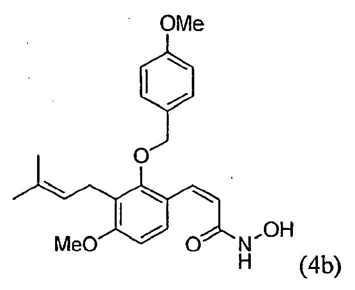

¹H-NMR (500 MHz, CDCl₃): δ7.35 (1H, d, J=8.6), 7.29 (2H, d, J=8.5 Hz), 6.99 (1H, d, J= 12.5

Hz), 6.89 (2H, d, J=8.6 Hz), 6.66 (1H, d, J=8.6 Hz), 5.79 (1H, d, J=12.4 Hz), 5.17 (1H, t, J=6.8 Hz), 4.76 (2H, s), 3.83 (3H, s), 3.81 (3H, s), 3.37 (2H, d, J=6.6 Hz), 1.71 (3H, s), 1.67 (3H, s). (cis)

### (Z)-2-(4-Fluorobenzoxy)-3-prenyl-4-methoxy-N-hydroxycinamide (4c)

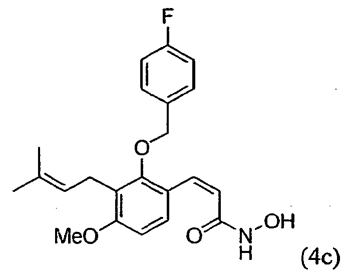

¹H-NMR (500 MHz, CDCl₃): 67.40 (1H, d, J=7.8 Hz), 7.36 (2H, d, J=8.5 Hz), 7.06 (2H, d, J= 8.5 Hz), 7.00 (1H, d, J=12.4 Hz), 6.65 (1H, d, J=7.8 Hz), 5.79 (1H, d, J=12.4 Hz), 5.14 (1H, t, J=6.2 Hz), 4.76 (2H, s), 3.80 (3H, s), 3.33 (2H, d, J=6.5 Hz), 1.67 (3H, s), 1.65 (3H, s). (cis)

### (Z)-2-(4-Chlorobenzoxy)-3-prenyl-4-methoxy-N-hydroxycinamide (4d)

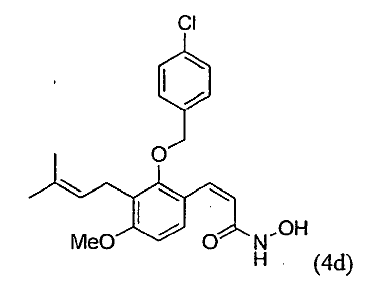

¹H-NMR (500 MHz, CDCl₃): δ7.34-7.31 (4H, m), 6.99 (1H, d, J= 12.2 Hz), 6.69 (2H, d, J=8.3 Hz), 5.81 (1H, d, J=12.4 Hz), 5.13 (1H, t, J=6.2 Hz), 4.77 (2H, s), 3.85 (3H, s), 3.33 (2H, d, J=6.5 Hz), 1.67 (3H, s), 1.65 (3H, s). (cis)

### (Z)-2-(4-Bromobenzoxy)-3-prenyl-4-methoxy-N-hydroxycinamide (4e)

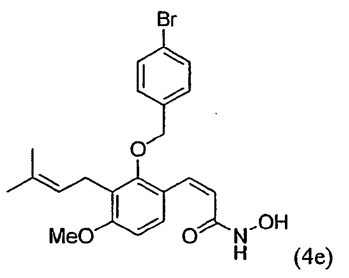

¹H-NMR (500 MHz, CDCl₃): δ7.47 (2H, d, J=8.1 Hz), 7.40 (1H, d, J= 8.4 Hz), 7.28 (2H, d, J=8.1 Hz), 6.95 (1H, d, J=12.5 Hz), 6.62 (1H, d, J=8.4 Hz), 5.76 (1H, d, J=12.4 Hz), 4.73 (2H, s), 3.76 (3H, s), 3.31 (2H, d, J=6.1 Hz), 1.64 (6H, s). (cis)

### (Z)-2-(4-Trifluoromethylbenzoxy)-3-prenyl-4-methoxy-N-hydroxycinamide (4f)

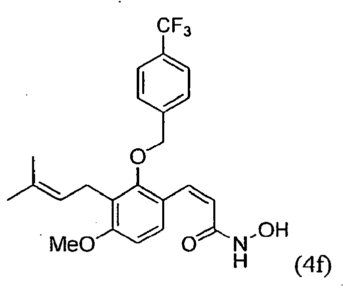

¹H-NMR (500 MHz, CDCl₃): δ7.64 (2H, d, J=8.1 Hz), 7.52 (2H, d, J=8.0 Hz), 7.43 (1H, d, J= 8.4 Hz), 7.00 (1H, d, J=12.4 Hz), 6.68 (1H, d, J=8.6 Hz), 5.80 (1H, d, J=12.4 Hz), 5.14 (1H, t, J=6.4 Hz), 4.86 (2H, s), 3.84(3H, s), 3.41 (2H, d, J=6.6 Hz), 1.66 (3H, s), 1.63 (3H, s). (cis)

### (Z)-2-(3,4,5-Trimethoxybenzoxy)-3-prenyl-4-methoxy-N-hydroxycinamide (4g)

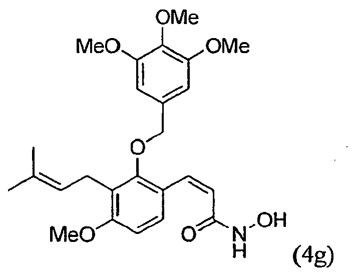

¹H-NMR (500 MHz, CDCl₃): δ7.41 (1H, d, J=8.3 Hz), 6.70 (1H, d, J=12.5 Hz), 6.65 (1H, d, J= 8.4 Hz), 6.62 (1H, s), 6.61 (1H, s), 5.77 (1H, d, J=12.3 Hz), 5.17 (1H, t, J=6.6 Hz), 4.75 (2H, s), 3.84(9H, s), 3.80 (3H, s), 3.35 (2H, d, J=6.4 Hz), 1.69 (3H, s), 1.65 (3H, s). (cis)

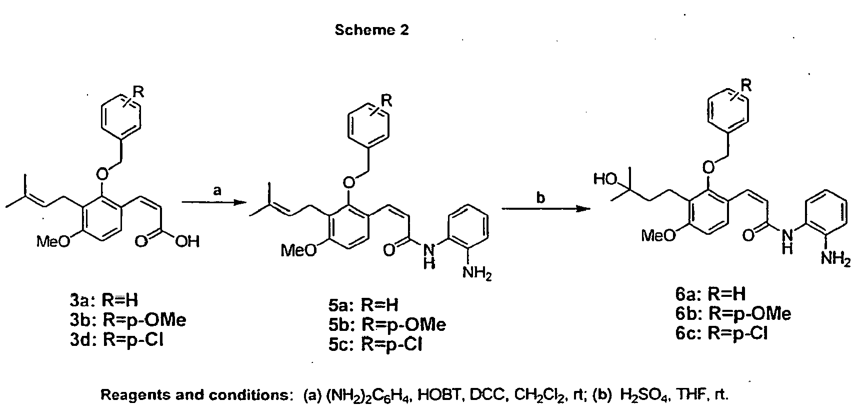

### General procedure for the preparation of 5

The mixture of 3 (17.04 mmol), HOBT (2.76 g, 20.44 mmol) and DCC (4.22 g, 20.44 mmol) in dry THF (30 mL) was stirred at room temperature for 0.5 h and then added o-phenylenediamine (1.84 g, 17.04 mmol). The resulting solution was stirred continuously overnight and then concentrated under reduced pressure to obtain the residue. The residue was dissolved in CH₂Cl₂ (50 ml), washed with saturated NaHCO₃ (25 mL x 3) and dried over Na₂SO₄. The organic layer was evaporated under reduced pressure and then purified by silica gel (EtOAc : *n*-Hexane= 1 : 3~1 : 1) to give 5.

### (Z)-2-Benzoxy-3-prenyl-4-methoxy-N-(2-aminophenyl)cinamide (5a)

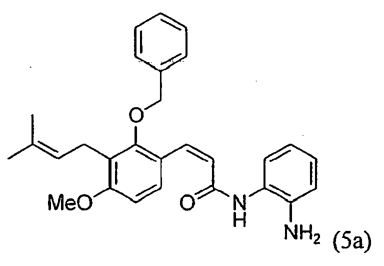

¹H-NMR (500 MHz, CDCl₃): δ7.43-7.38 (5H, m), 7.37 (1H, d, J=8.6 Hz), 7.03 (1H, d, J= 12.5 Hz), 7.01-7.02 (2H, m), 6.74-6.72 (2H, m), 6.68 (1H, d, J=8.6 Hz), 6.06 (1H, d, J=12.4 Hz), 5.14 (1H, t. J=6.6 Hz), 4.90 (2H, s), 3.84 (3H, s), 3.65 (2H, s), 3.38 (2H, d, J=6.5 Hz), 1.72 (3H, s), 1.65 (3H, s). (cis)

### (Z)-2-(4-Methoxybenzoxy)-3-prenyl-4-methoxy-N-(2-aminophenyl)cinamide (5b)

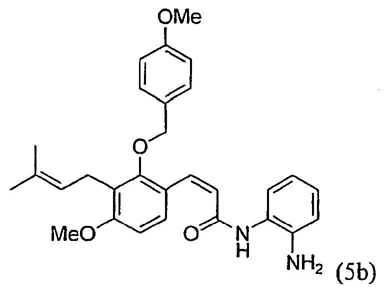

¹H-NMR (500 MHz, CDCl₃): δ7.41 (1H, d, J=8.5 Hz), 7.33 (2H, d, J=8.3 Hz), 7.00 (1H, d, J=12.9 Hz), 6.88 (2H, d, J=8.4 Hz), 6.73-6.72 (2H, m), 6.67 (1H, d, J=8.6 Hz), 6.04 (1H, d, J=12.4 Hz), 5.15 (1H, t, J=6.6 Hz), 4.83 (2H, s), 3.87 (3H, s), 3.77 (3H, s), 3.38 (2H, d, J=6.3 Hz), 1.74 (3H, s), 1.67 (3H, s). (cis)

### (Z)-2-(4-Chlorobenzoxy)-3-prenyl-4-methoxy-N-(2-aminophenyl)cinamide (5c)

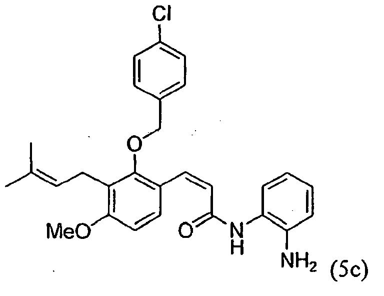

¹H-NMR (500 MHz, CDCl₃): δ7.44 (1H, d, J=8.6 Hz), 7.35 (4H, m), 7.12 (1H, d, J=12.4 Hz), 7.00 (2H, d, J=10.5 Hz), 6.74 (2H, m), 6.69 (1H, d, J=8.6 Hz), 6.07 (1H, d, J=12.4 Hz), 5.13 (1H, t, J=6.6 Hz), 4.85 (2H, s), 3.84 (3H, s), 3.35 (2H, d, J=6.3 Hz), 1.65 (3H, s), 1.59 (3H, s). (cis)

### General procedure for the preparation of 6

To a solution of 5 (1 mmol) in THF (15 mL) was added 49% H₂SO₄ (10 mL) and stirred at room temperature for 6 h. The resulting solution was extracted with CH₂Cl₂ (50 mL x 3) and then dried over Na₂SO₄ to give residue. The residue was purified by silica gel (EtOAc : n-Hexane= 2 : 1).

### (Z)-2-Benzoxy-3-(2-Hydroxy-2-methylbutyl)-4-methoxy-N-(2-aminophenyl)cinamide (6a)

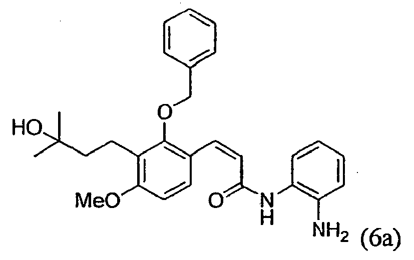

¹H-NMR (500 MHz, CDCl₃): δ7.43-7.38 (5H, m), 7.37 (1H, d, J=8.6 Hz), 7.03 (1H, d, J= 12.5 Hz), 7.01-7.02 (2H, m), 6.74-6.72 (2H, m), 6.68 (1H, d, J=8.6 Hz), 6.06 (1H, d, J=12.4 Hz), 4.92 (2H, s), 3.81 (3H, s), 2.68 (2H, m), 1.62 (2H, m), 1.18 (6H, s). (cis)

### (Z)-2-(4-Methoxybenzoxy)-3-(2-Hydroxy-2-methylbutyl)4-methoxy-N-(2-aminophenyl)cinamide (6b)

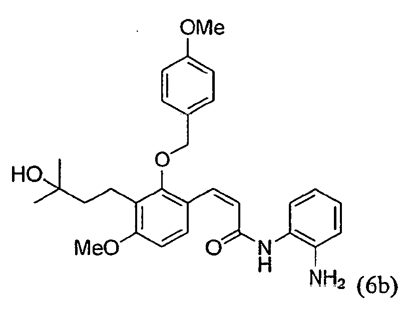

¹H-NMR (500 MHz, CDCl₃): δ7.41 (1H, d, J=8.5 Hz), 7.33 (2H, d, J=8.3 Hz), 7.00 (2H, d, J=12.9 Hz), 6.88 (2H, d, J=8.4 Hz), 6.73-6.72 (4H, m), 6.67 (1H, d, J=8.6 Hz), 6.04 (1H, d, J=12.4 Hz), 4.83 (2H, s), 3.87 (3H, s), 3.77 (3H, s), 2.67 (2H, m), 1.64 (2H, m),, 1.74 (3H, s), 1.67 (3H, s). (cis)

### (Z)-2-(4-Chlorobenzoxy)-3-(2-Hydroxy-2-methylbutyl)-4-methoxy-N-(2-aminophenyl)cinamide (6c)

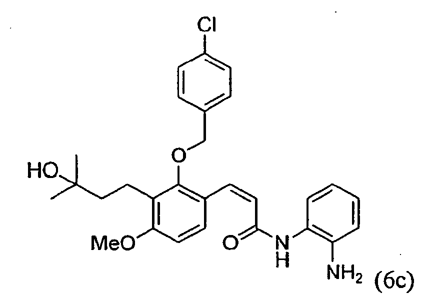

¹H-NMR (500 MHz, CDCl₃): δ7.44 (1H, d, J=8.6 Hz), 7.35 (4H, m), 7.12 (1H, d, J=12.4 Hz), 7.00 (2H, d, J=10.5 Hz), 6.74 (2H, m), 6.69 (1H, d, J=8.6 Hz), 6.07 (1H, d, J=12.4 Hz), 4.85 (2H, s), 3.84 (3H, s),3.35 (2H, d, J=6.3), 2.68-2.64 (2H, m),1.65 (3H, s), 1.63-1.57 (2H, m), 1.59 (3H, s). (cis)

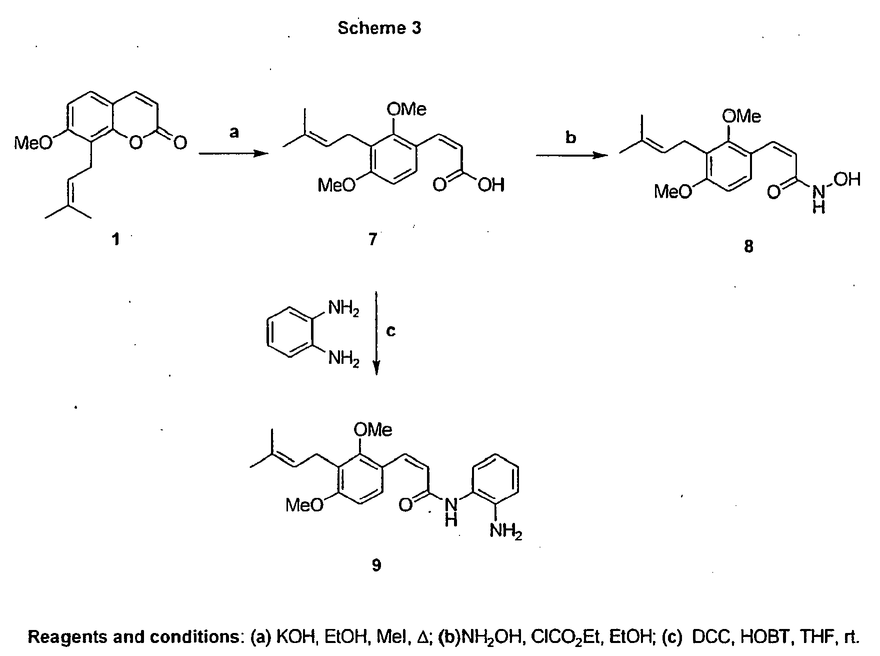

### (Z)-2,4-Dimethoxy-3-prenyl cinamate (7)

To a solution of **1** (2.44 g, 10 mmol) and KOH (4.20 g, 75 mmol) dissolved in EtOH (100 ml) was added Mel (2.51 ml, 40 mmol) dropwise and then heated to 90 °C for 24 h. The mixture was diluted with dis-H₂O (100 ml), acidified with 1N HCl to pH 3-4 and then extracted with EtOAc (50 ml x 3). The combined EtOAc layer was dried over Na₂SO₄, filtered and concentrated under reduced pressure. The residue was purified by silica gel (EtOAc : *n*-Hexane= 6: 1) to obtain 7.

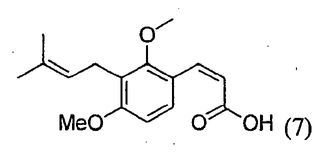

¹H-NMR (500 MHz, CDCl₃): δ7.62 (1H, d, J=8.6 Hz), 7.23 (1H, d, J=12.5 Hz), 6.64 (1H, d, J=8.7 Hz), 5.92 (1H, d, J=12.5 Hz), 5.17 (1H, t, J=6.3 Hz), 3.84 (3H, s), 3.71 (3H, s), 3.34 (2H, d, J=6.7 Hz), 1.77 (3H, s), 1.67 (3H, s).

### (Z)-2,4-Dimethoxy-3-prenyl-N-hydroxy cinamide (8)

Following the procedure similar to that of **4** and further purification by silica gel (EtOAc : *n*-Hexane= 1: 1) gave **8**.

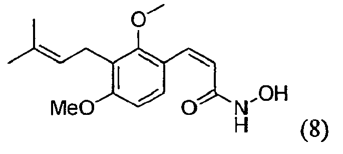

¹H-NMR (500 MHz, CDCl₃): δ7.36 (1H, d, J=8.6 Hz), 7.02 (1H, d, J= 12.5 Hz), 6.65 (1H, d, J=8.6 Hz), 5.87 (1H, d, J=12.5 Hz), 5.16 (1H, t. J=6.9 Hz), 3.82 (3H, s), 3.70 (3H, s), 3.34 (2H, d, J=6.8 Hz), 1.77 (3H, s), 1.68 (3H, s).

### (Z)-2,4-Dimethoxy-3-prenyl-N-(2-aminophenyl)cinamide (9)

Following the procedure similar to that of 5 and further purification by silica gel (EtOAc : *n*-Hexane=2: 3) gave **9**.

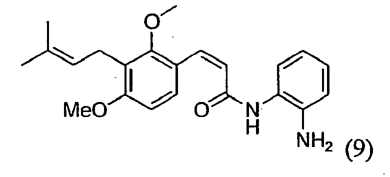

¹H-NMR (500 MHz, CD₃OD): δ7.48 (1H, d, J=8.6 Hz), 7.09 (1H, d, J=12.4 Hz), 7.00-6.97 (2H, m), 6.83 (1H, d, J=8.1 Hz), 6.72-6.70 (2H, m), 6.16 (1H, d, J=12.4 Hz), 5.12 (1H, t, J=6.9 Hz), 3.81 (3H, s), 3.73 (3H, s), 3.35 (2H, d, J=6.7 Hz), 1.75 (3H, s), 1.63 (3H, s).

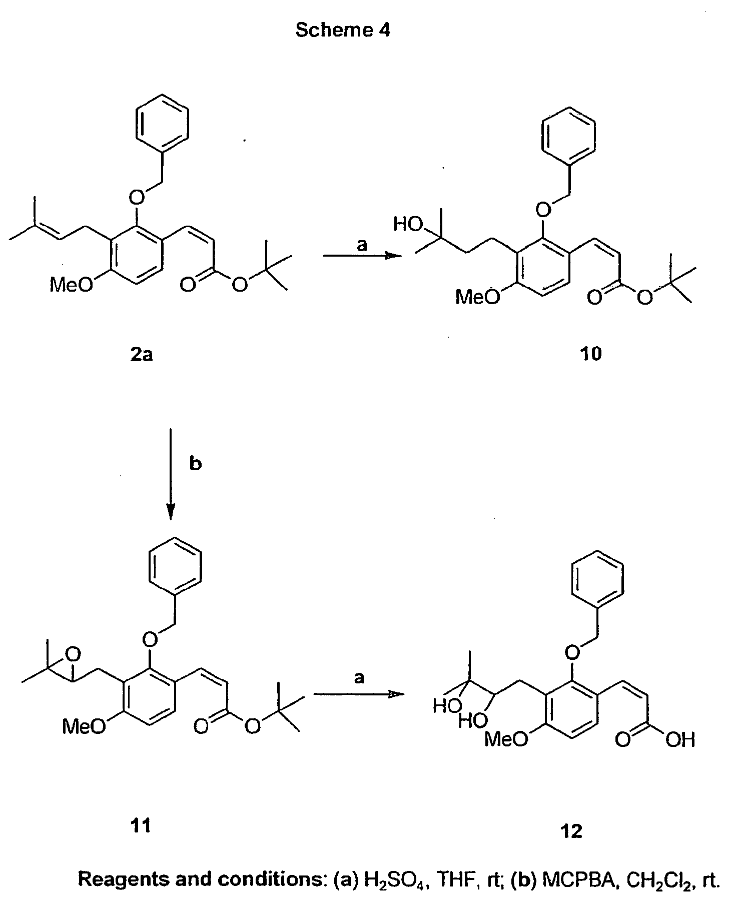

### (Z)-2-Benzoxy-3-(2-Hydroxy-2-methylbutyl)-4-methoxy-t-butyl cinamate (10)

To a solution of **2a** (0.27mmol) in THF (15 mL) was added 49%H₂SO₄ (10 mL) and stirred at room temperature for 6 h. The resulting solution was extracted with CH₂Cl₂ (50 mL × 3) and then dried over Na₂SO₄ to give residue. The residue was purified by silica gel (EtOAc : *n-*Hexane=1:1).

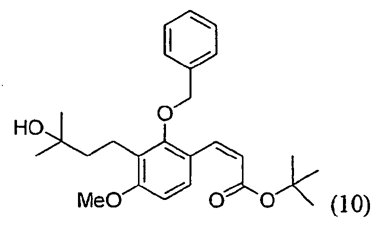

¹H-NMR (500 MHz, CDCl₃): δ7.38 (1H, d, J=8.7 Hz), 7.36-7.27 (5H, m), 7.00 (1H, d, J= 12.4 Hz), 6.60 (1H, d, J=8.7 Hz), 5.94 (1H, d, J=12.4 Hz), 5.13 (2H, s), 4.81 (2H, s), 3.82 (3H, s), 2.67 (2H, t, J=8.2 Hz), 1.61 (2H, t, J=8.2 Hz), 1.44 (9H, s), 1.18 (6H, s).

### (Z)-2-Benzoxy-3-(2,3-epoxy-2-methylbutyl)-4-methoxy-t-butyl cinamate (11)

To a solution of **2a** (1 g, 2.67 mmol) in CH₂Cl₂(10 ml) was added 70%MCPBA (553 mg, 3.20 mmol) and stirred at rt for 2 h. The reaction mixture was washed with 1NaHSO₃, 10% NaHCO₃ and dried over Na₂SO₄. The CH₂Cl₂ was evaporated under reduced pressure and then purified by silica gel (EtOAc : *n*-Hexane= 9 : 1) to obtain **11.**

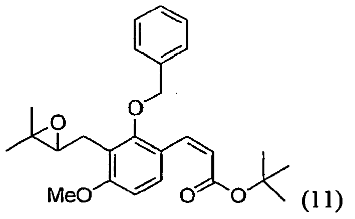

¹H-NMR (500 MHz, CDCl₃): δ7.55 (1H, d, J=8.6 Hz), 7.43 (2H, d, J=7.1 Hz), 7.38-7.32 (3H, m), 7.08 (1H, d, J= 12.4 Hz), 6.69 (1H, d, J=8.7 Hz), 5.86 (1H, d, J=12.4 Hz), 4.92 (1H, d, J=11.1 Hz), 4.83 (1H, d, J=11.1 Hz), 3.86 (3H, s), 2.97-2.94 (2H, m), 2.83-2.79 (1H, m), 1.44 (9H, s), 1.33 (3H, s), 1.25 (3H, s).

### (Z)-2-Benzoxy-3-(2,3-dihydroxy-2-methylbutyl)-4-methoxy-cinamate (12)

Following the reaction procedure similar to that of 5 and further purification by silica gel (EtOAc : *n*-Hexane= 1 : 4) gave **12**.

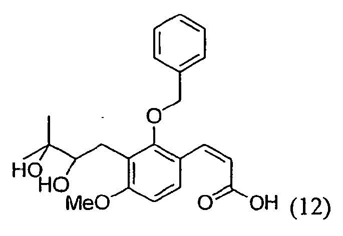

¹H-NMR (500 MHz, CDCl₃): δ7.57 (1H, d, J=8.6 Hz), 7.40-7.34 (5H, m), 7.23 (1H, d, J= 12.6 Hz), 6.72 (1H, d, J=8.7 Hz), 5.97 (1H, d, J=12.4 Hz), 4.91 (1H, d, J=11. Hz), 4.81 (1H, d, J=11.1 Hz), 3.88 (1H, t, J=10.5 Hz), 3.85 (3H,s), 2.63 (2H, d, J=10.5 Hz),1.22 (3H, s), 1.16 (3H, s).

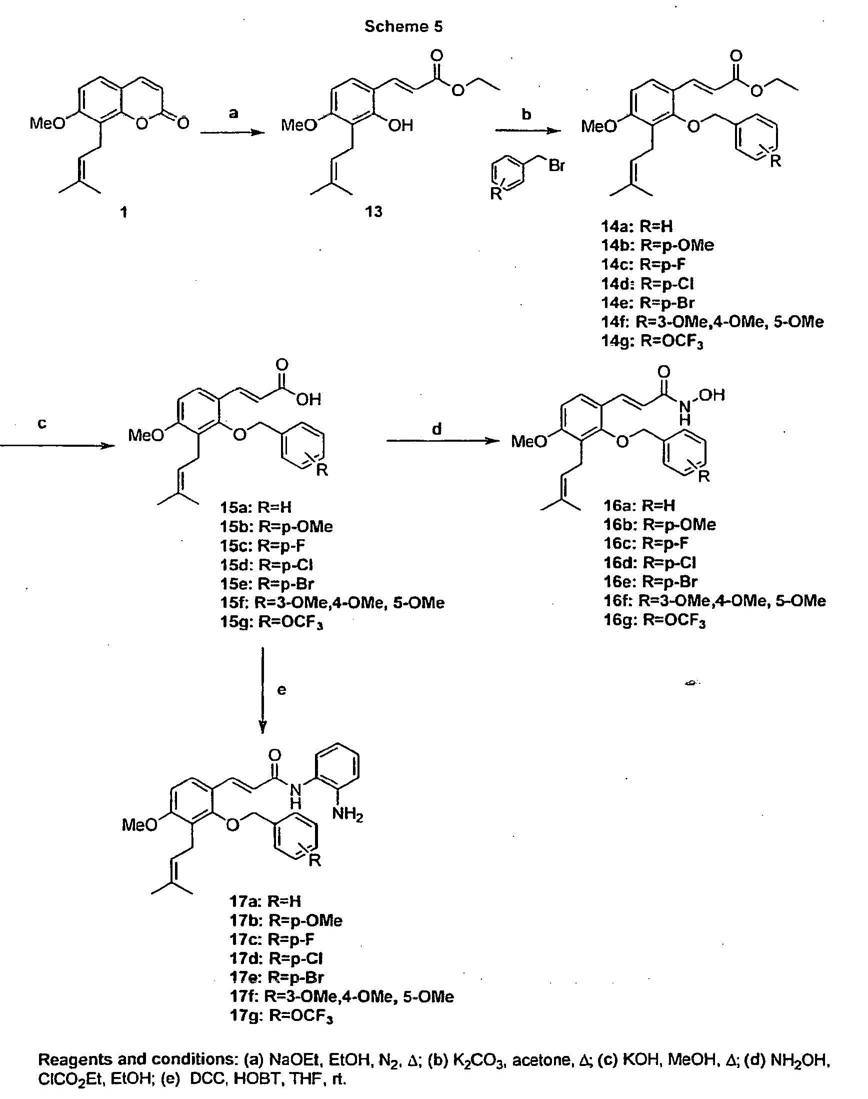

### (E) 2-Hydroxy-3-prenyl-4-methoxy-ethyl cinamate (13)

To the solution of **1** (2.40 g, 10 mmol) in dry EtOH (20 mL) was added the mixture of sodium ethoxide (1.36 g, 20 mmol) in dry EtOH (20 mL) dropwise, the resulting solution was heated under nitrogen for 6h and then diluted with dis-H₂O (50 mL), acidified with 1N HCl_{(aq)} to pH 4-5, extracted with EtOAc (50 mL x 3) and dried over Na₂SO₄. After removal of EtOAc under reduced pressure, the residue was purified by silica gel (EtOAc: *n*-Hexane=10:1) to give 13.

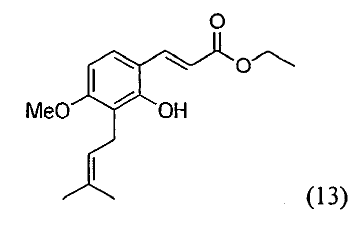

¹H-NMR (500 MHz, CDCl₃): δ7.92 (1H, d, J=16.1 Hz), 7.33 (1H, d, J=8.7 Hz), 6.49 (1H, d, J=8.7 Hz), 6.44 (1H, d, J=16.1 Hz), 6.10 (1H, s), 5.20 (1H, t, J=7.0 Hz), 4.23 (2H, q, J=7.2Hz), 3.82 (3H, s), 3.42 (1H, d, J=7.1 Hz), 1.82 (3H, s), 1.75 (3H, s), 1.31 (3H, t, J=7.2 Hz). (trans)

### General procedure for the preparation of 14

To the mixture of 13 (1.72 mmol) and K₂CO₃ (4.3 mmol) in acetone (20 mL) was added apropriate benzyl bromide (3.44 mmol), the resulting solution was heated under No overnight. After filteration to remove K₂CO₃, the filtrate was condensed under reducted pressure. The resulting residue was purified by gel (EtOAc: *n*-Hexane=15:1) to give **14.**

### (E)-2-(4-Methoxybenzoxy)- 3-prenyl -4-methoxy-ethyl cinamate (14a)

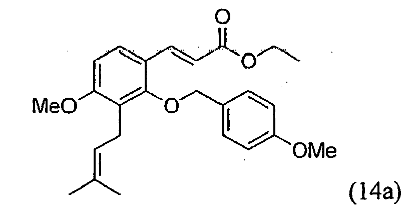

¹H-NMR (500 MHz, CDCl₃): δ7.98 (1H, d, J=16.1 Hz), 7.44 (1H, d, J=8.7 Hz), 7.40 (2H, d, J=8.4 Hz), 6.92 (2H, d, J=8.4 Hz), 6.71 (1H, d, J=8.7 Hz), 6.34 (1H, d, J=16.1 Hz), 5.17 (1H, t, J=6.4 Hz), 4.74 (2H, s), 4.25 (2H, q, J=7.1Hz), 3.86 (3H, s), 3.83 (3H, s), 3.39 (2H, d, J=6.5 Hz), 1.73 (3H, s), 1.67 (3H, s), 1.33 (3H, t, J=7.1 Hz). (trans)

### (E)-2-(4-Chlorobenzoxy)- 3-prenyl-4-methoxy-ethyl cinamate (14b)

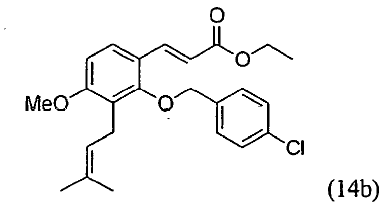

¹H-NMR (500 MHz, CDCl₃): δ7.92 (1H, d, J=16.1 Hz), 7.44 (1H, d, J=8.7 Hz), 7.41 (2H, d, J=8.4 Hz), 7.37 (2H, d, J=8.4 Hz), 6.72 (1H, d, J=8.7 Hz), 6.34 (1H, d, J=16.0 Hz), 5.16 (1H, t, J=6.6 Hz), 4.77 (2H, s), 4.23 (2H, q, J=7.2Hz), 3.86 (3H, s), 3.37 (2H, d, J=6.6 Hz), 1.69 (3H, s), 1.67 (3H, s), 1.31 (3H, t, J=7.2 Hz). (trans)

### (E)-2-(4-Bromobenzoacy)- 3-prenyl-4-methoxy-ethyl cinamate (14c)

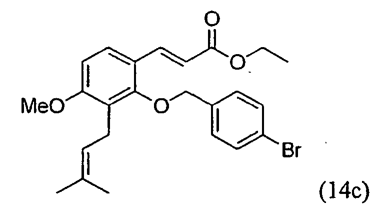

¹H-NMR (500 MHz, CDCl₃): δ7.91 (1H, d, J=16.1 Hz), 7.52 (2H, d, J=8.4 Hz), 7.44 (1H, d, J=8.7 Hz), 7.34 (2H, d, J=8.4 Hz), 6.72 (1H, d, J=8.7 Hz), 6.34 (1H, d, J=16.0 Hz), 5.16 (1H, t, J=6.6 Hz), 4.75 (2H, s), 4.23 (2H, q, J=7.1Hz), 3.87 (3H, s), 3.36 (2H, d, J=6.6 Hz), 1.70 (3H, s), 1.68 (3H, s), 1.31 (3H, t, J=7.1 Hz). (trans)

### (E)-2-(3,4,5-Trimethoxybenzoxy)- 3-prenyl-4-methoxy-ethyl cinamate (14d)

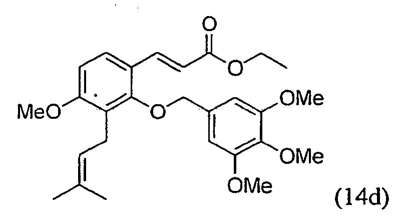

¹H-NMR (500 MHz, CDCl₃): δ7.96 (1H, d, J=16.1 Hz), 7.45 (1H, d, J=8.6 Hz), 6.72 (1H, d, J=8.8 Hz), 6.70 (2H, s), 6.38 (1H, d, J=16.0 Hz), 5.20 (1H, t, J=6.6 Hz), 4.74 (2H, s), 4.22 (2H, q, J=7.1Hz), 3.90 (6H, s), 3.87 (3H, s), 3.86 (3H, s), 3.40 (2H, d, J=6.5 Hz), 1.74 (3H, s), 1.68 (3H, s), 1.30 (3H, t, J=7.2 Hz). (trans)

### General procedure for the preparation of 15

The mixture of **14** (1.81 mmol) and 10% KOH/MeOH (20 mL) was refluxed overnight under N₂ and then diluted with dis-H₂O (100 mL), acidified with 2N HCl to pH 5~6 and extracted with EtOAc (50 mL x 3), respectively. The combined EtOAc layer was dried over Na₂SO₄ and concentrated under reduced pressure to give 15.

### (E)-2-(4-Methoxybenzoxy)- 3-prenyl-4-methoxy-cinamate (15a)

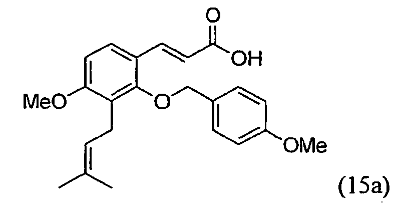

¹H-NMR (500 MHz, CDCl₃): δ8.06 (1H, d, J=16.1 Hz), 7.47 (1H, d, J=8.7 Hz), 7.39 (2H, d, J=8.5 Hz), 6.93 (2H, d, J=8.5 Hz), 6.73 (1H, d, J=8.7 Hz), 6.35 (1H, d, J=16.0 Hz), 5.18 (1H, t, J=6.5 Hz), 4.76 (2H, s), 3.88 (3H, s), 3.82 (3H, s), 3.39 (1H, d, J=6.5 Hz), 1.73 (3H, s), 1.68 (3H, s). (trans)

### (E)-2-(4-Chlorobenzoxy)- 3-prenyl-4-methoxy-cinamate (15b)

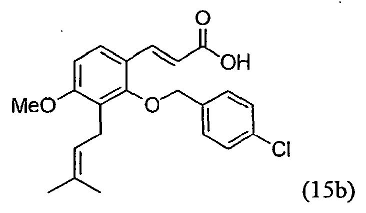

¹H-NMR (500 MHz, DMSO-d6): δ7.69 (1H, d, J=16.0 Hz), 7.64 (1H, d, J=8.8 Hz), 7.47 (2H, d, J=9.3 Hz), 7.44 (2H, d, J=9.3 Hz), 6.87 (1H, d, J=8.8 Hz), 6.37 (1H, d, J=16.0 Hz), 5.06 (1H, t, J=6.4 Hz), 4.75 (2H, s), 3.82 (3H, s), 3.25 (1H, d, J=6.6 Hz), 1.58 (6H, s). (trans)

### (E)-2-(4-Bromobenzoxy)- 3-prenyl-4-methoxy-cinamate (15c)

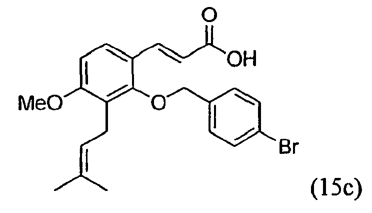

¹H-NMR (500 MHz, DMSO-d6): δ7.71 (1H, d, J=16.1 Hz), 7.65 (1H, d, J=8.7 Hz), 7.60 (2H, d, J=8.3 Hz), 7.40 (2H, d, J=8.3 Hz), 6.88 (1H, d, J=8.8 Hz), 6.38 (1H, d, J=16.1 Hz), 5.06 (1H, t, J=6.5 Hz), 4.74 (2H, s), 3.82 (3H, s), 3.26 (2H, d, J=6.6 Hz), 1.59 (6H, s). (trans)

### (E)-2-(3,4,5-Trimethoxybenzoxy)- 3-prenyl-4-methoxy-cinamate (15d)

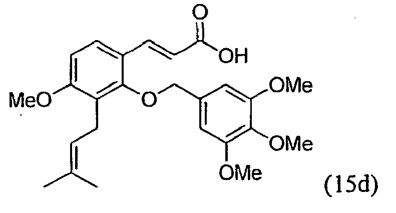

¹H-NMR (500 MHz, CDCl₃): δ7.96 (1H, d, J=16.1 Hz), 7.45 (1H, d, J=8.6 Hz), 6.72 (1H, d, J=8.8 Hz), 6.70 (2H, s), 6.38 (1H, d, J=16.0 Hz), 5.20 (1H, t, J=6.6 Hz), 4.74 (2H, s), 3.90 (6H, s), 3.87 (3H, s), 3.86 (3H, s), 3.40 (2H, d, J=6.5 Hz), 1.74 (3H, s), 1.68 (3H, s). (trans)

### General procedure for the preparation of 16

To a solution of potassium hydroxide (637 mg, 11.36 mmol) in MeOH (4mL) was added hydroxylamine hydrochloride (790 mg, 11.36 mmol) dropwise and then stirred in an ice-bath for 1 h. Filtration to remove the white salt gave free hydroxylame in MeOH solution. To the mixture of **15** (2.84 mmol) in dry THF (25 mL) was added ethyl chloroformate (0.6 mL, 5.68 mmol) and triethylamine (0.6 mL, 5.68 mmol) and stirred for 0.5 h and then added the prepared free hydroxylamine solution. After reacting for 3 h, the reaction mixture was concentrated under reduced pressure to give residue. The residue was purified by silica gel (EtOAc: *n*-Hexane=1:1) to give **16**.

### (E)-2-(4-Methoxybenzoxy)- 3-prenyl-4-methoxy-N-hydroxy cinamide (16a)

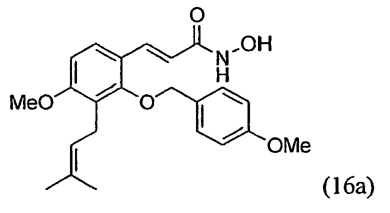

¹H-NMR (500 MHz, CDCl₃): δ7.83 (1H, d, J=15.5 Hz), 7.33 (2H, d, J=8.4 Hz), 7.27 (1H, d, J=8.6 Hz), 6.88 (2H, d, J=8.3 Hz), 6.52 (1H, d, J=8.6 Hz), 6.28 (1H, d, J=15.5 Hz), 5.12 (1H, t, J=5.7 Hz), 4.64 (2H, s), 3.76 (3H, s), 3.74 (3H, s), 3.33 (2H, d, J=6.2 Hz), 1.70 (3H, s), 1.64 (3H, s). (trans)

### (E)-2-(4-Chlorobenzoxy)- 3-prenyl-4-methoxy-N-hydroxy cinamide (16b)

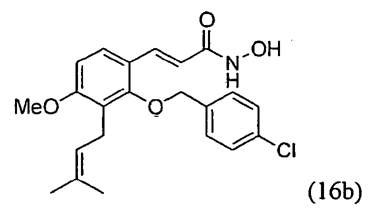

¹H-NMR (500 MHz, MeOH-d4): δ7.83 (1H, d, J=15.8 Hz), 7.47 (1H, d, J=8.7 Hz), 7.44 (2H, d, J=8.2 Hz), 7.37 (2H, d, J=8.3 Hz), 6.81 (1H, d, J=8.7 Hz), 6.36 (1H, d, J=15.8 Hz), 5.09 (1H, t, J=6.5 Hz), 4.76 (2H, s), 3.84 (3H, s), 3.32 (1H, d, J=6.6 Hz), 1.63 (3H, s), 1.62 (3H, s). (trans)

### (E)-2-(4-Bromobenzoxy)- 3-prenyl-4-methoxy-N-hydroxy cinamide (16c)

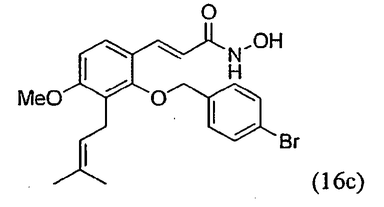

¹H-NNM (500 MHz, acetone-d6): δ7.86 (1H, d, J=15.8 Hz), 7.60 (2H, d, J=8.4 Hz), 7.51 (1H, d, J=8.7 Hz), 7.49 (2H, d, J=8.3 Hz), 6.86 (1H, d, J=8.7 Hz), 6.50 (1H, d, J=15.8 Hz), 5.14 (1H, t, J=6.7 Hz), 4.80 (2H, s), 3.87 (3H, s), 3.36 (1H, d, J=6.7 Hz), 1.65 (3H, s), 1.61 (3H, s). (trans)

### (E)-2-(3,4,5-Trimethoxybenzoxy)- 3-prenyl-4-methoxy-N-hydroxy cinamide (16d)

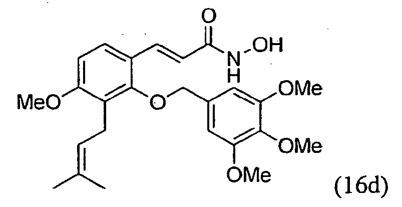

¹H-NMR (500 MHz, CDCl₃): δ7.88 (1H, d, J=15.7 Hz), 7.35 (1H, d, J=8.6 Hz), 6.68 (2H, s), 6.62 (1H, d, J=8.7 Hz), 6.28 (1H, d, J=15.7 Hz), 5.18 (1H, t, J=6.0 Hz), 4.68 (2H, s), 3.86 (6H, s), 3.84 (3H, s), 3.82 (3H, s), 3.38 (2H, d, J=6.4 Hz), 1.73 (3H, s), 1.66 (3H, s). (trans)

### (E)-2-Benzoxy-3-prenyl-4-methoxy-N-(2-aminophenyl) cinamide (17a)

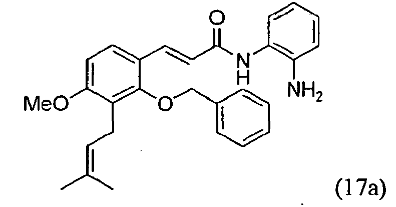

¹H-NMR (500 MHz, CDCl₃): δ 7.87 (1H, d, J=15.1 Hz), 7.40-7.35 (5H, m), 7.32 (1H, d, J=7.8 Hz), 7.03 (2H, m), 6.77 (2H, m), 6.67 (1H, d, J=8.1 Hz), 6.49 (1H, d, J=15.6 Hz), 5.19 (1H, m), 4.79 (2H, s), 3.85 (3H, s), 3.40 (2H, d, J=6.1 Hz), 1.70 (3H, s), 1.67 (3H, s)

### (E)-2-(4-Methoxybenzoxy)-3-prenyl-4-methoxy-N-(2-aminophenyl) cinamide

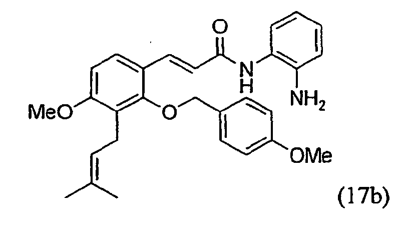

¹H-NMR (500 MHz, CDCl₃): δ 7.85 (1H, d, J=15.1 Hz), 7.39 (4H, d, J=7.6 Hz), 7.12-7.04 (2H, m), 6.90 (1H, d, J=8.2 Hz), 6.78 (2H, d, J=7.3 Hz), 6.68 (1H, d, J=8.1 Hz), 6.54 (1H, d, J=15.2 Hz), 5.19 (1H, m), 4.73 (2H, s), 3.86 (3H, s), 3.77 (3H, s), 3.41 (2H, m), 1.74 (3H, s), 1.67 (3H, s)

### (E)-2-(4-Fluorobenzoxy) -3-prenyl-4-methoxy-N-(2-aminophenyl) cinamide (17c)

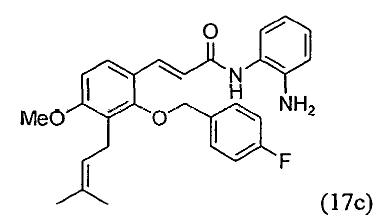

¹H-NMR (500 MHz, CDCl₃): δ 7.90 (1H, d, J=15.2 Hz), 7.43-7.38 (4H, m), 7.07-7.03 (4H, m), 6.76 (2H, d, J=7.0 Hz), 6.65 (1H, d, J=8.4 Hz), 6.51 (1H, d, J=15.7 Hz), 5.16 (1H, m), 4.76 (2H, s), 3.84 (3H, s), 3.34 (2H, d, J=6.5 Hz), 1.69 (3H, s), 1.66 (3H, s)

### (E)-2-(4-Chlorobenzoxy)-3-prenyl-4-methoxy-N-(2-aminophenyl) cinamide (17d)

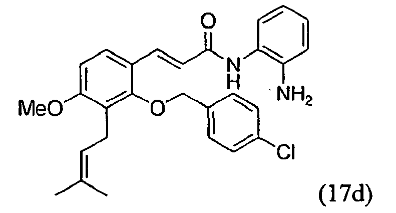

¹H-NMR (500 MHz, CDCl₃): δ 7.88 (1H, d, J=15.4 Hz), 7.39-7.35 (4H, m), 7.34 (1H, d, J=7.9), 7.05 (2H, m), 6.78 (2H, m), 6.69 (1H, d, J=8.0 Hz), 6.50 (1H, d, J=15.4 Hz), 5.15 (1H, m), 4.78 (2H, s), 3.86 (3H, s), 3.34 (2H, d, J=6.3 Hz), 1.68 (3H, s), 1.66 (3H, s)

### (E)-2-(4-Bromobenzoxy) -3-prenyl-4-methoxy-N-(2-aminophenyl) cinamide (17e)

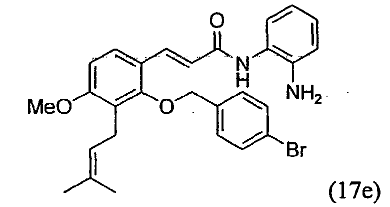

¹H-NMR (500 MHz, CDCl₃): δ 7.88 (1H, d, J=15.3 Hz), 7.50 (2H, d, J=7.8 Hz), 7.40 (1H, d, J=7.4 Hz), 7.34 (2H, m), 7.05 (2H, m), 6.81 (2H, m), 6.71 (1H, d, J=7.6 Hz), 6.52 (1H, d, J=15.5 Hz), 5.15 (1H, m), 4.77 (2H, s), 3.86 (3H, s), 3.37 (2H, m), 1.66 (3H, s), 1.57 (3H, s)

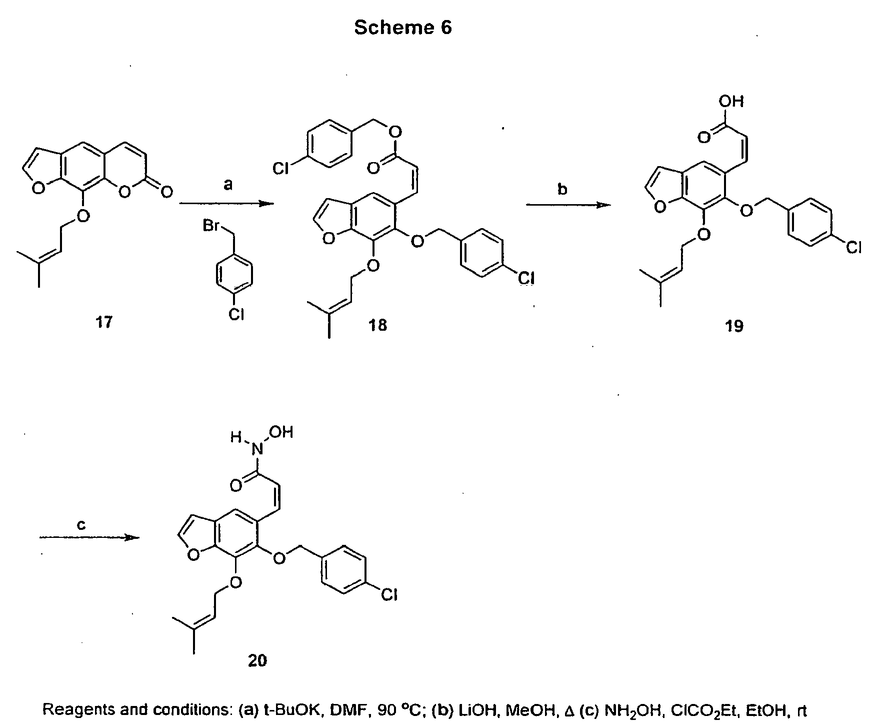

### (Z)-3-[6-(4-Chlorobenzoxy)-5-isoprenyloxybenzofuran-5-yl] 4-chlorobenzyl acrylate (18)

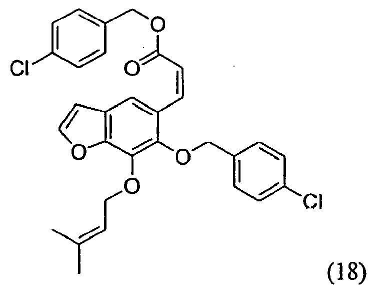

### (Z)-3-[6-(4-Chlorobenzoxy)-5-isoprenyloxybenzofuran-5-yl] 4-chlorobenzyl acrylate (18)

Following the procedure described as 2 gave **18.**
¹H-NMR (500 MHz, CDCl₃) δ7.61 (1H, d, J=2.0 Hz), 7.44 (1H, s), 7.36 (2H, d, J=8.3 Hz), 7.31 (2H, d, J=8.3 Hz), 7.23 (2H, d, J=8.3 Hz), 7.18 (1H, d, J=12.3 Hz), 7.11 (2H, d, J=8.3 Hz), 6.66 (1H, d, J=1.9 Hz), 5.99 (1H, d, J=12.3 Hz), 5.58 (1H, t, J=7.1 Hz), 5.06 (2H, s), 5.02 (2H, s), 4.86 (2H, d, J=7.1 Hz), 1.78 (3H, s), 1.71 (3H, s).

### (Z)-3-[6-(4-Chlorobenzoxy)-5-isoprenyloxybenzofuran-5-yl]acrylate(19)

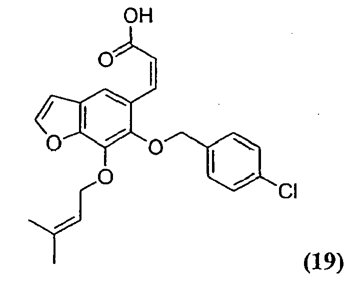

Following the procedure described as **3** gave **19.**
¹H-NMR (500 MHz, CDCl₃) δ7.58 (1H, d, J=2.0 Hz), 7.53 (1H, s), 7.33 (2H, d, J=8.3 Hz), 7.30 (2H, d, J=8.3 Hz), 7.22 (1H, d, J=12.5 Hz), 6.71 (1H, d, J=2.1 Hz), 5.92 (1H, d, J=12.4 Hz), 5.56 (1H, t, J=7.2 Hz), 5.02 (2H, s), 4.85 (2H, d, J=7.1 Hz), 1.75 (3H, s),1.66(3H,s).

### (Z)-3-[6-(4-Chlorobenzoxy)-5-isoprenyloxybenzofuran-5-yl]-N-hydroxyacrylmide(20)

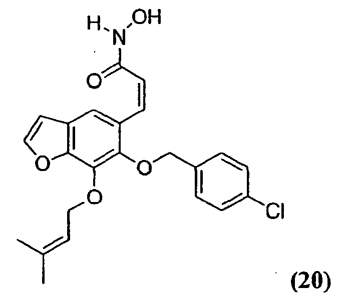

Following the procedure described as **4** gave **20.**
¹H-NMR (500 MHz, CDCl₃) δ7.58 (1H, s), 7.35∼7.30 (4H, m), 7.32 (1H, s), 7.00 (1H, d, J=12.4 Hz) (1H, s), 5.86 (1H, d, J=12.5 Hz), 5.56 (1H, t, J=6.9 Hz), 5.50 (2H, s), 4.89 (2H, d, J=7.1 Hz), 1.76 (3H, s), 1.69 (3H, s)

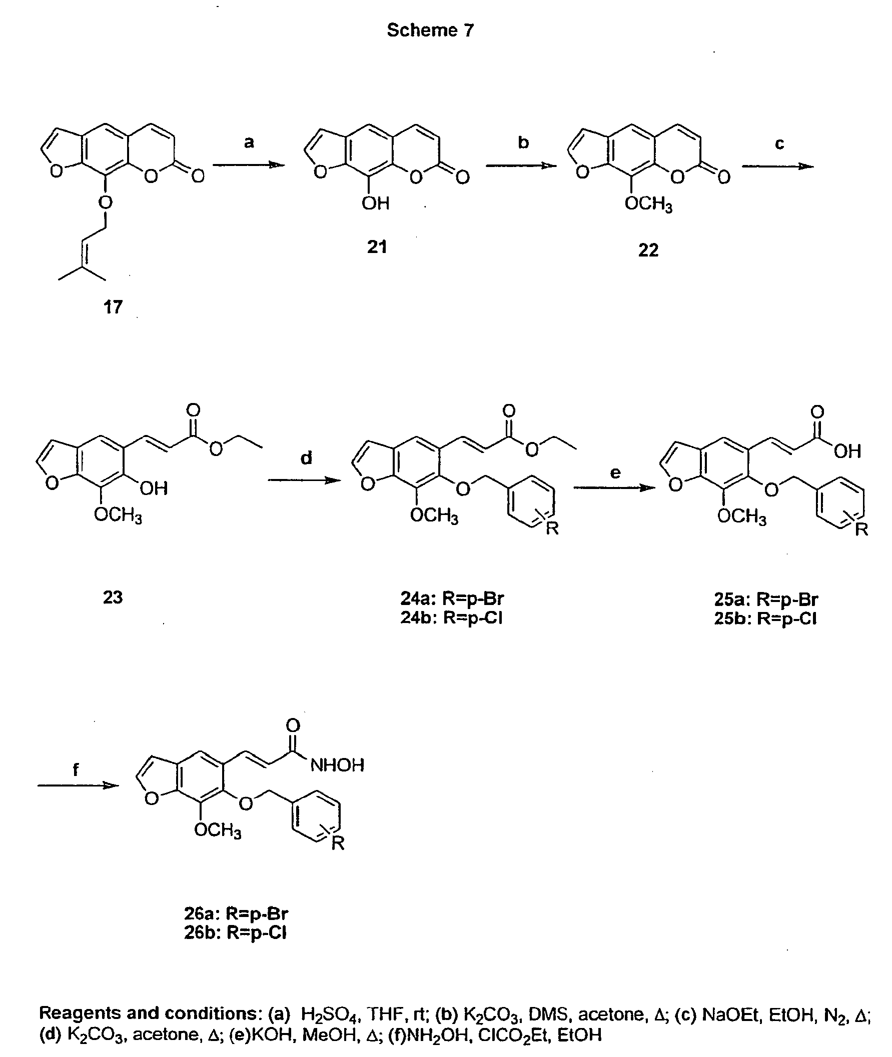

To a solution of 1 (18.51mmol) in THF (150 mL) was added 49%H₂SO₄ (100 mL) and stirred at room temperature for 6 h. The resulting solution was extracted with CH₂Cl₂ (50 mL × 3) and then dried over Na₂SO₄ to give residue. The residue was purified by silica gel (EtOAc : *n*-Hexane=1:2). 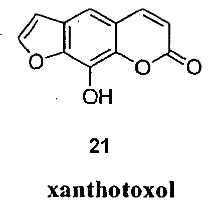 ¹H-NMR (500 MHz, *d*₆-acetone):δ 8.02(1H, d, J=9.5 Hz), 7.91 (1H, d, J=2.1 Hz), 7.41 (1H, s), 6.96(1H, d, J=2.1 Hz), 6.32 (1H, d, J=9.5 Hz)

### Preparation of 8-Methyl-xanthotoxol (22)

Compound 21(1 g, 4.95 mmol) dissolved in acetone (150 mL). To the mixture of the solution of 1 and K₂CO₃ (1.7 g, 12.4 mmol) was added dimethyl sulfate (0.96 mL, 5.85 mmol), the resulting solution was refluxed for 2 hour under nitrogen. After filteration to remove K₂CO₃, the filtrate was condensed under reducted pressure and then diluted with EtOAc (50 mL), washed with dis-H₂O (25 mL x3) and dried over Na₂SO₄. After removal of EtOAc under reduced pressure, the residue was purified by silica gel (EtOAc: *n*-Hexane=1:2) to give **22**

### 8-Methyl-xanthotoxol (22)

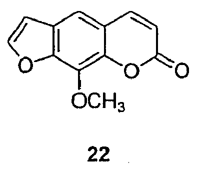

¹H-NMR (500 MHz, CDCl₃): δ 7.75(1H, d, J=9.5 Hz), 7.67 (1H, d, J=5.0 Hz), 7.32 (1H, s), 6.80(1H, d, J=5.0 Hz), 6.35 (1H, d, J=9.5 Hz)

To the solution of **22**(1.06 g, 4.94 mmol) in dry EtOH (25 mL) was added the mixture of sodium ethoxide (0.50 g, 7.41 mmol) in dry EtOH (25 mL) dropwise, the resulting solution was refluxed under nitrogen overnight and then diluted with dis-H₂O (50 mL), neutralized with 1N HCl_{(aq)} to pH 4-5, extracted with EtOAc (50 mL x 3) and dried over Na₂SO₄. After removal of EtOAc under reduced pressure, the residue was purified by silica gel (EtOAc: *n*-Hexane=2:1) to give **23.**

### (E)-3-(6-Hydroxy-5-methoxybenzofuran-5-yl)-4-ethyl acrylate (23)

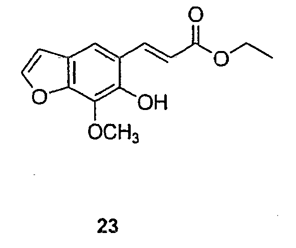

¹H-NMR (500 MHz, CDCl₃) δ8.03 (1H, d, J=16.0 Hz), 7.51 (1H, d, J=2.0 Hz), 7.35 (1H, s), 6.68 (1H, d, J=2.0 Hz), 6.63 (1H, d, J=16.5 Hz), 4.29 (2H, q, J=7.1 Hz), 4.21 (3H, s), 1.33 (3H, t, J=7.2 Hz).

### General procedure for the preparation of 24

To the mixture of **23**(1.96 mmol) and K₂CO₃ (4.9 mmol) in acetone (20 mL) was added apropriate benzyl bromide (3.92 mmol), the resulting solution was refluxed under nitrogen overnight. After filteration to remove K₂CO₃, the filtrate was condensed under reducted pressure and then diluted with EtOAc (50 mL), washed with dis-H₂O (25 mL x3) and dried over Na₂SO₄. After removal of EtOAc under reduced pressure, the residue was purified by silica gel (EtOAc: *n*-Hexane=1:10) to give **24.**

### (E)-3-[6-(4-Bromobenzoxy)-5-methoxybenzofuran-5-yl]-4-ethyl acrylate (24a)

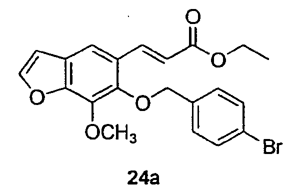

¹H-NMR (500 MHz, CDCl₃) δ 8.00 (1H, d, J=16.1 Hz), 7.60 (1H, d, J=2.0 Hz), 7.50 (2H, d, J=8.3 Hz), 7.43 (1H, s), 7.35 (2H, d, J=8.3 Hz), 6.72 (1H, d, J=2.0 Hz), 6.41 (1H, d, J=16.1 Hz), 5.00 (2H, s), 4.27 (2H, q, J=7.0 Hz), 4.17 (3H, s), 1.34 (3H, t, J=7.1 Hz).

### (E)-3-[6-(4-Chlorobenzoxy)-5-methoxybenzofuran-5-yl]-4-ethyl acrylate (24b)

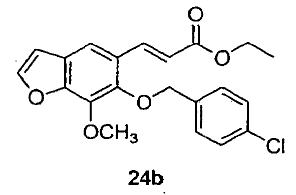

¹H-NMR (500 MHz, CDCl₃) δ 8.00 (1H, d, J=16.0 Hz), 7.61 (1H, d, J=2.5 Hz), 7.44 (1H, s), 7.41 (2H, d, J=8.0 Hz), 7.34 (2H, d, J=8.0 Hz), 6.73 (1H, d, J=2.0 Hz), 6.41 (1H, d, J=16.0 Hz), 5.02 (2H, s), 4.27 (2H, q, J=7.0 Hz), 4.18 (3H, s), 1.34 (3H, t, J=7.0 Hz).

### General procedure for the preparation of 25

The mixture of **24**(1.68 mmol) and 10% KOH/MeOH (20 mL) was refluxed for 6 hour under N₂ and then diluted with dis-H₂O (100 mL), acdified with 2N HCl to pH 2∼3 and extracted with EtOAc (50 mL x 3), respectively. The combined EtOAc layer was dried over Na₂SO₄ and concentrated under reduced pressure to give **25.**

### (E)-3-[6-(4-Bromobenzoxy)-5-methoxybenzofuran-5-yl] acrylate (25a)

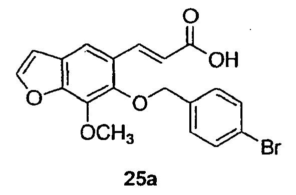

¹H-NMR (500 MHz, *d*₆-acetone): δ 8.02 (1H, d, J=16.1 Hz), 7.89 (1H, d, J=2.1 Hz), 7.79 (1H, s), 7.58 (2H, d, J=8.3 Hz), 7.49 (2H, d, J=8.2 Hz), 6.92 (1H, d, J=2.1 Hz), 6.50 (1H, d, J=16.0 Hz), 5.10 (2H, s), 4.19 (3H, s)

### (E)-3-[6-(4-Chlorobenzoxy)-5-methoxybenzofuran-5-yl]-acrylate (25b)

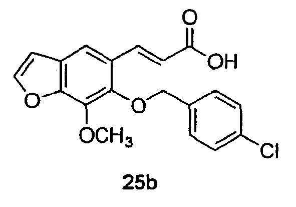

¹H-NMR (500 MHz, CDCl₃): δ 8.11 (1H, d, J=16.0 Hz), 7.63 (1H, d, J=2.0 Hz), 7.48 (1H, s), 7.41 (2H, d, J=8.5 Hz), 7.36 (2H, d, J=8.0 Hz), 6.75 (1H, d, J=2.0 Hz), 6,44 (1H, d, J=116.0 Hz), 5.04 (2H, s), 4.19 (3H, s)

### General procedure for the prepartion of 26 (compounds 26a and 26b are not according to the present invention)

[0057] To a solution of potassium hydroxide (379 mg, 6,76 mmol) in MeOH (1.8 mL) was added hydroxylamine hydrochloride (470 mg, 6.76 mmol) in MeOH (4.7 mL) dropwiase for 1 h. Filtration to remove the white salt gave the free hydroxylame in MeOH solution. To the mixture of 23 (1.69 mmol) in dry THF (25 mL) was added ethyl chloroformat (0.3 mL, 2.62 mmol) and triethylamine (0.4 mL, 6.38 mmol) and stirred at for 0.5 h and then added the prepared free hydroxylamine solution under N₂. After reaction for 2h, the reaction mixture was diluted with dis-H₂O (100 mL), acdified with 1N HCl to pH 2-3 and extracted with EtOAc (50 mL x 3), respectively. The combined EtOAc layer was dried over Na₂SO₄ and concentrated under reduced prassure to give residue. The residue was purified by silica gel (EtOAc: *n*-Hexane=1:1) to give 26.

### (E)-3-[6-(4-Bromobeazoxy)-5-Methoxybenzfuran-5-yl]-N-hydroxyacrylmide (26a)

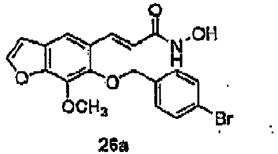

¹H-NMR (500 MHz, CD₃OD) δ 7.92 (1H, d, J=16,0 Hz), 7,75 (1H, d, J=2.0 Hz), 7.50 (2H, d, J-8.0Hz), 7.47 (1H, s), 7.39 (2H, d, J=8.0 Hz), 6.81 (1H, d, J=2.0 Hz), 6.43 (1H, d, J=16.0 Hz), 5.07 (2H, s), 4.14 (3H,s)

### (E)-3-[6-(4-Chlorobenzoxy)-5-Methoxybenzofuran-5-yl]-N-hydroxyacrylmide (26b)

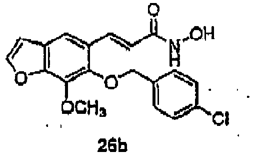

¹H-NMR (500 MHz, *d*₆-acetone) δ 7.96 (1H, d, J=15.8 Hz), 7.83 (1H, d, J=2.0 Hz), 7.53 (2H, d, J=8.1 Hz), 7.52 (1H, s), 7.38 (2H, d, J=8.1 Hz), 6.85 (1H, d, J=2.0 Hz), 6.59 (1H, d, J=15.5 Hz), 5.05 (2H, s), 4.15 (3H, s)

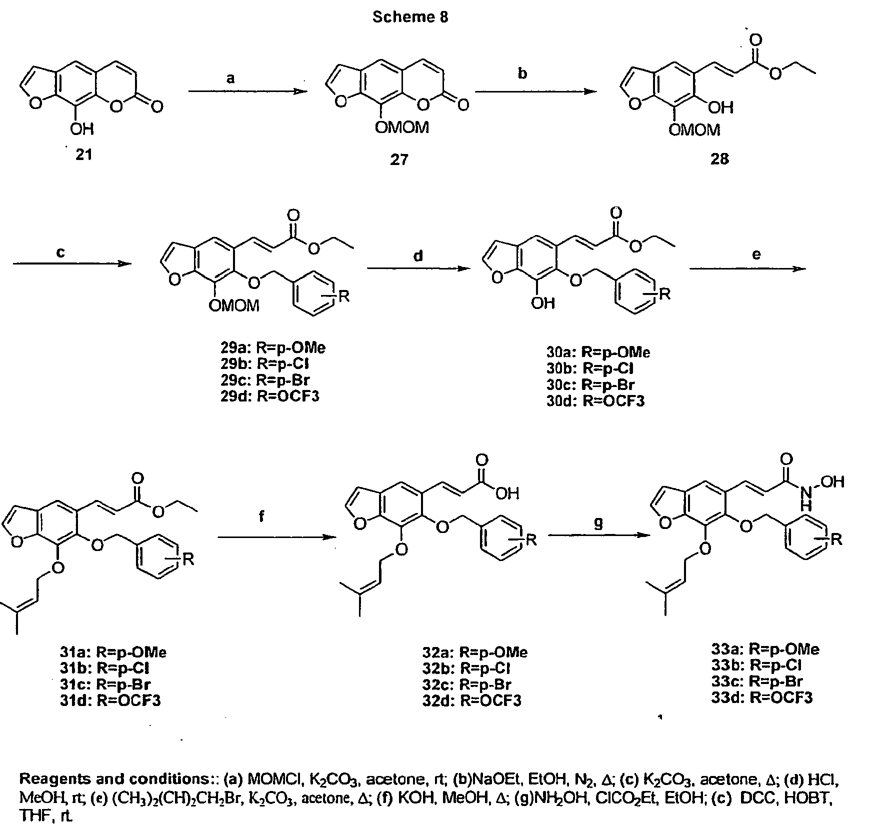

### 8-Methoxyrnethyl-xanthotoxol (27)

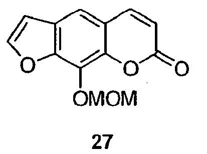

To the mixture of xanthotoxol (**21**), K₂CO₃ (2.5 eq.) and acetone was added MOMCl (2 eq.), the resulting solution was stirred at room temperature for 12h. After removal of acetone, the residue was diluted with EtOAc, washed with dis-H₂O and dried over Na₂SO₄. The EtOAC layer was concentrated under reduced pressure. The residue was subjected to silica gel chromatography to give **27.**

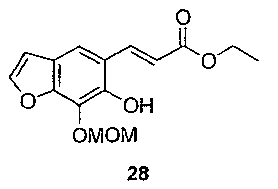

Following the procedure similar to that of **13** gave **28.**

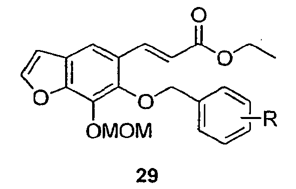

### General procedure for the preparation of 29

Following the procedure similar to that of **14** gave **29.**

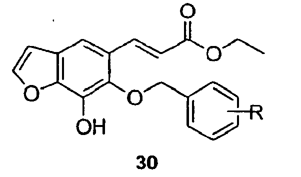

### General procedure for the preparation of 30

To the mixture of 29 and MeOH was added 37% HCl(aq), the resulting solution was stirred at rt for 2h, then diluted with dis-H2O and extracted with EtOAc. The combined organic layer was dried over Na₂SO₄ and concentrated under reduced pressure. The residue was subjected to silica gel to give **30.**

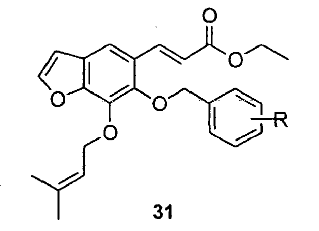

### General procedure for the preparation of 31

To the mixture of 30, K2CO3 (2.5 eq.) and acetone was added isoprenyl bromide (2 eq.), the resulting solution was stirred at room temperature for 24h. After removal of acetone, the residue was diluted with EtOAc, washed with dis-H₂O and dried over Na₂SO₄. The EtOAC layer was concentrated under reduced pressure. The residue was subjected to silica gel chromatography to give **31.**

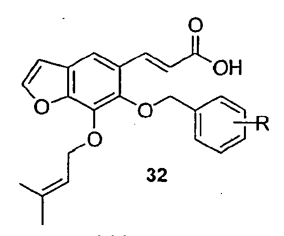

### General procedure for the preparation of 32

Following the procedure described as **3** gave **32.**

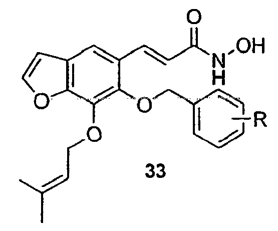

### General procedure for the preparation of 33

Following the procedure described as **4** gave **33.**

### Salts

Pharmaceutically acceptable salts of the compounds of the present invention can be prepared by any conventional means. Exemplified processes for synthesizing the salts are provided below.

### (E)-2-(4-Methoxybenzoxy)-4-methoxy-3-prenyl-N-hydroxycinamide lysine salt

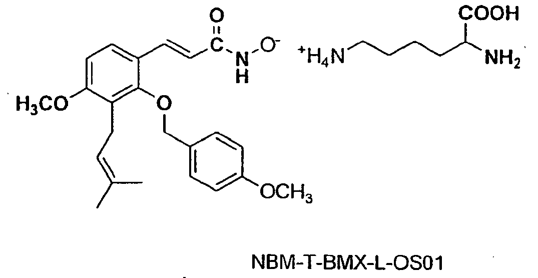

To the mixture of hydroxamate (1mmole) and EtOH (15 mL) was added a solution of L-lysine (1 mmole) in H₂O (10 mL). The resultant was stirred at 40°C for 4 h, cooled to room temperature, then stirred for additional 12 h. The solvent was removed under reduced pressure and the residue was precipitate from EtOH. After filtration, the solid was wash with dis-H₂O and dried in a vacuum oven.

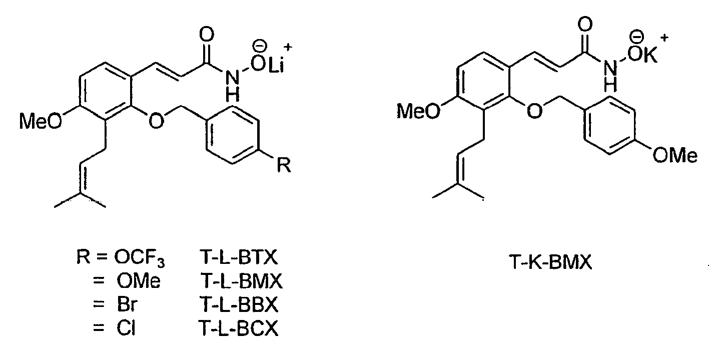

To the mixture of hydroxamate (1mmole) and EtOH (15 mL) was added a solution of lithium hydroxide or potassium hydroxide (1.2 mmole) in H₂O (5 mL). The resultant was stirred at room temperature overnight. The solvent was removed under reduced pressure and the residue was precipitate from EtOH. After filtration, the solid was wash with dis-H₂O and dried in a vacuum oven.

### Pharmaceutical Composition of the Invention

The compounds of formula (I) and pharmaceutically acceptable salts, stereoisomers, enantiomers and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 10 to 30 wt% (percent by weight), more preferably from 30 to 50 wt%, still more preferably from 50 to 70 wt%, and even more preferably from 70 to 100 wt%, of the active ingredient, all percentages by weight being based on total composition. In addition, the pharmaceutical composition of the invention may further comprise other agents for the prevention or treatment of diseases associated with histone deacetylase (HDAC).

The pharmaceutical compositions may be administered systemically, e.g., by oral administration in the form of tablets, capsules, syrups, powders or granules; or by parenteral administration in the form of solutions or suspensions; or by subcutaneous administration; or by rectal administration in the form of suppositories; or transdermally.

The compounds and pharmaceutical compositions of the invention are an HDAC inhibitor and can be retained long in the cells and continuously induce the acetylation of histone H4. They are HDAC inhibitors inducing differentiation of cells and neural stem cells. In addition, the compounds of the invention significantly inhibit HDAC activity. The compounds of the invention significantly decrease both S and G2/M phases of the cells in a dose-dependent manner and change the morphology of cancer cells. Therefore, the compounds of the invention can treat tumor or cell proliferative disease. Moreover, the compounds of the invention can enhance the neurite outgrowth and treat neurodegenerative diseases (such as Huntington's disease and poly-glutamine disease) and human spinal muscular atrophy (SMA).

### EXAMPLE

The following examples illustrate preferred methods for synthesizing and using the compounds:

### Example 1 Preparation of 2-(4-Nitrobenzoxy)-4-methoxy-3-prenyl-4-nitrobenzyl cinamate

To the mixture of 1 (2 g, 8.20 mmol) and potassium *t*-butoxide (1.84 g, 16.4 mmol) in dry DMF (20 mL) were added various benzyl chlorides (16.4 mmol), and the resulting solution was stirred at room temperature under nitrogen for 6 h and then diluted with EtOAc (50 mL), washed with dis-H₂O (25 mL x3) and dried over Na₂SO₄. After removal of EtOAC under reduced pressure, the residue was purified by silica gel (EtOAc: *n*-Hexane=1:10∼1:1) to give the title compound: ¹H-NMR (400 MHz, CDCl₃) 8.24-8.22 (4H, m), 7.59-7.46 (4H, m), 7.45 (1H, d, J=8.6 Hz), 6.98 (1H, d, J=12.3 Hz), 6.69 (1H, d, J=8.6 Hz), 5.81 (1H, d, J=12.3 Hz), 5.12 (1H, t, J=6.5 Hz), 4.91 (2H, s), 4.64 (2H, s), 3.85 (3H, s), 3.32 (2H, d, J=6.6 Hz), 1.61 (3H, s), 1.57 (3H, s).

### Example 2 Preparation of 2-Benzoxy-4-methoxy-3-prenyl cinamate

The mixture of 2 (11.36 mmol) and 10% KOH/MeOH (40 mL) was refluxed overnight under N₂ and then diluted with dis-H₂O (100 mL), acidified with 2N HCl to pH 5-6 and extracted with EtOAC (50 mL x 3). The combined EtOAc layer was dried over Na₂SO₄ and concentrated under reduced pressure. The residual was purified by silica gel (EtOAc : *n-*Hexane= 1: 2) to give the title compound: ¹H-NMR (400 MHz, CDCl₃) 7.63 (1H, d, J=8.6 Hz), 7.42-7.26 (5H, m), 7.25 (1H, d, J=12.5 Hz), 6.80 (1H, d, J=8.7 Hz), 5.88 (1H, d, J=12.5 Hz), 5.16 (1H, t, J=6.6 Hz), 4.82 (2H, s), 3.85 (3H, s), 3.36 (2H, d, J=6.7 Hz), 1.65 (3H, s), 1.62 (3H, s).ESIMS m/z [M-H]⁻ 351.13 (100).

### Example 3 Preparation of 2-Benzoxy-4-methoxy-3-prenyl-N-hydroxy cinamamide

To a solution of potassium hydroxide (637 mg, 11.36 mmol) in MeOH (4mL) was added hydroxylamine hydrochloride (790 mg, 11.36 mmol) dropwise, and then the solution was stirred in an ice bath for 1 h. Filtration was performed to remove the white salt to give free hydroxylame in MeOH solution. To the mixture of 3a (1 g, 2.84 mmol) in dry THF (25 mL) were added ethyl chlorofonnat (0.6 mL, 5.68 mmol) and triethylamine (0.6 mL, 5.68 mmol), the mixture was stirred for 0.5 h, and then the prepared free hydroxylamine solution was added. After reaction for 3 h, the reaction mixture was concentrated under reduced pressure to give residual. The residual was purified by silica gel (EtOAc : *n*-Hexane= 1 : 2) to give the title compound: ¹H-NMR (400 MHz, CDCl₃) 7.40-7.33 (5H, m), 7.30 (1H, d, J=8.6 Hz), 7.24 (1H, d, J= 8.7 Hz), 7.01 (1H, d, J= 12.5 Hz), 6.66 (1H, d, J=8.7 Hz), 5.79 (1H, d, J=12.4 Hz), 5.17 (1H, t. J=6.6 Hz), 4.82 (2H, s), 3.83 (3H, s), 3.37 (2H, d, J=6.7 Hz), 1.69 (3H, s), 1.66 (3H, s); ¹³C-NMR (100 MHz, CDCl₃) 159.7 (s), 159.2 (s), 155.9 (s), 136.9 (s), 135.9 (d), 131.8 (s), 128.6 (d), 128.5 (d), 128.2 (d), 128.1 (d), 128.0 (d), 123.9 (s), 122.7 (d), 121.1 (s), 118.2 (d), 106.8 (d), 76.6 (t), 55.7 (q), 25.7 (q), 23.2 (t), 17.9 (q), 14.4 (q); ESIMS m/z [M+H]⁺ 368.13 (100).

### Example 4 Preparation of 2-Benzoxy-4-methoxy-3-(2-Hydroxy-2-methylbutyl) -N-hydroxy cinamamide

To a solution of 4a (100 mg, 0.27 mmol) in THF (15 mL) was added 49% H₂SO₄ (10 mL) and the solution was stirred at room temperature for 6 hours. The resulting solution was extracted with CH₂Cl₂ (50 mL x 3) and then dried over Na₂SO₄ to give residue. The residue was purified by silica gel (EtOAc : *n*-Hexane= 1: 1) to give the title compound: ¹H-NMR (400 MHz, CDCl₃). 7.40-7.33 (5H, m), 7.30 (1H, d, J=8.6 Hz), 7.00 (1H, d, J= 12.4 Hz), 6.69 (1H, d, J=8.6 Hz), 5.89 (1H, d, J=12.4 Hz), 4.83 (2H, s), 3.82 (3H, s), 2.66 (2H, t, J=7.8 Hz), 1.65 (2H, t, J=7.8 Hz), 1.16 (6H, s)..

### Example 5 Preparation of 2-Benzoxy-4-methoxy-3-prenyl-N-(2-aminophenyl)cinamamide

The mixture of 3 (17.04 mmol), HOBT (2.76 g, 20.44 mmol) and DCC (4.22 g, 20.44 mmol) in dry THF (30 mL) was stirred at room temperature for 0.5 h and then o-phenylenediamine (1.84 g, 17.04 mmol) was added. The resulting solution was continuously stirred overnight and then concentrated under reduced pressure to give residue. The residue was dissolved in CH₂Cl₂ (50 ml), washed with saturated NaHCO₃ (25 mL x 3), and dried over Na₂SO₄. The organic layer was evaporated under reduced pressure and then purified by silica gel (EtOAc : *n*-Hexane= 1 : 3-1 : 1) to give the title compound: ¹H-NMR (400 MHz, CDCl₃) 7.43-7.38 (5H, m), 7.37 (1H, d, J=8.6 Hz), 7.26 (1H, d, J= 8.7 Hz), 7.03 (1H, d, J= 12.5 Hz), 7.01-7.02 (2H, m), 6.74-6.72 (2H, m), 6.68 (1H, d, J=8.6 Hz), 6.06 (1H, d, J=12.4 Hz), 5.14 (1H, t. J=6.6 Hz), 4.90 (2H, s), 3.84 (3H, s), 3.65 (2H, s), 3.38 (2H, d, J=6.5 Hz), 1.72 (3H, s), 1.65 (3H, s).

### Example 6 Preparation of 2-Benzoxy-4-methoxy-3-(2-Hydroxy-2-methylbutyl) -benzyl cinamamate

To a solution of 2 (0.27 mmol) in THF (15 mL) was added 49% H₂SO₄ (10 mL) and the solution was stirred at room temperature for 6 h. The resulting solution was extracted with CH₂Cl₂ (50 mL x 3) and then dried over Na₂SO₄ to give residue. The residue was purified by silica gel (EtOAc : *n*-Hexane= 1 : 1) give the title compound: ¹H-NMR (400 MHz, CDCl₃) 7.38 (1H, d, J=8.7 Hz), 7.36-7.27 (10H, m), 7.00 (1H, d, J= 12.4 Hz), 6.60 (1H, d, J=8.7 Hz), 5.94 (1H, d, J=12.4 Hz), 5.13 (2H; s), 4.81 (2H,s), 3.82 (3H, s), 2.67 (2H, t, J=8.2 Hz), 1.61 (2H, t, J=8.2 Hz), 1.18 (6H, s); ESIMS m/z [M+Na]⁺ 483.6(100).

### Example 7 Inhibition of cancer cell growth by the compounds of the invention

Three cancer cell lines, Rat C6 giloma cells, Human breast cancer MCF-7 cells, and Human lung cancer A549 cells, were cultured in Dulbecco's modified Eagle's medium (DMEM; Gibco) containing 10% fetal bovine serum (FBS) and 1% penicillin/ streptomycin. All three cell lines were maintained at 37 in a humidified atmosphere of 95% air and 5% CO₂. For the experiments, the cells were seeded in 6-well plates. After 24 hours, the cells were treated with different concentrations of the various compounds. The cells were observed at 24, 48, and 72 hours. Inhibition of cancer cell growth of NBM-HB-OS01 in various concentrations after 48 hours in Rat C6 glioma cells (see Fig. 1(a)) or in Human colon cancer HT-29 cells (see Fig. 1(b)) was shown. NBM-C-BX-OS01 arrested the cell growth in various concentrations in Rat C6 glioma cells in 48 hours (see Fig. 2(a)), in human breast cancer MCF-7 cells in 24 hours (see Fig. 2(b)) and in human lung cancer A549 cells in 48 hours (see Fig. 2(c)). Treatments with NBM-C-BA-OS01, NBM-C-BCA-OS01 and NBM-C-BMA-OS01 inhibited the Rat C6 glioma cell growth in a fixed concentration (7.5µg/mL) in 48 hours (see Fig. 4). NBM-C-BA-OS01 (5µg/mL), NBM-C-BCX-OS01 (2.5, 5.0 µg/mL), and NBM-C-BMX-OS01 (2.5, 5.0 µg/mL) inhibited the cell growth of Rat C6 glioma cells in 24 hours (see Fig. 5(a)).

### Example 8 Inhibition of cancer cell growth and change of the morphology by the compounds of the invention

Five cancer cell lines, Rat C6 giloma cells, Human breast cancer MCF-7 cells, Human glioma Hs683 cells, Human glioblastoma 05-MG cells, and Human lung cancer A549 cells, were cultured in Dulbecco's modified Eagle's medium (DMEM; Gibco) containing 10% fetal bovine serum (FBS) and 1% penicillin/streptomycin, and were kept at 37°C in a humidified atmosphere of 95% air and 5% CO₂. Human breast cancer MDA-MB-231 cells were cultured in L-15 medium (Gibco) containing 10% fetal bovine serum (FBS), 1% penicillin/streptomycin, and 2 mM glutamine, and were maintained at 37°C in a humidified atmosphere of 95% air and 0% CO₂. For these experiments, the cells were seeded in 6-well plates or a 60-mm dish. After 24 hours, the cells were treated with different concentrations of the various compounds. The cells were observed at 24, 48and 72 hours. Human breast cancer MCF-7 cells (see Fig. 3(a)), and Rat C6 glioma cells (see Fig. 3(b)) exhibited changes in morphology in response to NBM-C-BA-OS01 2.5, 5.0, 7.5 µg/mL treatment for 72 hours. Treatments with NBM-C-BX-OS01 (7.5 µg/mL), NBM-C-BCX-OS01 (2.5, 5.0, 7.5 µg/mL) and NBM-C-BMX-OS01 (2.5, 5.0, 7.5 µg/mL) induced A549 cell growth inhibition in dose-dependent amounts for 72 hours (see Fig. 5(b)) and the results were similar to those in Rat C6 glioma cells for 24 hours (see Fig. 5(c)). Human glioma Hs683 cells were treated with NBM-C-BX-OS01 (1.25, 2.5, 5.0 µg/mL) for 72 hours (see Fig. 5(d)). Human glioblastoma 05-MG cells were treated with NBM-C-BCX-OS01 (1.0, 2.0, 4.0 µg/mL) and NBM-C-BMX-OS01 (1.0, 2.0, 4.0 µg/mL) for 72 hours (see Fig. 5(e)). The results obtained by cell counting showed the same tendency (see Fig. 5(f)). NBM-C-BCX-OS01, NBM-C-BMX-OS01 and NBM-C-BFX-OS01 inhibited the growth of human breast cancer MDA-MB-231 cells and changed the morphology of the cells for 72 hours (see Fig. 6(a)). The results were counted by a trypan blue exclusion assay (see Fig. 6(b)).

### Example 9 Effects of the invention (NBM-HB-OS01) on mRNA expression of Rat C6 glioma cells

The cell-cycle related mRNA expression was examined by RT-PCR. The total RNA was isolated from the treated Rat C6 glioma cells using the RNeasy Mini Kit (Qiagen) as described by the manufacturer. The cDNA was produced from 500 ng of total RNA using ReverTra-Plus-TM (TOYOBO). The RT product (1µl) was amplified by PCR with primers for amplification of several genes (p21, cyclin B1, and cyclin D1) for the cell cycle analyses and GAPDH was used as an internal control. Rat C6 glioma cells were treated with NBM-HB-OS01 for 48 hours, and as shown in Fig. 1 (d), NBM-HB-OS01 induced p21 mRNA expression.

### Example 10 Effects of the invention on the cell cycle of various human cancer cells

Human lung cancer A549 cells, Human glioma Hs683 cells and Human glioblastoma 05-MG cells were seeded 1×10⁶ cells in a 100-mm dish. After 24 hours of incubation in DMEM+10% BSA, Human lung cancer A549 cells were treated with NBM-HB-OS01 (2.5, 5.0, 7.5, 10.0 µg/mL) for 24 hours and were treated with NBM-C-BCX-OS01 (2.5, 5.0, 7.5 µg/mL) and NBM-C-BMX-OS01 (2.5, 5, 7.5 µg/mL) for 72 hours. Human glioblastoma 05-MG cells were treated with NBM-C-BCX-OS01 (1.0, 2.0, 4.0 µg/mL) and NBM-C-BMX-OS01 (1.0, 2.0, 4.0 µg/mL) for 72 hours. Human glioma Hs683 cells were treated with NBM-C-BCX-OS01 (1.0, 2.0, 4.0 µg/mL). For cell cycle analysis, cells were fixed in 80% ethanol for 1 hour (or overnight) at -20°C and then incubated with 2 µg/mL RNase A for 30 minutes at 37°C. Cells were stained with propidium iodide (5g/mL PI) in PBS and analyzed using a Becton Dickinson flow cytometer, BD FACScan and CellQuest acquisition and analysis programs. The results of Fig.1(c) show that NBM-HB-OS01 arrested the human lung cancer A549 cells on the G0/G1 phase in a dose-dependent manner. Human lung cancer A549 cells were inhibited by various concentrations (2.5, 5, 7.5 µ/mL) of NBM-C-BCX-OS01 and NBM-C-BMX-OS01 for 72 hours (see Fig.6(c)) and Human glioblastoma 05-MG cells were inhibited by various concentrations (1.0, 2.0, 4.0 µg/mL) of NBM-C-BCX-OS01 and NBM-C-BMX-OS01 for 72 hours (see Fig.6(d)). Human glioma Hs683 cells were treated with NBM-C-BCX-OS01 (1.0, 2.0, 4.0 µg/mL) for 72 hours. NBM-C-BCX-OS01 could arrest the growth of Human glioma Hs683 cells (see Fig. 6(e)).

### Example 11 Up-regulation of the HDAC associated proteins was observed after treatment of the compounds of the inventions

Rat C6 glioma cells, Human breast cancer MCF-7 cells, and Human lung cancer A549 cells were seeded in 6-well plates. After 24 hrs of incubation in medium+10% BSA, Human breast cancer MCF-7 cells were treated with NBM-HB-OS01 (10.0 µ/mL) and vorinostat (SAHA, 5 µM) for 24 hours. Rat C6 glioma cells were treated with NBM-C-BX-OS01 (7.5µg/mL) and vorinostat (SAHA, 5µM) for 6 hours. Human lung cancer A549 cells were treated with NBM-C-BA-OS01 (7.5µg/mL) and vorinostat (SAHA, 5 µM) for 6 hours. The treated cells were fixed in 80% methanol for 30 minutes and then washed 3 times with PBS solution. Cells were permeabilized with 0.3% Triton X-100 for 30 minutes, and then blocked in 10% fetal bovine serum (FBS) in PBS-T (0.1 % Twin 20 in PBS) for 1 hour. The treated cells were detected with primary antibody against acetyl-Histone H3, acetyl-tubulin, and Gelsolin. Photographs were taken with a Nikon microscope. The results indicated that the compounds of the invention could induce the HDAC associated proteins expression of the various cancer cells (see Fig. 1(g), Fig. 2(e), and Fig. 3(c)).

### Example 12 Increased accumulation of hyperacetylated histone and tubulin and p21 in various cell lines treated with the compound of the invention

Rat C6 glioma cells and Human breast cancer MCF-7 cells were seeded 5×10⁵ cells in a 60-mm dish or 1×10⁶ cells in a 100-mm dish. After 24 hours, Rat C6 glioma cells were treated with various concentrations of NBM-HB-OS01 (2.5, 5.0, 7.5, 10.0 µg/mL) for 72 hours and were treated with 10.0 µg/mL NBM-HB-OS01 for 1, 2, 3, and 4 hours. Human breast cancer MCF-7 cells were treated with 7.5 µg/mL NBM-C-BX-OS01 for 1, 2, 3, and 4 hours. Lysates of C6, and MCF-7 cells were prepared for the immunoblotting of acetyl-Histone H3 (Cell Signaling Technology, Inc.), acetyl-Histone H4 (Upstate), acetyl-tubulin (Sigma Chemical Co.), p21 (BD Pharmingen Technology, Inc.), and actin (Sigma Chemical Co.). Proteins were detected by chemiluminescence (ECL, Amersham). The results indicated that the accumulation of hyperacetylated histone H3, hyperacetylated histone H4, acetylated-tubulin, and p21 were induced in Rat C6 glioma and MCF-7 cells (see Fig 1(e), Fig. 1(f), and Fig. 2(d)). β-actin is an internal control.

### Example 13 Effects of NBM-C-BCX-OS01 and NBM-C-BMX-OS01 on histones and the HDAC associated proteins

Human glioma Hs683 cells were seeded at 5×10⁵ cells in a 60-mm dish or 1×10⁶ cells in a 100-mm dish. After 24 hours, Human glioma Hs683 cells were treated with different doses of NBM-C-BCX-OS01, NBM-C-BMX-OS01 (1.0, 2.0, 4.0 µg/mL), and vorinostat (SAHA, 5µM) for 72 hours. Lysates of Hs683 cells were prepared for the immunoblotting of acetyl-Histone H3 (Cell Signaling Technology, Inc.), acetyl-Histone H4 (Upstate), acetyl-tubulin (Sigma Chemical Co.), p21 (BD Pharmingen Technology, Inc.), CTPS (ABNOVA TAIWAN Corporation), Gelsolin (Sigma Chemical Co.), Hsp90 (Cell Signaling Technology, Inc.), acetyl-Hsp90 (ROCKLAND, Inc.) and actin (Sigma Chemical Co.). Proteins were detected by chemiluminescence (ECL, Amersham). The increase of acetylated Hsp90 and gelsolin proteins was observed in a dose-dependent manner. Hsp90 and CTPS proteins were decreased in a dose-dependent manner (see Fig 7(a)). The expression of p21, acetylated tubulin, acetylated Histone H3, and acetylated Histone H4 was induced in a dose-dependent manner. SAHA was used as a positive control and β-actin as an internal control (see Fig 7(b)).

## Claims

1. A compound represented by the following formula (I): 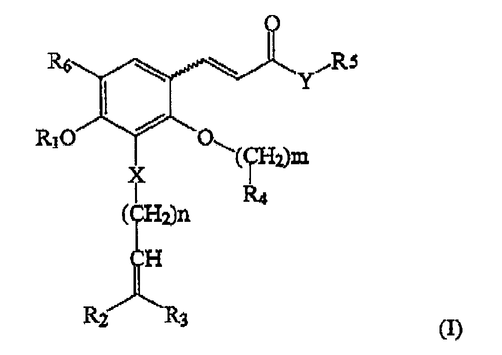 wherein
R₁ is hydrogen, alkyl, alkenyl, C₃₋₈ cycloalkyl, 5-membered or 6-membered unsaturated carbocycle or 5-membered or 6-membered heterocycle;
X is C, O, N or S;
Y is O, NH or O-C₁₋₄alkyl;
n is an integer of 0 to 10;
m is an integer of 0 to 5;
R₂ and R₃ is independently C₁₋₆ alkyl;
R₄ is C₅₋₆ cycloalkyl or 5-membered or 6-membered unsaturated carbocycle or heterocycle which may be substituted with halogen, CF₃, OR₇ or NR₇R₈, wherein R₇ and R₈ are independently hydrogen or C₁₋₆ alkyl;
R₅ is OH, NH₂ or C₅₋₄ cycloalkyl, 5-membered or 6-membered unsaturated carbocycle or heterocycle wherein the cycloalkyl, carboycle and heterocycle may be optionally substituted with halogen, NH₂, NO₂, C₁₋₆ alkoxy, C₁₋₆ alkylthio, OR₇, NR₇R₈ or CF₃,; and
R₆ is H, C₁₋₁₀alkyl which may be substituted by hydroxy or C₂₋₁₀alkenyl, or together with R₁ being -C₂H₂-;
and pharmaceutically acceptable salts, stereoisomers, enantiomers, and solvates thereof.

2. The compound according to Claim 1, wherein R₁, R₂ and R₃ are independently C₁₋₄ alkyl; R₄ is phenyl or phenyl substituted with halogen, CF₃, OC₁₋₄ alkyl, R₅ is OH, phenyl or phenyl substituted with NH₂ and R₆ is hydrogen.

3. The compound according to Claim 1, wherein R₁, R₂ and R₃ are independently methyl, R₄ is phenyl or phenyl substituted with F, Cl, Br, CF₃, OCH₃, R₅ is OH or phenyl substituted with NH₂ and R₆ is hydrogen.

4. The compound according to Claim 1, wherein X is carbon.

5. The compound according to Claim 1, wherein n and m is 1.

6. The compound according to Claim 1, wherein the carbocycle is cycloalkenyl or aryl.

7. The compound according to Claim 6, wherein the cycloalkenyl is selected from the group consisting of cyclopropenyl, cyclobutenyl, cyclopentenyl, cyclohexenyl, cycloheptenyl and cyclooctenyl, and the aryl is selected from the group consisting of phenyl, naphthyl and tetrahydronaphthaienyl.

8. The compound according to Claim 1, wherein the 5-membered or 6-membered unsaturated heterocycle is selected from the group consisting of furyl, thienyl, pyrrolyl, imidazolyl, pyrazolyl, triazolyl, tetrazolyl, oxazolyl, isoxazolyl, thiazolyl, thiadiazolyl, isothiazolyl, pyridyl, pyridazinyl, pyrimidinyl, furazanyl, pyrazinyl, oxadiazolyl, 1-pyrrolinyl, 2-pyrrolinyl, 3-pyrrolinyl, pyrrolidino, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidyl, 3-piperidyl, 4-piperidyl, piperazino, 2-piperazinyl, 2-morpholinyl, 3-morpholinyl, morpholino and tetrahydropyranyl.

9. The compound according to Claim 1, which is selected from the group consisting of:

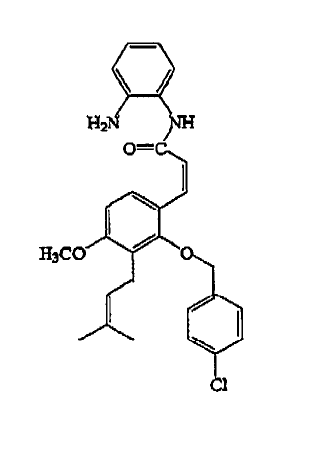

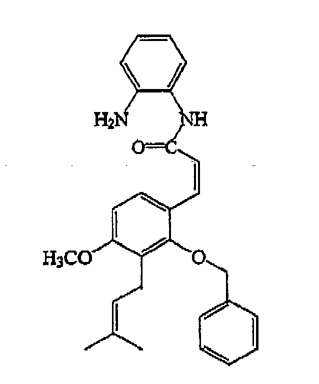

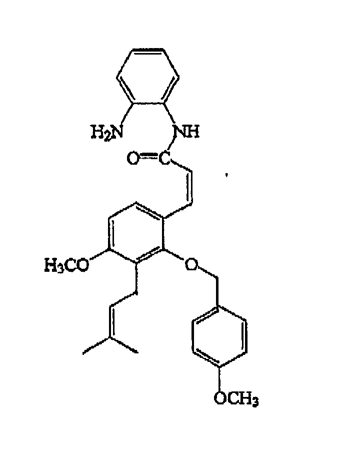

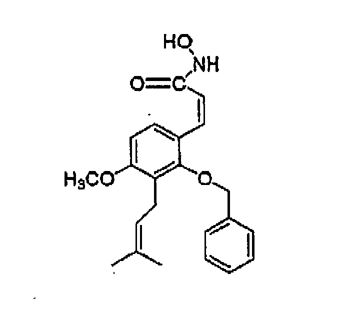

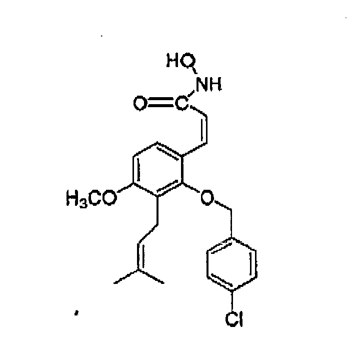

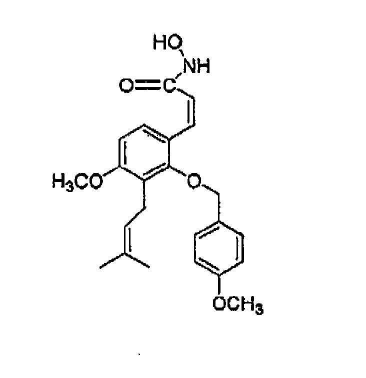

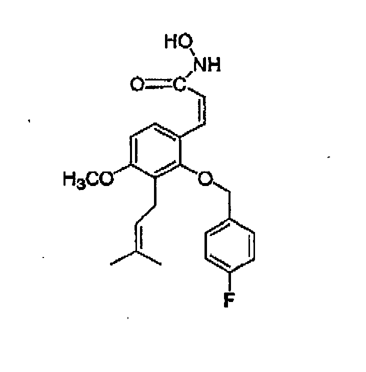

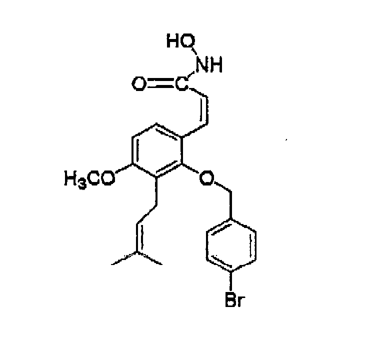

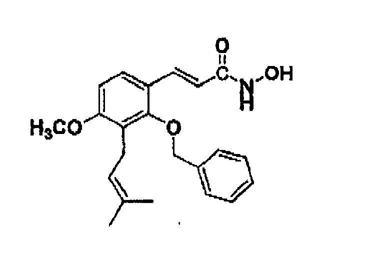

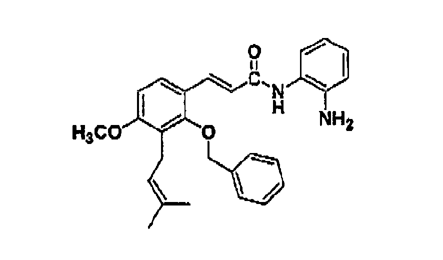

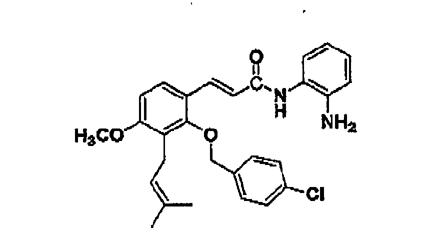

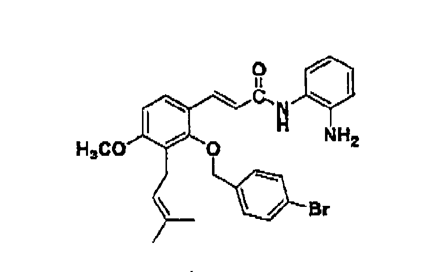

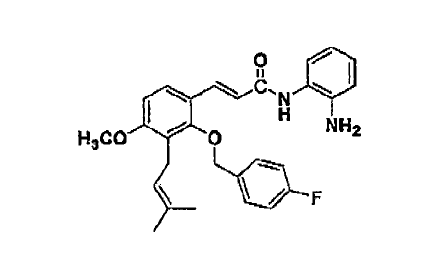

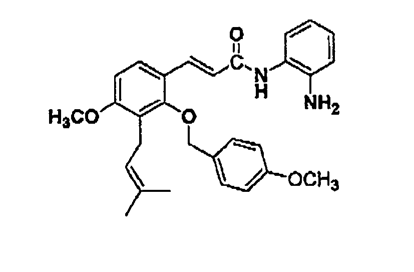

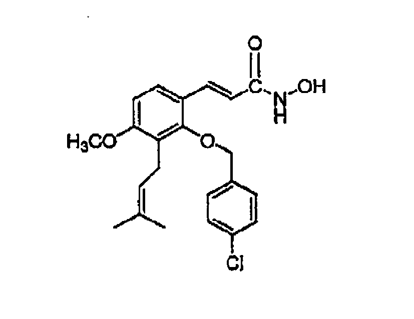

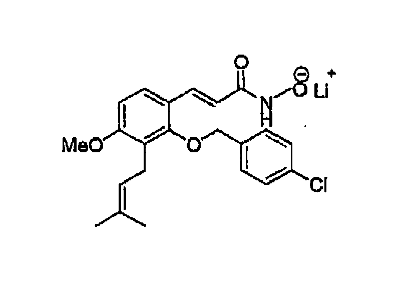

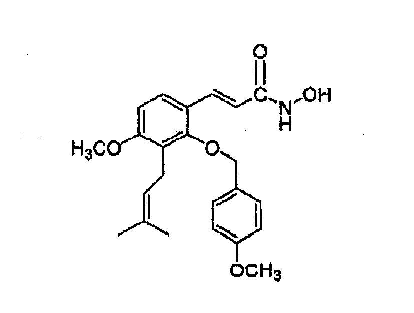

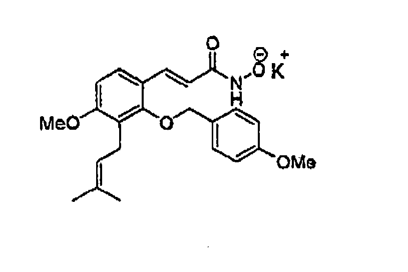

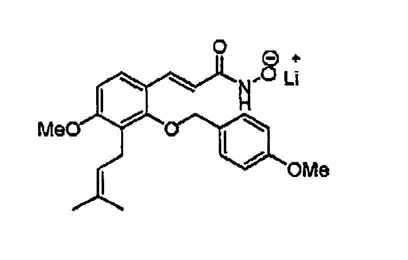

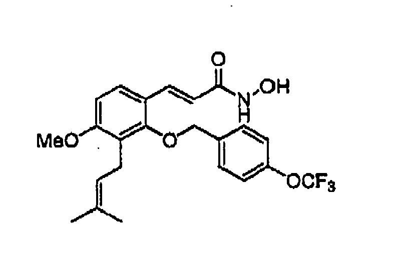

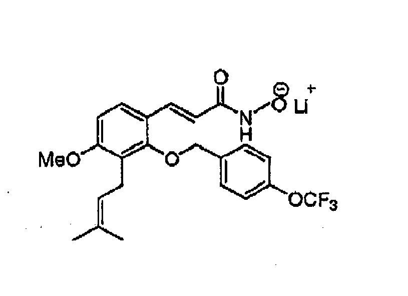

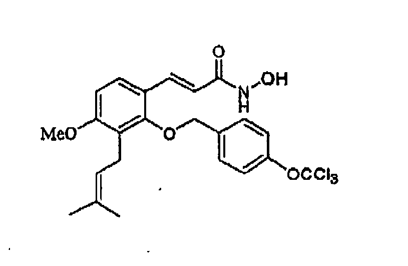

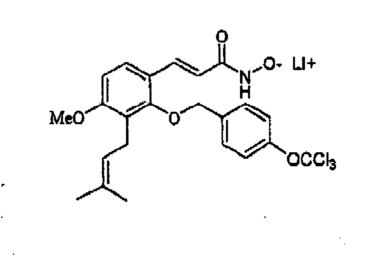

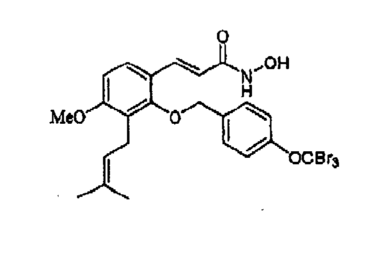

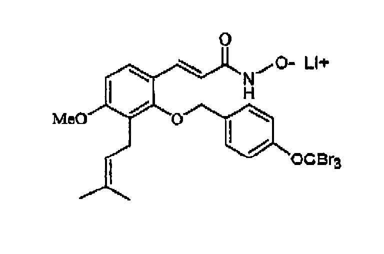

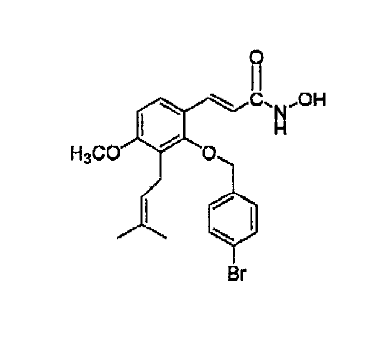

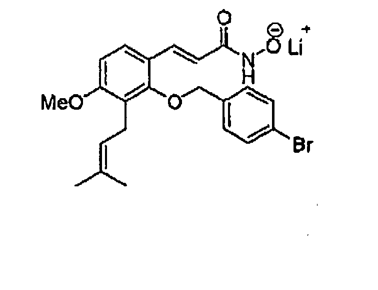

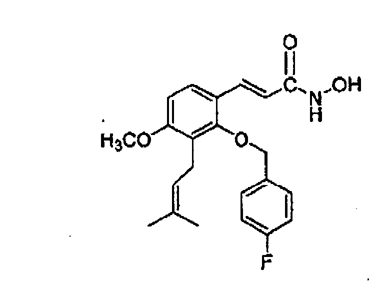

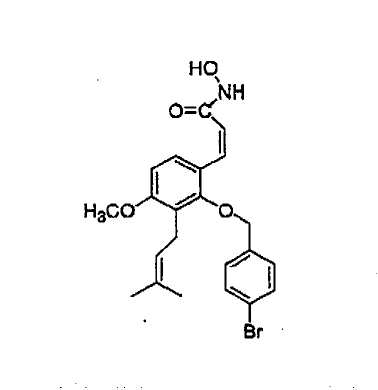

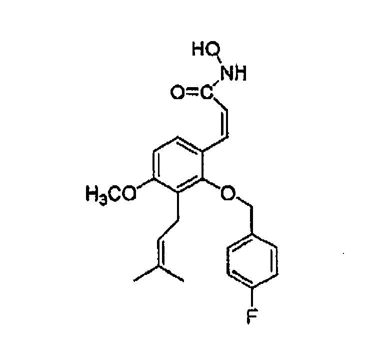

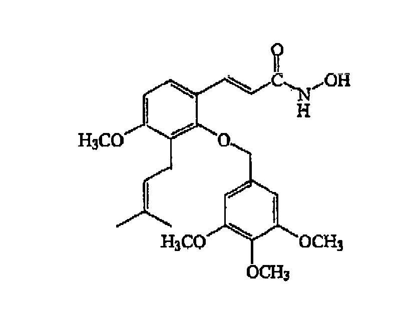

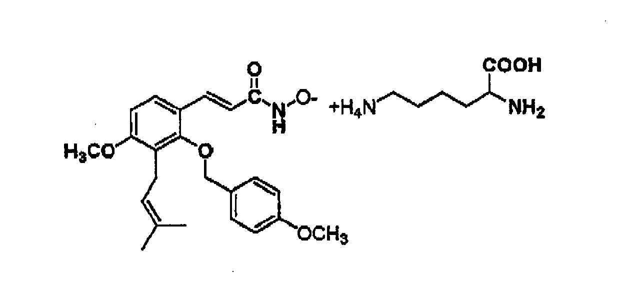

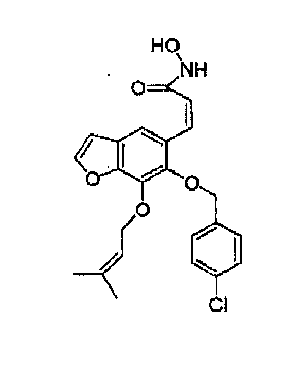

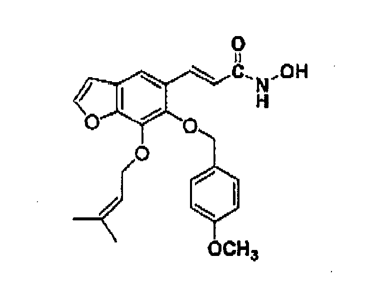

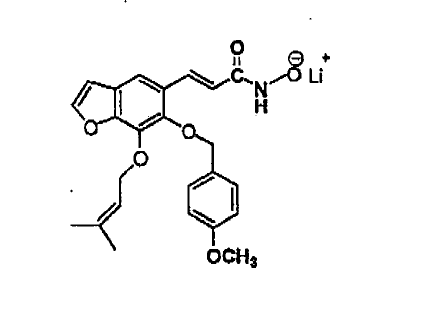

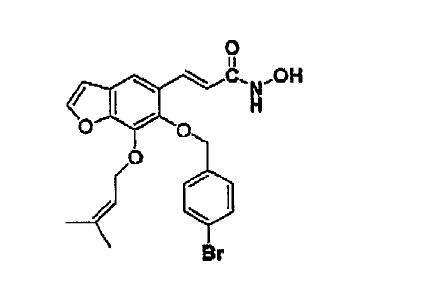

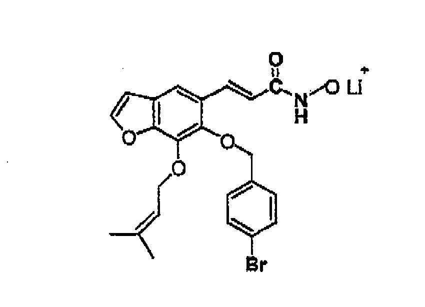

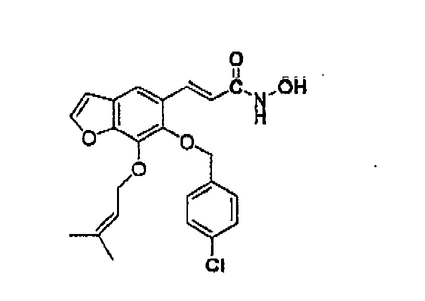

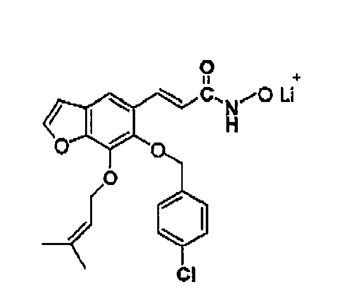

10. A pharmaceutical composition comprising the compound of Claim 1 or pharmaceutically acceptable salts, stereoisomers, enantiomers, and solvates thereof as an active ingredient and a pharmaceutically acceptable carrier.

11. Use of the compound of Claim 1 or pharmaceutically acceptable salts, stereoisomers, enantiomers, and solvates thereof in the manufacture of a medicament for inhibiting histone deacetylase (HDAC).

12. Use of the compound of Claim 1 or pharmaceutically acceptable salts, stereoisomers, enantiomers, and solvates thereof in the manufacture of a medicament for treating diabetes.

13. Use of the compound of Claim 1 or pharmaceutically acceptable salts, stereoisomers, onantiomers, and solvates thereof in the manufacture of a medicament for treating tumor or cell proliferative disease.

14. Use of the compound of Claim 1 or pharmaceutically acceptable salts, stereoisomers, enantiomers, and solvates thereof in the manufacture of a medicament for enhancing neurite outgrowth.

15. Use of the compound of Claim 1 or pharmaceutically acceptable salts, stereoisomers, enantiomers, and solvates thereof in the manufacture of a medicament for treating neurodegenerative diseases and human spinal muscular atrophy (SMA) disease.

16. The use of Claim 15, wherein the neurodegenerative disease is Hunimgton's disease or poly-glutamine disease.

17. A compound respresented by the formula

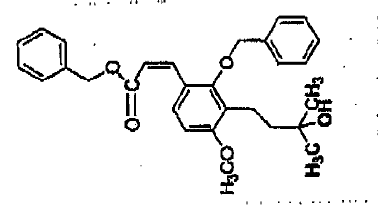

## Patentansprüche

1. Verbindung, die durch die folgende Formel (I) 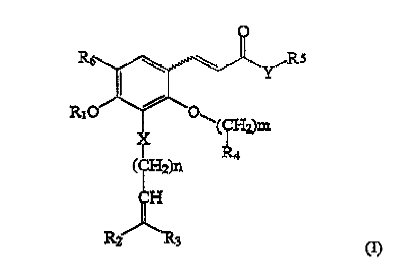 dargestellt wird, worin:
R₁ Wasserstoff, Alkyl, Alkenyl, C₃₋₈-Cycloalkyl, ein 5-gliedriger oder 6-gliedriger ungesättigter Carbocyclus oder ein 5-gliedriger oder 6-gliedriger Heterocyclus Ist,
X C, O, N oder S Ist,
Y O, NH oder O-C₁₋₄-Alkyl ist,
n eine ganze Zahl von 0 bis 10 ist,
m eine ganze Zahl von 0 bis 5 ist,
R₂ und R₃ unabhängig C₁₋₆-Alkyl sind,
R₄ ein C₅₋₆-Cycloalkyl oder ein 5-gliedriger oder 6-gliedriger ungesättigter Carbocyclus oder
Heterocyclus ist, das oder der mit Halogen, CF₃, OR₇ oder NR₇R₈ substituiert sein kann, wobei R₇ und R₈ unabhängig Wasserstoff oder C₁-₆-Alkyl sind,
R₅ OH, NH₂ oder C₅₋₆-Cycloalkyl, ein 5-gliedriger oder 6-gliedriger ungesättigter Carbocyclus oder Heterocyclus ist, wobei das Cycloalkyl, der Carbocyclus und der Heterocyclus gegebenenfalls mit Halogen, NH₂, NO₂, C₁₋₆-Alkoxy, C₁₋₆-Alkylthio, OR₇, NR₇R₈ oder CF₃ substituiert sein können, und
R₆ H, C₁₋₁₀-Alkyl, das mit Hydroxy oder C₂₋₁₀-Alkenyl substituiert sein kann oder zusammen mit R₁ -C₂H₂- ist,
und pharmazeutisch verträgliche Salze, Stereoisomere, Enantiomere und Solvate davon.

2. Verbindung nach Anspruch 1, worin R₁, R₂ und R₃ unabhängig C₁₋₄-Alkyl sind, R₄ Phenyl oder Phenyl, das mit Halogen, CF₃, OC₁₋₄-Alkyl substituiert ist, ist, R₅ OH, Phenyl oder Phenyl, das mit NH₂ substituiert ist, Ist, und R₆ Wasserstoff Ist.

3. Verbindung nach Anspruch 1, worin R₁, R₂ und R₃ unabhängig Methyl sind, R₄ Phenyl oder Phenyl, das mit F, Cl, Br, CF₃, OCH₃ substituiert ist, ist, R₅ OH oder Phenyl, das mit NH₂ substituiert ist, ist, und R₆ Wasserstoff Ist.

4. Verbindung nach Anspruch 1, worin X Kohlenstoff ist.

5. Verbindung nach Anspruch 1, worin n und m 1 sind.

6. Verbindung nach Anspruch 1, worin der Cerbocyclus Cycloalkenyl oder Aryl ist.

7. Verbindung nach Anspruch 6, worin das Cycloalkenyl aus der Gruppe, bestehend aus Cyclopropenyl, Cyclobutenyl, Cyclopentenyl, Cyclohexenyl, Cycloheptenyl und Cyclooctenyl, ausgewählt ist, und das Aryl aus der Gruppe, bestehend aus Phenyl, Naphthyl und Tetrahydronaphthalenyl, ausgewählt Ist.

8. Verbindung nach Anspruch 1, worin der 5-gliedrige oder 6-gliedrige ungesättigte Heterocyclus aus der Gruppe, bestehend aus Furyl, Thienyl, Pyrrolyl, Imidazolyl, Pyrazolyl, Triazolyl, Tetrazolyl, Oxazolyl, Isoxazolyl, Thiazolyl, Thiadiazolyl, Isothiazolyl, Pyridyl, Pyridazinyl, Pyrimidinyl, Furazanyl, Pyrazinyl, Oxadiazolyl, 1-Pyrrolinyl, 2-Pyrrolinyl, 3-Pyrrolinyl, Pyrrolidino, 2-Pyrrolidinyl, 3-Pyrrolidinyl, 1-Imidazolinyl, 2-Imidazolinyl, 4-Imidazolinyl, 1-Imidazolidinyl, 2-Imidazolidinyl, 4-Imidazolidinyl, 1-Pyrazolinyl, 3-Pyrazolinyl, 4-Pyrazolinyl, 1-Pyrazolidinyl, 3-Pyrazolldlnyl, 4-Pyrazolidinyl, Piperidino, 2-Piperidyl, 3-Piperidyl, 4-Piperidyl, Piperazino, 2-Piperazinyl, 2-Morpholinyl, 3-Morpholinyl, Morpholino und Tetrahydropyranyl, ausgewählt Ist.

9. Verbindung nach Anspruch 1, die aus der Gruppe, bestehend aus 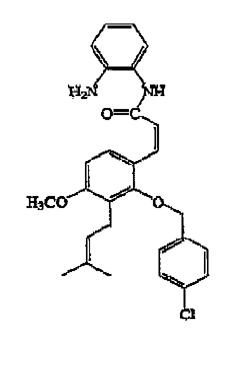 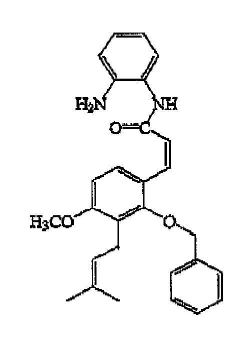 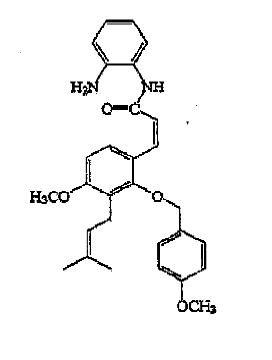 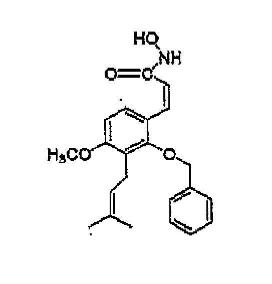 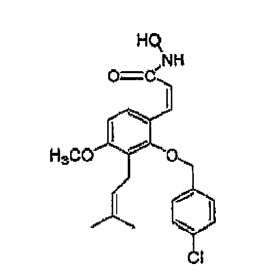 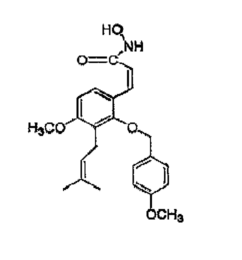 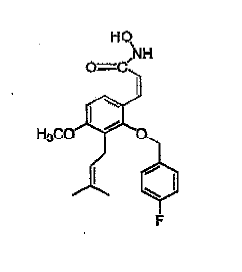 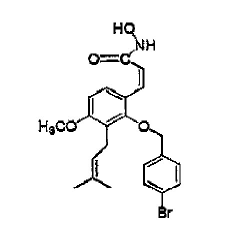 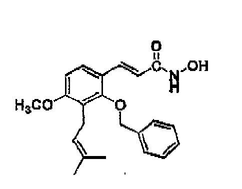 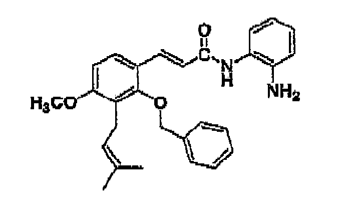 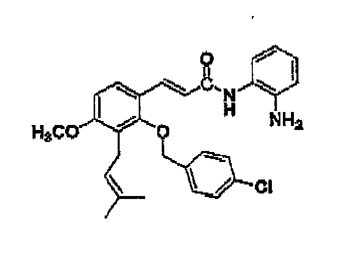 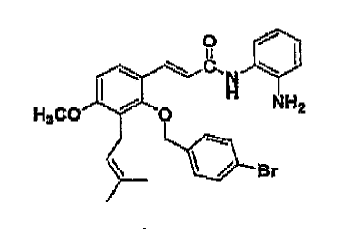 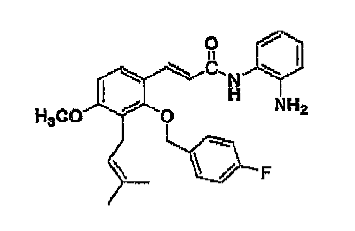 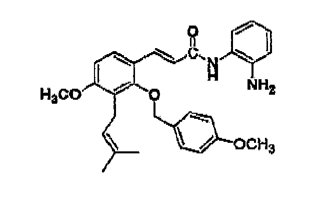 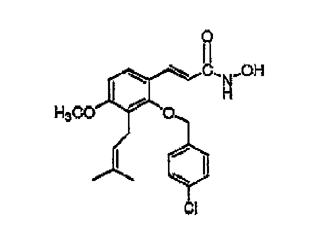 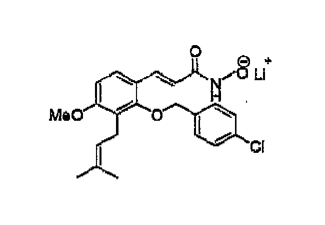 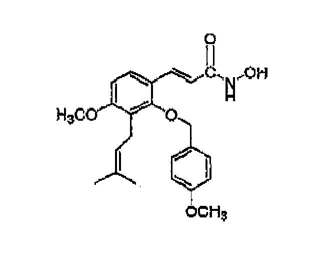 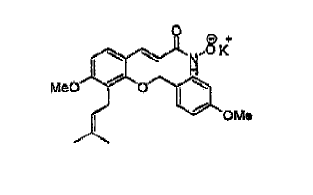 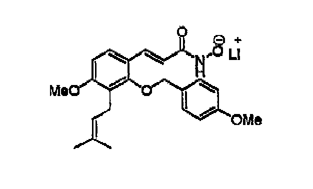 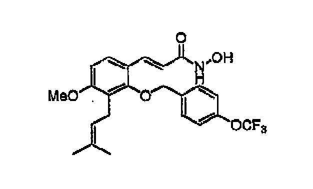 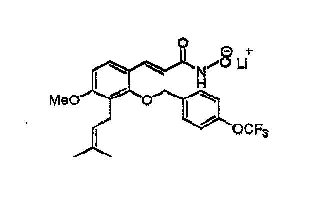 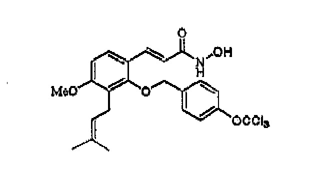 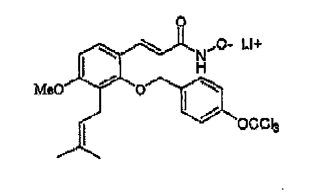 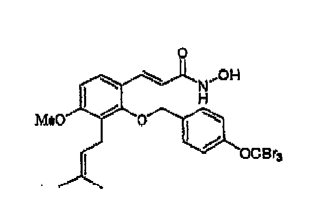 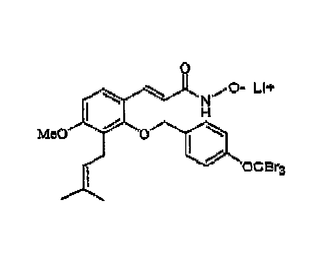 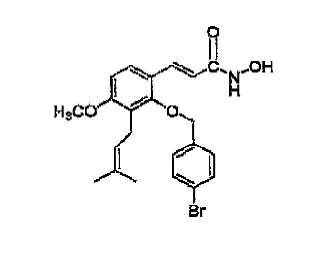 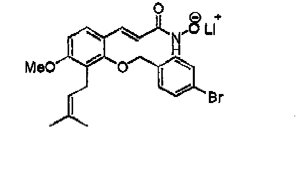 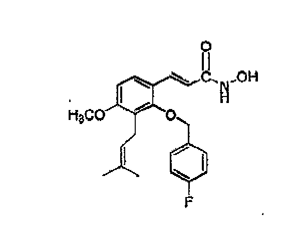 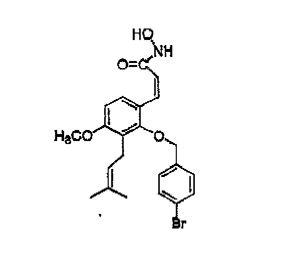 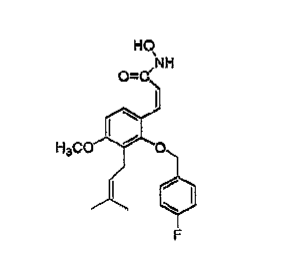 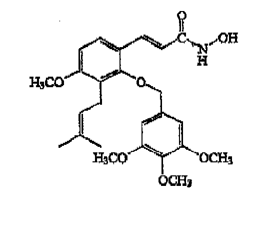 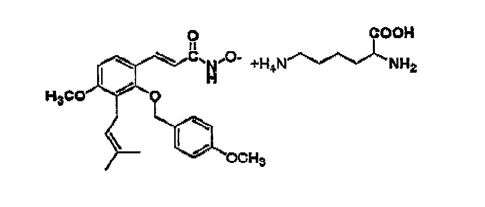 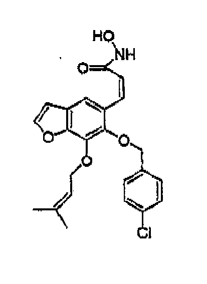 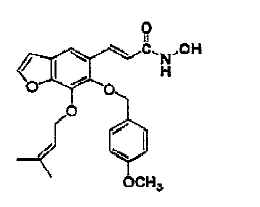 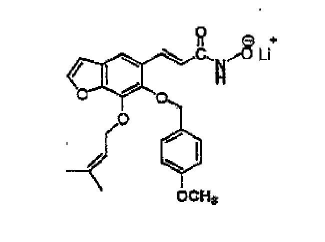 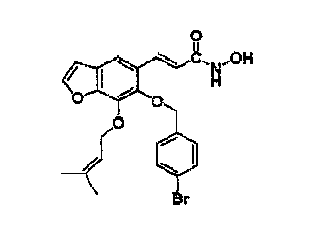 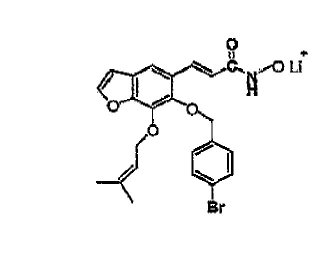 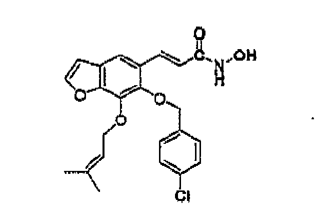 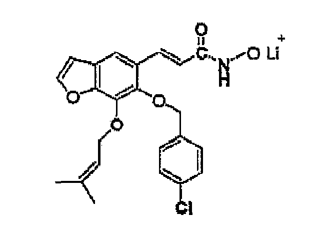 ausgewählt ist.

10. Pharmazeutische Zusammensetzung, welche die Verbindung nach Anspruch 1 oder pharmazeutisch verträgliche Salze, Stereoisomere, Enantiomere und Solvate davon als Wirkstoff und einen pharmazeutlsch verträglichen Träger umfasst.

11. Verwendung der Verbindung nach Anspruch 1 oder pharmazeutisch verträglicher Salze, Stereoisomere, Enantiomere und Solvate davon bei der Herstellung eines Medikaments zur Hemmung von Histondesacetylase (HDAC).

12. Verwendung der Verbindung nach Anspruch 1 oder pharmazeutisch verträglicher Salze, Stereoisomere, Enantiomere und Solvate davon bei der Herstellung eines Medikaments zur Behandlung von Diabetes.

13. Verwendung der Verbindung nach Anspruch 1 oder pharmazeutisch verträglicher Salze, Stereoisomere, Enantiomere und Solvate davon bei der Herstellung eines Medikaments zur Behandlung eines Tumors oder einer zellproliferativen Erkrankung.

14. Verwendung der Verbindung nach Anspruch 1 oder pharmazeutisch verträglicher Salze, Stereoisomere, Enantiomere und Solvate davon bei der Herstellung eines Medikaments zur Verstärkung des Neuritenauswuchses.

15. Verwendung der Verbindung nach Anspruch 1 oder pharmazeutisch verträglicher Salze, Stereoisomere, Enantiomere und Solvate davon bei der Herstellung eines Medikaments zur Behandlung neurodegenerativer Erkrankungen und von menschlicher spinaler Muskelatrophie (SMA).

16. Verwendung nach Anspruch 15, wobei die neurodegenerative Erkrankung Chorea Huntington oder Polyglutaminkrankheit ist.

17. Verbindung, die durch die Formel 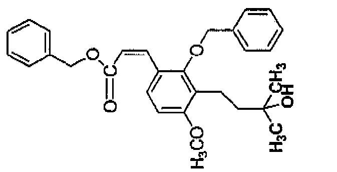 dargestellt wird.

## Revendications

1. Composé représenté par la formule (I) suivante : 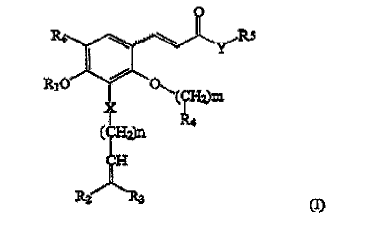 dans laquelle :
- R₁ représente hydrogène, alkyle, alcényle, cycloalkyle en C₃-C₈, carbocyclyle insaturé à 5 ou 6 chaînons ou hétérocyclyle à 5 ou 6 chaînons ;
- X représente C, O, N ou S ;
- Y représente O, NH ou O-alkyle en C₁-C₄ ;
- n est un entier de 0 à 10 ;
- m est un entier de 0 à 5 ;
- R₂ et R₃ représentent indépendamment alkyle en C₁-C₆ ;
- R₄ représente cycloalkyle en C₅-C₆ ou carbocyclyle ou hétérocyclyle insaturé à 5 ou 6 chaînons qui peut être substitué par halogène, CF₃, OR₇ ou NR₇R₈, R₇ et R₈ représentant indépendamment hydrogène ou alkyle en C₁-C₆ ;
- R₅ représente OH, NH₂ ou cycloalkyle en C₅-C₆, carbocyclyle ou hétérocyclyle insaturé à 5 ou 6 chaînons, les cycloalkyle, carbocyclyle et hétérocyclyle pouvant être facultativement substitués par halogène, NH₂, NO₂, alcoxy en C₁-C₆, alkylthio en C₁-C₆, OR₇, NR₇R₈ ou CF₃ ; et
- R₆ représente H, alkyle en C₁-C₁₀ qui peut être substitué par hydroxy ou alcényle en C₂-C₁₀ ou conjointement avec R₁ représentant -C₂H₂- ;
et les sels, stéréoisomères, énantiomères et solvates pharmaceutiquement acceptables de celui-ci.

2. Composé selon la revendication 1, dans lequel R₁, R₂ et R₃ représentent indépendamment alkyle en C₁-C₄ ; R₄ représente phényle ou phényle substitué par halogène, CF₃, O-alkyle en C₁-C₄, R₅ représente OH, phényle ou phényle substitué par NH₂ et R₆ représente hydrogène.

3. Composé selon la revendication 1, dans lequel R₁, R₂ et R₃ représentent indépendamment méthyle, R₄ représente phényle ou phényle substitué par F, Cl, Br, CF₃, OCH₃, R₅ représente OH ou phényle substitué par NH₂ et R₆ représente hydrogène.

4. Composé selon la revendication 1, dans lequel X représente carbone.

5. Composé selon la revendication 1, dans lequel n et m sont 1.

6. Composé selon la revendication 1, dans lequel le carbocyclyle représente cycloalcényle ou aryle.

7. Composé selon la revendication 6, dans lequel le cycloalcényle est choisi dans le groupe consistant en cyclopropényle, cyclobutényle, cyclopentényle, cyclohexényle, cycloheptényle et cyclooctényle, et l'aryle est choisi dans le groupe consistant en phényle, naphtyle et tétrahydronaphtalényle.

8. Composé selon la revendication 1, dans lequel l'hétérocycle insaturé à 5 ou 6 chaînons est choisi dans le groupe consistant en furyle, thiényle, pyrrolyle, imidazolyle, pyrazolyle, triazolyle, tétrazolyle, oxazolyle, isoxazolyle, thiazolyle, thiadiazolyle, isothiazolyle, pyridyle, pyridazinyle, pyrimidinyle, furazanyle, pyrazinyle, oxadiazolyle, 1-pyrrolinyle, 2-pyrrolinyle, 3-pyrrolinyle, pyrrolidino, 2-pyrrolidinyle, 3-pyrrolidinyle, 1-imidazolinyle, 2-imidazolinyle, 4-imidazolinyle, 1-imidazolidinyle, 2-imidazolidinyle, 4-imidazolidinyle, 1-pyrazolinyle, 3-pyrazolinyle, 4-pyrazolinyle, 1-pyrazolidinyle, 3-pyrazolidinyle, 4-pyrazolidinyle, pipéridino, 2-pipéridyle, 3-pipéridyle, 4-pipéridyle, pipérazino, 2-pipérazinyle, 2-morpholinyle, 3-morpholinyle, morpholino et tétrahydropyranyle.

9. Composé selon la revendication 1, qui est choisi dans le groupe consistant en :

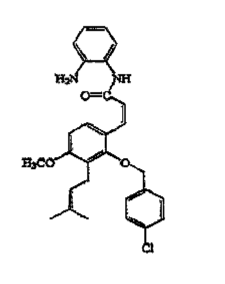

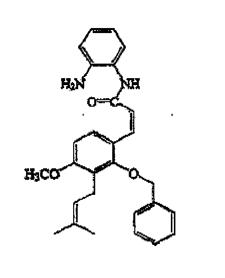

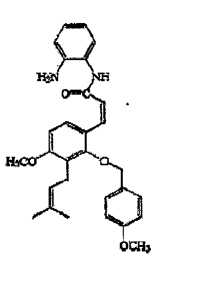

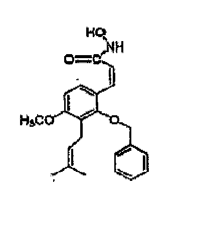

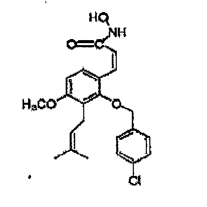

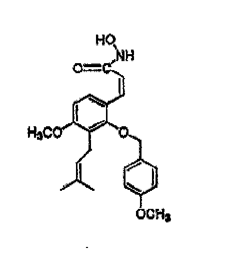

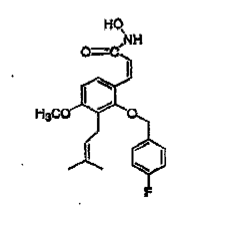

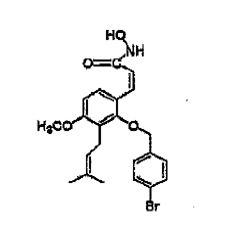

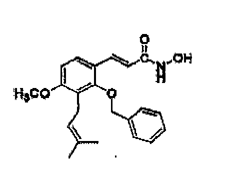

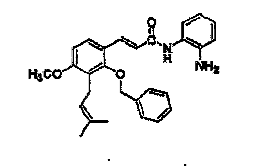

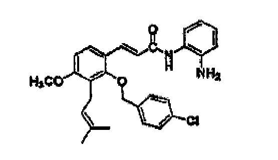

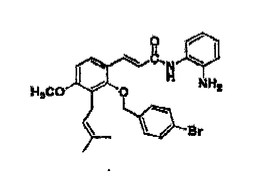

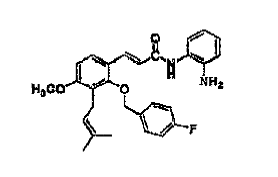

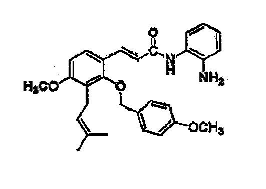

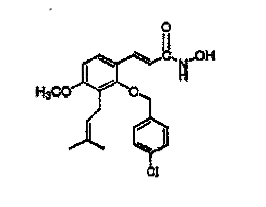

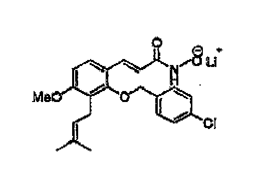

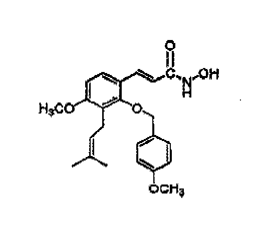

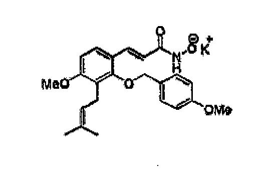

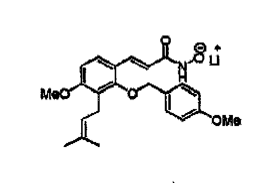

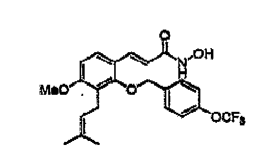

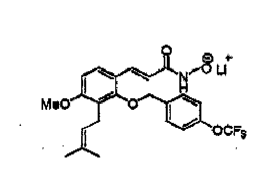

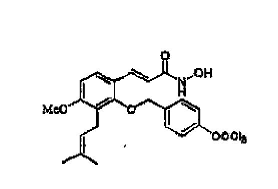

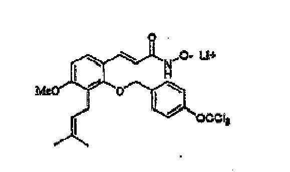

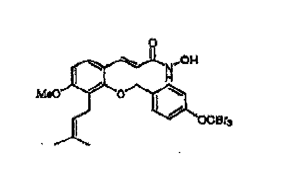

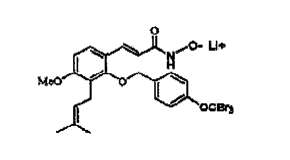

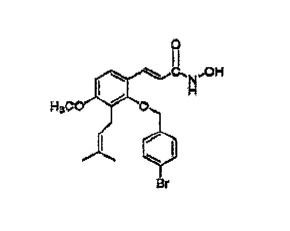

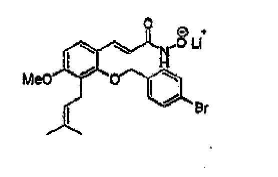

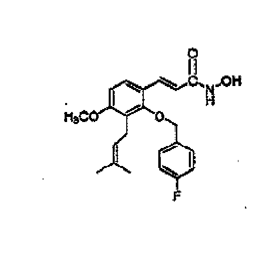

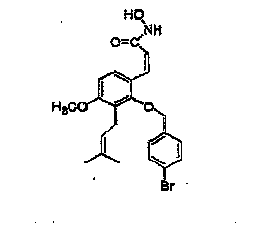

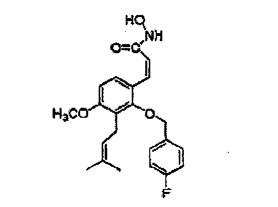

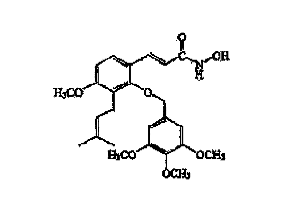

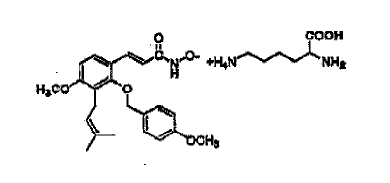

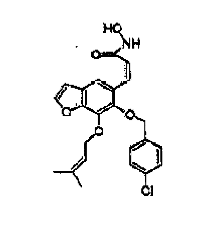

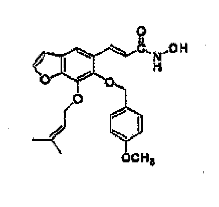

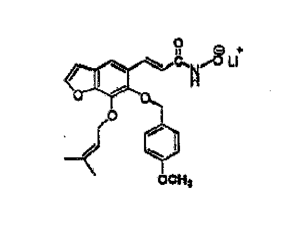

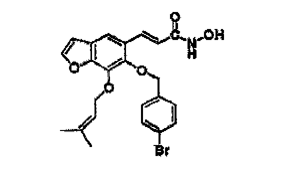

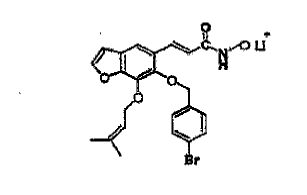

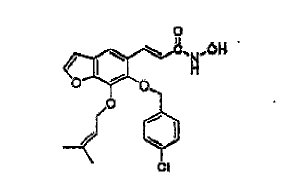

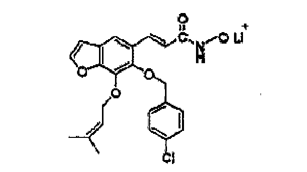

10. Composition pharmaceutique comprenant le composé de la revendication 1 ou les sels, stéréoisomères, énantiomères et solvates pharmaceutiquement acceptables de celui-ci comme principe actif et un support pharmaceutiquement acceptable.

11. Utilisation du composé selon la revendication 1 ou des sels, stéréoisomères, énantiomères et solvates pharmaceutiquement acceptables de celui-ci dans la fabrication d'un médicament pour l'inhibition de l'histone désacétylase (HDAC).

12. Utilisation du composé selon la revendication 1 ou des sels, stéréoisomères, énantiomères, et solvates pharmaceutiquement acceptables de celui-ci dans la fabrication d'un médicament pour le traitement du diabète.

13. Utilisation du composé selon la revendication 1 ou des sels, stéréoisomères, énantiomères et solvates pharmaceutiquement acceptables de celui-ci dans la fabrication d'un médicament pour le traitement d'une tumeur ou d'une maladie proliférative des cellules.

14. Utilisation du composé selon la revendication 1 ou des sels, stéréoisomères, énantiomères et solvates pharmaceutiquement acceptables de celui-ci dans la fabrication d'un médicament pour améliorer l'excroissance des neurites.

15. Utilisation du composé selon la revendication 1 ou des sels, stéréoisomères, énantiomères et solvates pharmaceutiquement acceptables de celui-ci dans la fabrication d'un médicament pour traiter des maladies neurodégénératives et la maladie d'atrophie musculaire spinale (SMA) humaine.

16. Utilisation selon la revendication 15, dans laquelle la maladie neurodégénérative est la maladie de Huntington ou une maladie à polyglutamine.

17. Composé représenté par la formule :
